# FASCICULE DE BREVET EUROPEEN

(11) **EP 2 285 952 B1**
(45) Date de publication et mention de la délivrance du brevet: **23.03.2016**
(21) Numéro de dépôt: 09766080.7
(22) Date de dépôt: 16.06.2009
(51) Int. Cl.: C12N 7/04, C07K 14/02, C07K 14/18, A61K 39/29, A61K 39/295, C12N 15/62

(54) **NOUVELLES PROTEINES DE FUSION ET LEUR APPLICATION POUR LA PREPARATION DE VACCINS CONTRE L'HEPATITE C**
NEUE FUSIONSPROTEINE UND DEREN VERWENDUNG ZUR HERSTELLUNG VON HEPATITIS-C-IMPFSTOFFEN
NOVEL FUSION PROTEINS AND USE THEREOF FOR PREPARING HEPATITIS C VACCINES

(30) Priorité: 17.06.2008 FR 0803377
(43) Date de publication de la demande: 23.02.2011
(73) Titulaire: Université François Rabelais de Tours, 37041 Tours Cedex 1 (FR)
(72) Inventeur: ROINGEARD, Philippe, F-37510 Savonnieres (FR); HOURIOUX, Christophe, F-37190 Druye (FR); PATIENT, Romuald, F-37300 Joue-les-tours (FR)
(74) Mandataire: Grosset-Fournier, Chantal Catherine
(86) Numéro de dépôt international: PCT/FR2009/051142
(87) Numéro de publication internationale: WO 2009/153518

(56) Documents cités:
- WO-A-2008/025067
- US-A1- 2004 146 529
- NETTER H J ET AL: "Antigenicity and immunogenicity of novel chimeric hepatitis B surface antigen particles with exposed hepatitis C virus epitopes" JOURNAL OF VIROLOGY, THE AMERICAN SOCIETY FOR MICROBIOLOGY, US, vol. 75, no. 5, 1 mars 2001 (2001-03-01), pages 2130-2141, XP002977998 ISSN: 0022-538X
- PATIENT R ET AL: "Chimeric hepatitis B and C viruses envelope proteins can form subviral particles: implications for the design of new vaccine strategies" NEW BIOTECHNOLOGY,, vol. 25, no. 4, 1 avril 2009 (2009-04-01), pages 226-234, XP026059214 ISSN: 1871-6784 [extrait le 2009-01-21]

## Description

L'invention a pour objet de nouvelles protéines de fusion et leur application notamment pour la préparation de vaccins destinés à la prévention et/ou au traitement prophylactique de l'hépatite C, ou à la prévention et/ou au traitement prophylactique e l'hépatite C et de l'hépatite B. La présente invention concerne également l'obtention de particules d'enveloppes sous virales chimères entre les protéines d'enveloppe du virus humain de l'hépatite B (HBV, pour hepatitis B virus) et du virus de l'hépatite C (HCV, pour hepatitis C virus).

Le virus de l'hépatite C, identifié en 1989, puis cloné et séquencé, représente encore à l'heure actuelle un réel problème de santé publique du fait de sa large répartition à travers le monde et de l'évolution fréquente de la maladie vers la chronicité. En 2000, l'OMS estimait qu'environ 3% de la population mondiale, soit environ 170 millions de personnes, était infectée par le HCV.

Le HCV induit des hépatites chroniques qui peuvent évoluer vers la cirrhose et le carcinome hépatocellulaire. L'interféron et la ribavirine forment le traitement de base des hépatites chroniques induites par le HCV, mais ces traitements ne sont pas suffisamment efficaces et ont des effets secondaires importants. Les traitements disponibles restent coûteux, relativement toxiques et ne sont efficaces que dans la moitié des cas d'infection.

Bien que l'intégralité du génome et que les protéines virales soient connus depuis de nombreuses années, sa structure et sa morphogenèse restent hypothétiques. Aujourd'hui encore, il n'existe aucun vaccin contre l'hépatite C, et la recherche d'un tel candidat vaccin est actuellement très active, [Houghton, M., and Abrignani, S. (2005). Prospects for a vaccine against the hepatitis C virus. Nature 436 (7053), 961-6].

Dans le cas d'un vaccin prophylactique, la quasi-totalité des candidats potentiels reposent sur l'utilisation de l'une des deux ou des deux protéines d'enveloppe du HCV, communément dénommées les protéines E1 et E2, et susceptibles d'induire à la fois une réponse immunitaire cellulaire, et une réponse humorale neutralisante.

Cependant, les protéines E1 et E2 du HCV ne s'auto-assemblent pas en particules sous-virales comme cela peut être le cas pour d'autres virus. Par ailleurs, du fait d'une forte rétention de leur région transmembranaire dans le réticulum endoplasmique, leur purification nécessite de les solubiliser avec des détergents. Il en résulte des rendements décevants et la pureté des fractions obtenues est médiocre [Forns, X., Bukh, J., and Purcell, R. H. (2002). The challenge of developing a vaccine against hepatitis C virus. J Hepatol 37(5), 684-95].

L'alternative consistant à recourir aux protéines d'enveloppe délétées de leur domaine transmembranaire permet de favoriser la sécrétion de E1 et E2 à l'extérieur de la cellule, mais le changement de conformation tridimentionelle qui en résulte peut s'avérer indésirable du fait d'une qualité antigénique amoindrie au regards des protéines sauvages.

Le virus HBV existe sous deux formes : sous la forme de virions infectieux, et sous la forme de particules d'excès d'enveloppe que l'on retrouve dans le sang des sujets infectés en quantité bien plus importante que le virus lui-même. Ce phénomène est dû à la capacité de la protéine S du HBV à s'auto-assembler et à bourgeonner en particules sous-virales (ou d'excès d'enveloppe) qui ne contiennent pas.de protéine de capside ni d'acide nucléique [Moriarty, A. M., Hoyer, B. H., Shih, J. W., Gerin, J. L., and Hamer, D. H. (1981). Expression of the hepatitis B virus surface antigen gene in cell culture by using a simian virus 40 vector. Proc Natl Acad Sci U S A 78(4), 2606-10]. La protéine S sauvage du HBV comporte quatre domaines transmembranaires. Les particules sous-virales qui résultent de l'auto-assemblage de la protéine S sauvage sont non infectieuses mais très immunogènes : elles sont considérées comme un bon candidat vaccin contre l'hépatite B depuis le milieu des années 70. Elles ont en effet servi de fondement à l'élaboration de vaccins ayant fait la preuve de leur efficacité à induire une réponse immunitaire protectrice de l'infection par le HBV.

Le document WO 2008025067 décrit des protéines de fusion comprenant la protéine S du virus de l'hépatite B du canard (DHBV).

L'utilisation des particules sous-virales d'enveloppe de HBV comme vecteur de protéines étrangères au virus de l'hépatite B a déjà fait l'objet de travaux antérieurs. Ainsi la demande de brevet n° US2004/0146529 intitulée « HBV /HCV virus like particle » décrit l'obtention de chimères d'enveloppe HBV-HCV comportant, d'une part, systématiquement l'intégralité de la protéine S du virus HBV, et, d'autre part, d'après les exemples, un fragment de l'ectodomaine d'une des protéines d'enveloppes E1 ou E2 de HCV, greffé à l'extrémité N-terminale de la protéine S du virus HBV [Mark Selby, Edward Glazer and Michael Houghton « HBV/HCV virus-like particle" brevet US n°2004/01465529].

Cependant il n'est pas démontré que ces constructions peuvent induire la formation de particules sous-virales bien structurées, et qu'elles sont susceptibles d'induire une réponse antigénique de qualité.

En réponse aux inconvénients de l'art antérieur, la présente invention a pour but de fournir une protéine de fusion HCV-HBV capable de former des particules sous-virales non infectieuses, bien structurées et efficacement secrétées, et contenant la quasi-totalité de E1 et/ou E2.

Un des objectifs de l'invention est également de fournir un vaccin contre l'hépatite C et/ou contre l'hépatite B.

Un intérêt supplémentaire de l'invention vient du fait qu'elle peut aisément s'adapter aux chaînes existantes de production industrielles des vaccins contre l'hépatite B actuellement commercialisés.

L'invention dans son sens le plus large est telle que définie dans les revendications indépendantes.

L'invention a pour objet une protéine de fusion immunogène comprenant au moins les deux peptides suivants:
a)- du côté C-terminal, un premier peptide constitué :
   - de la séquence d'acides aminés de la protéine S d'un isolat du virus humain de l'hépatite B (HBV), laquelle protéine S est délétée de son domaine transmembranaire situé à son extrémité N-terminale, ladite séquence d'acides aminés conservant la capacité à former des particules sous-virales non-infectieuses immunogènes vis-à-vis du virus humain de l'hépatite B, où
   - d'une séquence d'acides aminés présentant un pourcentage d'identité d'au moins 91%, notamment d'au moins 93%, particulièrement d'au moins 95%, et plus particulièrement d'au moins 97%, avec ladite séquence d'acides aminés de la protéine S délétée en N-terminal, sous réserve que ladite séquence d'acides aminés soit aussi délétée du domaine transmembranaire situé à l'extrémité N-terminale de la protéine S de HBV, et qu'elle conserve la capacité à former des particules sous-virales non infectieuses immunogènes vis-à-vis du virus humain de l'hépatite B, ou,
   - de la séquence d'acides aminés d'un variant synthétique dérivé de ladite séquence d'acides aminés de ladite protéine S délétée, sous réserve que ladite séquence d'acides aminés dudit variant synthétique soit aussi délétée du domaine transmembranaire situé à l'extrémité N-terminale de la protéine S de HBV, et qu'elle conserve la capacité à former des particules sous-virales non infectieuses immunogènes vis-à-vis du virus humain de l'hépatite B, et,
b)- du côté N-terminal, un second peptide constitué :
   - de la séquence d'acides aminés du domaine transmembranaire et de l'ectodomaine d'au moins une protéine d'enveloppe d'un isolat du virus de l'hépatite C (HCV), ou
   - d'une séquence d'acides aminés présentant un pourcentage d'identité d'au moins 78%, notamment d'au moins 80%, particulièrement d'au moins 85%, et plus particulièrement d'au moins 90%, avec ladite séquence d'acides aminés du domaine transmembranaire et de l'ectodomaine d'une protéine d'enveloppe d'un isolat du virus de l'hépatite C, sous réserve que ladite séquence d'acides aminés conserve les propriétés dudit domaine transmembranaire et les propriétés immunogènes vis-a-vis du virus de l'hépatite C , ou
   - de la séquence d'acides aminés d'un variant synthétique dérivé de ladite séquence d'acides aminés du domaine transmembranaire et de l'ectodomaine de la protéine E1 ou E2 de HCV, sous réserve que ladite séquence d'acides aminés conserve les propriétés dudit domaine transmembranaire et les propriétés immunogènes vis-à-vis du virus de l'hépatite C, ladite protéine d'enveloppe d'un isolat du virus de l'hépatite C étant choisie parmi la protéine E1, la protéine E2 ou un peptide de fusion comprenant la protéine E1 et la protéine E2.

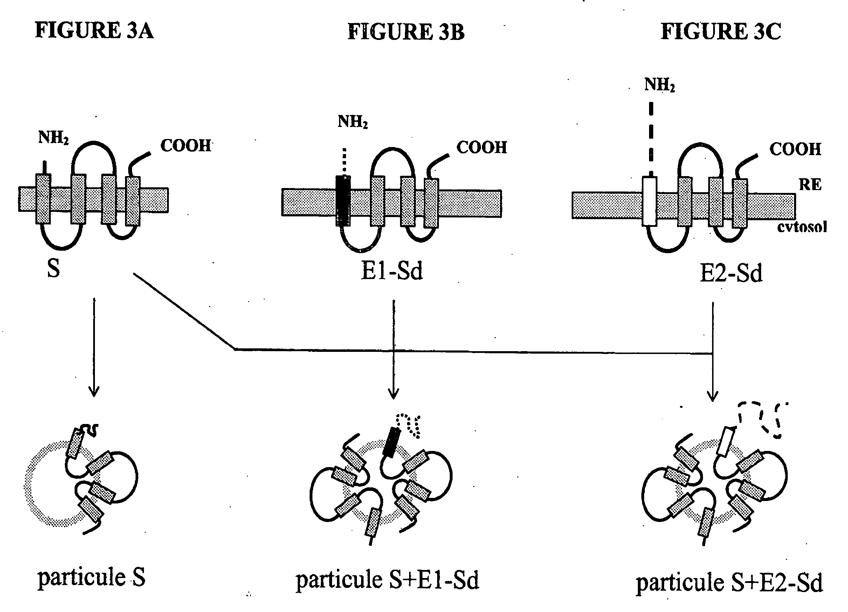

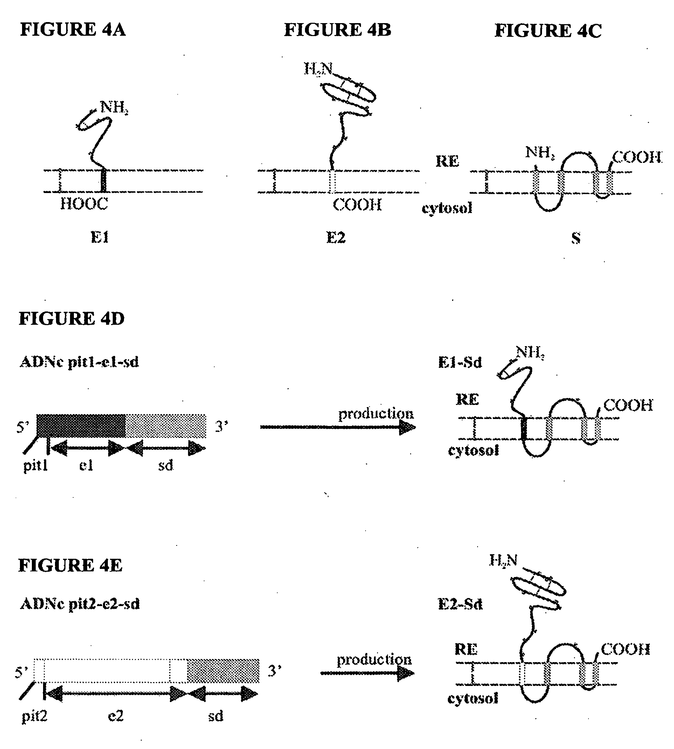

On entend par « **protéine de fusion** », toute protéine comprenant au moins la protéine S délétée de son domaine transmembranaire situé à son extrémité N-terminale d'un isolat du HBV (Sd), et la quasi-intégralité d'une protéine d'enveloppe E1 et/ou E2 d'un isolat du HCV, dont le domaine transmembranaire se substitue à celui délété en N-terminal de S du HBV (Figures 3B, 3C, 4D et 4E).

On entend par « **protéine immunogène** », toute protéine, notamment toute protéine de fusion selon l'invention ainsi que tout fragment de toute protéine de fusion décrit ici, dotée de propriétés antigéniques et capable d'induire une réaction ou une réponse immunitaire. En particulier, une protéine est considérée comme immunogène envers HCV et/ou HBV, si elle induit respectivement après immunisation une réponse humorale anti-E1, anti-E2 et/ou anti-S, détectée par exemple selon un protocole décrit :
- par Huzly *et al*., 2008, en ce qui concerne HBV, [Huzly D, Schenk T, Jilg W, Neumann-Haefelin D. Comparison of nine commercially available assays for quantification of antibody response to hepatitis B virus surface antigen. J Clin Microbiol. 2008 Apr, 46(4):1298-306], ou,
- par Hamed *et al*., 2008, en ce qui concerne HCV, [Hamed MR, Tarr AW, McClure CP, Ball JK, Hicklirig TP, Irving WL. Association of antibodies to hepatitis C virus glycoproteins 1 and 2 (anti-E1E2) with HCV disease. J Viral Hepat. 2008 May, 15(5):339-45].

On entend par « **capacité à former des particules sous-virales** », l'aptitude de toute protéine, notamment la protéine S du HBV, particulièrement de la protéine S délétée de son domaine transmembranaire N-terminal, et plus particulièrement d'une protéine de fusion de l'invention comprenant la protéine S délétée en N-terminal, à s'assembler en présence de la protéine S sauvage ou, le cas échéant, à s'auto-assembler, en particules sous-virales filamenteuses ou sphériques ; la dite capacité à former des particules sous-virales pouvant au moins être mise en évidence par observation selon notamment une analyse en microscopie électronique, et notamment par le test décrit dans l'exemple 1 et 2 (§I-5 et §II-7) de ce document et notamment illustré par les figures 7D, 7G, 11B à 11D.

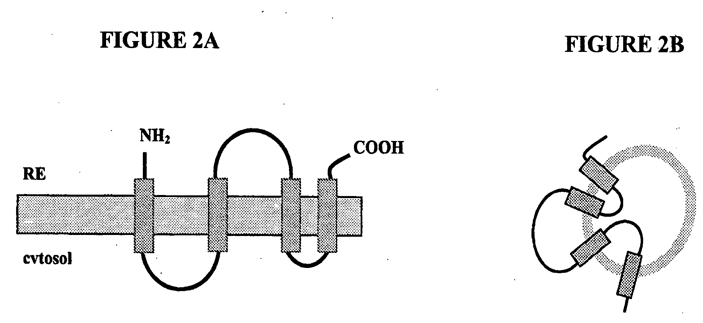

On entend par **« particule sous-virale non infectieuse** », toute particule filamenteuse ou sphérique, résultant de l'assemblage de la protéine S sauvage du HBV (figure 2B) et/ou de la protéine S délétée de son domaine transmembranaire N-terminal, ladite particule étant dépourvue du génome viral, et notamment synthétisée et sécrétée en très large excès, et qui peut être analysé par tout protocole permettant de mettre en évidence l'absence de fragments nucléotidiques spécifiques de HBV ou de HCV, tel que notamment, par l'amplification par PCR antérieurement décrite par exemple:
- en ce qui concerne HCV, par [Halfon P, Bourlière M, Ouzan D, Sène D, Saadoun D, Khiri H, Pénaranda G, Martineau A, Oulès V, Cacoub P.Occult HCV infection revisited with ultra-sensitive real-time PCR assay. J. Clin. Microbiol. 2008 Apr 30],
- en ce qui concerne HCV, par [Thibault V, Pichoud C, Mullen C, Rhoads J, Smith JB, Bitbol A, Thamm S, Zoulim F. Characterization of a new sensitive PCR assay for quantification of viral DNA isolated from patients with hepatitis B virus infections. J. Clin. Microbiol. 2007 Dec. 45(12):3948-53],
et dont le résultat est négatif.

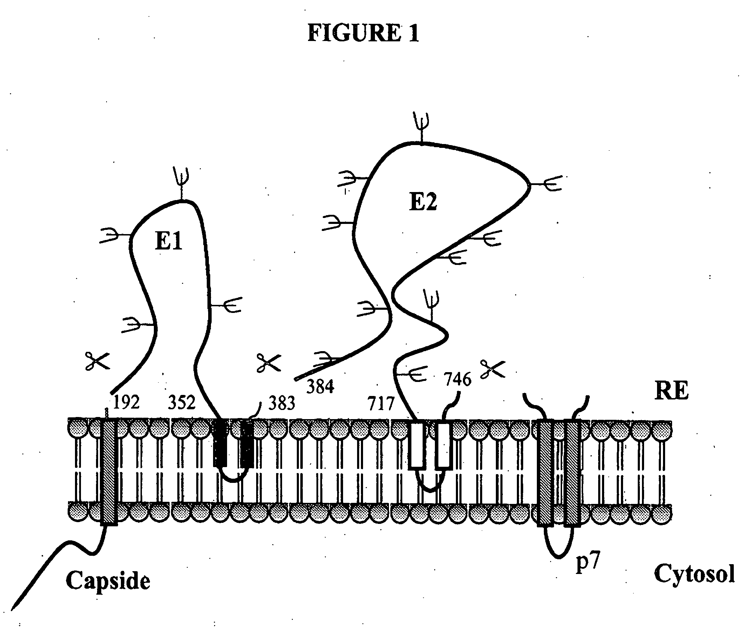

On entend par « **isolat du virus HCV** », tout isolat membre de la famille des *Flaviviridae* et appartenant au genre *Hepacivirus* et constitué d'une polyprotéine de plus de 3 000 acides aminés du virus HCV, schématisée dans la figure 1 [Choo, Q. L., Kuo, G., Weiner, A. J., Overby, L. R., Bradley, D. W., and Houghton, M (1989). Isolation of a cDNA clone derived from a blood-borne non-A, non-B viral hepatitis genome. Science 244(4902), 359-362 ; Choo et al. Proc. Natl. Acad. Sci. USA (1991) v88:2451-2455; Han et al. Characterization of the terminal regions of hepatitis C viral RNA: identification of conserved sequences in the 5' untranslated region and poly(A) tails at the 3' end. Proc. Natl. Acad. Sci. USA (1991) 88:1711-1715]; ou tout isolat classifié par l'International Committee for the Taxonomy of Viruses (ICTV) comme étant apparenté au HCV.

On entend par « **isolat du virus HBV** », tout isolat membre de la famille des *Hepadnaviridae* et appartenant au genre *Orthohepadnavirus*, ou tout isolat classifié par l'International Committee for the Taxonomy of Viruses (ICTV) comme étant apparenté au HBV [Schaefer S. Hepatitis B virus taxonomy and hepatitis B virus genotypes. World J Gastroenterol. 2007 Jan 7: 13(1):14-21].

On entend par « **protéines S** » ou par « **protéine S sauvage »** (S) :
- la protéine d'enveloppe d'un isolat du virus HBV comprenant notamment 226 acides aminés et comportant quatre domaines transmembranaires (Figure 2A), et notamment la protéine d'enveloppe de l'isolat HBV adw, ou
- le variant naturel issu d'un isolat du virus HBV, ou le variant synthétique de la protéine susmentionnée.

On entend par « **assemblage** » la capacité d'une protéine à former des particules sous-virales en s'associant à la protéine S sauvage. On entend par « auto assemblage » la capacité d'une protéine à former par elle-même des particules sous-virales.

On entend par « **protéine S délétée de son domaine transmembranaire situé à son extrémité N-terminale »** ou par « **protéine S délétée en N-terminal** » ou par « **protéine S délétée** » (Sd):
- la protéine S susdéfinie, délétée de la région comprise de l'acide aminé en position 1 à celui en position 19, ou en position 1 à celui en position 20, ou en position 1 à celui en position 21, et préférentiellement en position 1 à celui en position 22 de la protéine S d'un isolat du virus HBV, ou
- la protéine S susmentionnée présentant un pourcentage d'identité d'au moins 91%, notamment d'au moins 93%, particulièrement d'au moins 95%, et plus particulièrement d'au moins 97%, avec ladite région de la protéine S délétée, ou
- le « variant naturel » issu d'un isolat du virus HBV, ou le « variant synthétique » de la protéine S délétée susmentionnée.

On entend par « **protéine d'enveloppe d'un isolat du virus de l'hépatite C** », la quasi-intégralité de l'une des deux protéines E1 et E2, constituées de leur ectodomaine et de leur domaine transmembranaire (Figure 4B et 4C), et, correspondant :
* aux fragments peptidiques de la polyprotéine du virus HCV, et notamment de l'isolat HCV-la du virus HCV, localisés dans les régions comprises:
   - de l'acide aminé en position 192 à celui en position 383, ou notamment de l'acide aminé en position 192 à celui en position 380, en ce qui concerne la protéine E1, et,
   - de l'acide aminé en position 384 à celui en position 746, ou notamment de l'acide aminé en position 384 à celui en position 743, en ce qui concerne la protéine E2; ou
* les fragments présentant un pourcentage d'identité d'au moins 75%, notamment d'au moins 80%, particulièrement d'au moins 85%, et plus particulièrement d'au moins 90%, avec ladite région E1 susmentionnée, ou
* les fragments présentant un pourcentage d'identité d'au moins 78%, notamment d'au moins 80%, particulièrement d'au moins 85%, et plus particulièrement d'au moins 90%, avec ladite région E2 susmentionnée, ou
* les variants naturels ou à des variants synthétiques de la polyprotéine d'un isolat du virus HCV.

On entend par « **ectodomaine des protéines d'enveloppe du virus de l'hépatite C** »,
* les parties de fragments peptidiques de E1 ou E2, issues de la polyprotéine du virus HCV, et notamment de l'isolat HCV-1a du virus HCV, localisés dans les régions comprises:
   - de l'acide aminé en position 192 à celui en position 352, ou notamment de l'acide aminé en position 192 à celui en position 352, en ce qui concerne l'ectodomaine de la protéine E1, et,
   - de l'acide aminé en position 384 à celui en position 717, ou notamment de l'acide aminé en position 384 à celui en position 717, en ce qui concerne l'ectodomaine de la protéine E2 ; ou
* à des fragments présentant un pourcentage d'identité d'au moins 75%, notamment d'au moins 80%, particulièrement d'au moins 85%, et plus particulièrement d'au moins 90%, avec ladite région E1 susmentionnée, ou
* à des fragments présentant un pourcentage d'identité d'au moins 78%, notamment d'au moins 80%, particulièrement d'au moins 85%, et plus particulièrement d'au moins 90%, avec ladite région E2 susmentionnée, ou
* à des variants naturels ou à des variants synthétiques de la polyprotéine d'un isolat du virus HCV.

On entend par « **domaines transmembranaires des protéines d'enveloppe du virus de l'hépatite C** »,
* les parties de fragments peptidiques de E1 ou E2, issues de la polyprotéine du virus HCV, et notamment de l'isolat HCV-1a du virus HCV, localisés dans les régions comprises :
   - de l'acide aminé en position 353 à celui en position 383, et avantageusement de l'acide aminé en position 353 à celui en position 380, en ce qui concerne le domaine transmembranaire de la protéine E1, et,
   - de l'acide aminé en position 718 à celui en position 746 et avantageusement de l'acide aminé en position 718 à celui en position 743, en ce qui concerne le domaine transmembranaire de la protéine E2 ; ou
* les fragments présentant un pourcentage d'identité d'au moins 75%, notamment d'au moins 80%, particulièrement d'au moins 85%, et plus particulièrement d'au moins 90%, avec ladite région E1 susmentionnée, ou
* les fragments présentant un pourcentage d'identité d'au moins 78%, notamment d'au moins 80%, particulièrement d'au moins 85%, et plus particulièrement d'au moins 90%, avec ladite région E2 susmentionnée, ou
*** les variants naturels ou variants synthétiques** de la polyprotéine d'un isolat du virus HCV.

On entend par « **pourcentage d'identité** », le pourcentage déterminé par comparaison directe de deux séquences de molécules polypeptidiques, en déterminant le nombre de résidus d'acides aminés aux deux séquences, puis en le divisant par le nombre de résidus d'acides aminées de la séquence la plus longue des deux, et en multipliant le résultat par 100.

Un objet de la présente invention porte sur une protéine de fusion telle que revendiquée et comportant toute séquence peptidique d'un quelconque isolat du virus HCV et/ou du virus HBV, et ce quel que soit le pourcentage d'identité susmentionné de ladite séquence au regard des séquences spécifiques mentionnées ici.

Le terme **« variant naturel** » fait référence à toute variabilité, tout polymorphisme, toute diversité, d'une séquence d'ADN, d'un allèle, ou d'une séquence de protéine, entre isolats d'une même espèce ou d'une même population. On détermine le « **pourcentage de variabilité naturelle** » par comparaison directe de deux molécules polypeptidiques, ou polynucléotidiques, dérivé d'une molécule de référence sauvage et dotées de propriétés biologiques d'intérêt, telles que des propriétés immunogènes et/ou la capacité à former des particules sous-virales. Il se quantifie en déterminant le nombre exact de résidus d'acides aminés, ou d'acides nucléiques, identiques entre les deux séquences, puis en les divisant par le nombre de résidus d'acides aminés, ou d'acides nucléiques, de la séquence la plus courte des deux, et en multipliant le résultat par 100.

Ledit pourcentage de variabilité entre deux séquences est particulièrement versatile car il dépend notamment du virus considéré, du génotype considéré, du fragment de séquence considéré - la région de l'ectodomaine du HCV étant par exemple plus variable que la région du domaine transmembranaire -, etc... Ainsi, le pourcentage de variabilité des protéines E1 et E2 du HCV, est de 88% en nucléotide, et de 90% en acides aminés, entre souches d'un même génotype. Mais elle tombe à 55% en nucléotide, et à 59% en acides aminés, entre souches de différents génotypes. HBV étant un virus à ADN, il est beaucoup moins variable que le HCV [Zhang M, Gaschen B, Blay W, Foley B, Haigwood N, Kuiken C, Korber B. Tracking global patterns of N-linked glycosylation site variation in highly variable viral glycoproteins: HIV, SIV, and HCV envelopes and influenza hemagglutinin. Glycobiology. 2004 Dec. 14(12):1229-46].

Un objet de la présente invention porte sur une protéine de fusion, et/ou une molécule d'acides nucléiques hybride, telle que revendiquée et comportant tout variant naturel ou tout fragment de variant naturel, dotée d'une séquence peptidique et/ou nucléotidique issue d'un quelconque isolat du virus HCV et/ou du virus HBV humain, et ce quel que soit le pourcentage de variabilité naturelle et/ou synthétique, susmentionnée de ladite séquence au regard des séquences spécifiques décrites ici.

Le terme **« variant synthétique** » fait référence à toute molécule polypeptidique, ou polynucléotidique selon l'invention, ou tout fragment de molécule polypeptidique, ou polynucléotidique décrit ici, dérivé par recombinaison d'une molécule sauvage de référence, soit par adition, délétion, substitution, à ladite molécule sauvage de référence, sous réserve qu'elle conserve les propriétés biologiques d'intérêt, telles que des propriétés immunogènes et/ou la capacité à former des particules sous-virales. On détermine le « **pourcentage de variabilité synthétique** » par comparaison directe de ladite molécule dérivé à ladite molécule sauvage de référence, et en déterminant le nombre exact de résidus d'acides aminés, ou d'acides nucléiques, identiques entre les deux séquences, au regard de leur position et de leur nature, puis en les divisant par le nombre de résidus d'acides aminés, ou d'acides nucléiques, de la séquence la plus courte des deux, et en multipliant le résultat par 100.

Un objet de la présente invention porte sur une protéine de fusion telle que revendiquée et comportant tout variant synthétique ou tout fragment de variant synthétique, doté d'une séquence nucléotidique et/ou peptidique dérivé d'un quelconque isolat du virus HCV et/ou du virus HBV.

Selon un aspect particulièrement avantageux de l'invention, les domaines transmembranaires de E1 et/ou de E2, et constituant une partie de la protéine de fusion, sont délétés d'au moins l'un des trois derniers acides aminés, et notamment des trois derniers acides aminés, situés en position C-terminale, de sorte que lesdits domaines transmembranaires délétés de E1 et/ou de E2 présentent un pourcentage d'identité avec les domaines transmembranaires de E1 et/ou E2 sauvages, d'au moins 91%, en ce qui concerne E1 et d'au moins 90%, en ce qui concerne E2.

Ladite délétion d'au moins l'un des trois acides aminés, et notamment des trois derniers acides aminés, en position C-terminale présente l'avantage d'inactiver le site de clivage des peptidases représenté sur la figure 1 par le symbole 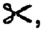 et qui est nécessaire à la maturation de la polyprotéine du HCV, mais qui est indésirable dans le cadre des constructions chimériques de la présente invention.
[1]. L'invention a pour objet une protéine de fusion immunogène comprenant au moins les deux peptides suivants:
   a)- du côté C-terminal, un premier peptide constitué:
      - de la séquence d'acides aminés de la protéine S d'un isolat du virus humain de l'hépatite B (HBV), laquelle protéine S est délétée de son domaine transmembranaire situé à son extrémité N-terminale, ladite séquence d'acides aminés conservant la capacité à former des particules sous-virales non-infectieuses immunogènes vis-à-vis du virus humain de l'hépatite B, ou
      - d'une séquence d'acides aminés présentant un pourcentage d'identité d'au moins 91%, notamment d'au moins 93%, particulièrement d'au moins 95%, et plus particulièrement d'au moins 97%, avec ladite séquence d'acides aminés de la protéine S délétée en N-terminal, sous réserve que ladite séquence d'acides aminés soit aussi délétée du domaine transmembranaire situé à l'extrémité N-terminale de la protéine S de HBV, et qu'elle conserve la capacité à former des particules sous-virales non infectieuses, immunogènes vis-à-vis du virus humain de l'hépatite B, ou,
      - de la séquence d'acides aminés d'un variant synthétique dérivé de ladite séquence d'acides aminés de la protéine S délétée, sous réserve que ladite séquence d'acides aminés dudit variant synthétique soit aussi délétée du domaine transmembranaire situé à l'extrémité N-terminale de la protéine S de HBV, et qu'elle conserve la capacité à former des particules sous-virales non infectieuses, immunogènes vis-à-vis du virus humain de l'hépatite B, et,
   b)- du côté N-terminal, un second peptide constitué :
      - de la séquence d'acides aminés du domaine transmembranaire et de l'ectodomaine d'au moins une protéine d'enveloppe d'un isolat du virus de l'hépatite C (HCV), ou
      - d'une séquence d'acides aminés présentant un pourcentage d'identité d'au moins 78%, notamment d'au moins 80%, particulièrement d'au moins 85%, et plus particulièrement d'au moins 90%, avec ladite séquence d'acides aminés du domaine transmembranaire et de l'ectodomaine d'une protéine d'un isolat du virus de l'hépatite C, sous réserve que ladite séquence d'acides aminés conserve les propriétés dudit domaine transmembranaire, la capacité à former des particules sous-virales non infectieuses, et les propriétés immunogènes vis-à-vis du virus de l'hépatite C , ou
      - de la séquence d'acides aminés d'un variant synthétique dérivé de ladite séquence d'acides aminés du domaine transmembranaire et de l'ectodomaine de la protéine E1 ou E2 de HCV, sous réserve que ladite séquence d'acides aminés conserve les propriétés dudit domaine transmembranaire et les propriétés immunogènes vis-à-vis du virus de l'hépatite C, ladite protéine d'enveloppe d'un isolat du virus de l'hépatite C étant choisie parmi la protéine E1, la protéine E2 ou un peptide de fusion comprenant la protéine E1 et la protéine E2.

   L'objet de la présenté invention porte notamment sur une protéine de fusion telle que revendiquée et comprenant au moins :
   - d'une part la protéine S de HBV délétée (Sd), essentiellement constitué de ses trois domaines transmembranaires situés à l'extrémité C-terminale (figures 2A et 3A), ladite protéine S délétée conservant la capacité d'assemblage en particules sous-virales, et conservant les propriétés immunogènes contre le virus HBV humain, et
   - d'autre part la quasi-intégralité de la séquence d'une des protéines E1 et E2, conservant également les propriétés immunogènes contre le virus HCV,
   de sorte que ladite protéine de fusion, notamment représentée par les figures 3B ou 3C, soit capable de s'assembler en particules sous-virales, en présence de la protéine S sauvage, et soit susceptible d'induire une immunisation contre les virus HBV et/ou HCV, et notamment d'induire une double immunisation contre les virus HBV et HCV.
[1a]. Avantageusement, la présente invention a pour objet une protéine de fusion immunogène susmentionnée, comprenant à l'extrémité N-terminale dudit second peptide (E1 ou E2), un troisième peptide constitué de la séquence d'acide aminés d'un peptide d'initiation de transfert (PIT) d'un isolat du virus de l'hépatite C.
   On entend par « **peptide d'initiation de transfert** » d'une protéine E1 ou E2 (respectivement PIT1 et PIT2) ou d'une protéine de fusion selon l'invention,
   * le fragment peptidique de la polyprotéine du virus HCV, et notamment de l'isolat HCV-1a, localisé dans la région comprise :
      - de l'acide aminé en position 166 à celui en position 191 en ce qui concerne la protéine E1, et,
      - de l'acide aminé en position 366 à celui en position 383 en ce qui concerne la protéine E2 ; ou
   * le variant naturel issu d'un isolat du virus HCV, ou le variant synthétique dudit fragment peptidique susmentionné, ou
   * tout fragment peptidique greffé en N-terminal dudit second peptide,
      sous réserve que ledit fragment peptidique, quand il constitue une partie de la protéine de fusion de la présente invention, conserve la capacité à adresser, après traduction, ladite protéine de fusion au réticulum endoplasmique de sorte qu'elle soit correctement glycosyléè et que sa conformation tridimentionnelle, et/ou que ses qualités antigéniques, soient au mieux préservées au regard des protéines sauvages.
[2]. Selon un autre aspect particulièrement avantageux, la présente invention a pour objet une protéine de fusion immunogène susmentionnée, dans laquelle le second peptide situé du côté N-terminal, est ccnstitué :
   - de la totalité de la séquence d'acides aminés du domaine transmembranaire et de l'ectodomaine de la protéine d'enveloppe E1, ou
   - d'une séquence d'acides aminés présentant un pourcentage d'identité d'au moins 75%, notamment d'au moins 80%, particulièrement d'au moins 85%, et plus particulièrement d'au moins 90%, avec ladite séquence d'acides aminés du domaine transmembranaire et de l'ectodomaine de la protéine d'enveloppe E1, sous réserve que ladite séquence d'acides aminés conserve les propriétés dudit domaine transmembranaire et les propriétés immunogènes vis-à-vis du virus de l'hépatite C, ou
   - de la séquence d'acides aminés
   d'un variant synthétique dérivé de ladite protéine E1; sous réserve que ladite séquence d'acides aminés conserve les propriétés dudit domaine transmembranaire et les propriétés immunogènes vis-à-vis du virus de l'hépatite C.
   Un objet particulier de l'invention réside dans la protéine de fusion E1-Sd, dont on trouve une représentation schématique dans la figure 3B, et qui comprend la quasi-intégralité de la protéine E1 du HCV, greffée en N-terminal de la protéine S délétée de HBV.
[3]. Avantageusement, la présente invention a particulièrement pour objet une protéine de fusion immunogène susmentionnée dans laquelle le second peptide situé du côté N-terminal, est constitué :
   - de la totalité de la séquence d'acides aminés du domaine transmembranaire et de l'ectodomaine de la protéine d'enveloppe E2, ou
   - d'une séquence d'acides aminés présentant un pourcentage d'identité d'au moins 78%, notamment d'au moins 80%, particulièrement d'au moins 85%, et plus particulièrement d'au moins 90%, avec ladite séquence d'acides aminés du domaine transmembranaire et de l'ectodomaine de la protéine d'enveloppe E2, sous réserve que ladite séquence d'acides aminés conserve les propriétés dudit domaine transmembranaire et les propriétés immunogènes vis-à-vis du virus de l'hépatite C, ou
   - de la séquence d'acides aminés d'un variant synthétique dérivé de ladite protéine E2, sous réserve que ladite séquence d'acides aminés conserve les propriétés dudit domaine transmembranaire et les propriétés immunogènes vis-à-vis du virus de l'hépatite C.

   Un objet particulier de l'invention réside dans la protéine de fusion E2-Sd, dont on trouve une représentation schématique dans la figure 3C, et qui comprend la quasi-intégralité de la protéine E2 du HCV, greffée en N-terminal de la protéine S délétée de HBV.
[3b]. A ce titre, l'invention porte plus particulièrement sur une protéine de fusion immunogène telle que revendiquée et, comprenant les trois peptides suivants:
   a)- du côté C-terminal, un premier peptide constitué :
      - de la séquence d'acides aminés de la protéine S délétée de son domaine transmembranaire de l'extrémité N-terminale d'un isolat du virus humain de l'hépatite B ladite séquence d'acides aminés conservant la capacité à former des particules sous-virales non-infectieuses immunogènes vis-à-vis du virus humain de l'hépatite B, ou
      - d'une séquence d'acides aminés présentant un pourcentage d'identité d'au moins 91%, notamment d'au moins 93%, particulièrement d'au moins 95%, et plus particulièrement d'au moins 97%, avec ladite séquence d'acides aminés de la protéine S délétée en N-terminal, sous réserve que ladite séquence d'acides aminés soit aussi délétée du domaine transmembranaire situé à l'extrémité N-Teterminale de la protéine S de HBV, et qu'elle conserve la capacité à former des particules sous-virales non infectieuses, immunogènes vis-à vis du virus humain de l'hépatite B, ou
      - de la séquence d'acides aminés d'un variant synthétique dérivé de ladite la séquence d'acides aminés de la protéine S délétée en N-terminal, sous réserve que ladite séquence d'acides aminés soit aussi délétée du domaine transmembranaire situé à l'extrémité N-Teterminale de la protéine S de HBV, et qu'elle conserve la capacité à former des particules sous-virales non infectieuses, immunogènes vis-àvis du virus humain de l'hépatite B ;
   b)- un second peptide de séquence constitué :
      - de la totalité de la séquence d'acides aminés du domaine transmembranaire et de l'ectodomaine d'une protéine d'enveloppe E2 d'un isolat du virus de l'hépatite C, ou
      - d'une séquence d'acides aminés présentant un pourcentage d'identité d'au moins 78%, notamment d'au moins 80%, particulièrement d'au moins 85%, et plus particulièrement d'au moins 90%, avec ladite séquence d'acides aminés du domaine transmembranaire et de l'ectodomaine de ladite protéine d'enveloppe, sous réserve que ladite séquence d'acides aminés conserve les propriétés dudit domaine transmembranaire et les propriétés immunogènes vis-à-vis du virus de l'hépatite C, ou
      - de la séquence d'acides aminés d'un variant synthétique dérivé de ladite la séquence d'acides aminés de la protéine E2, sous réserve que ladite séquence d'acides aminés conserve les propriétés dudit domaine transmembranaire et les propriétés immunogènes vis-à-vis du virus de l'hépatite C, et,
   c)- du côté N-terminal, un troisième peptide de séquence constituée
      - de la totalité de la séquence d'acides aminés du domaine transmembranaire et de l'ectodomaine d'une protéine d'enveloppe E1, d'un isolat du virus de l'hépatite C, ou
      - d'une séquence d'acides aminés présentant un pourcentage d'identité d'au moins 75%, notamment d'au moins 80%, particulièrement d'au moins 85%, et plus particulièrement d'au moins 90%, avec ladite séquence d'acides aminés du domaine transmembranaire et de l'ectodomaine de ladite protéine d'enveloppe, sous réserve que ladite séquence d'acides aminés conserve les propriétés dudit domaine transmembranaire et les propriétés immunogènes vis-à-vis du virus de l'hépatite C, ou ,
      - de la séquence d'acides aminés
      d'un variant synthétique, dérivé de ladite la séquence d'acides aminés d'une protéine E1, sous réserve que ladite séquence d'acides aminés conserve les propriétés dudit domaine transmembranaire et les propriétés immunogènes vis-à-vis du virus de l'hépatite C,
   ledit second peptide étant situé entre le premier et le troisième peptide, le premier, second et troisième peptides étant de préférence contigus.
   Un objet particulier de l'invention réside dans la protéine de fusion E1-E2-Sd, qui comprend l'ectodomaine de E1 du HCV greffée en N-terminal de la quasi-intégralité de la protéine E2, elle même greffée en N-terminal de la protéine S délétée de HBV.
   Un objet particulier de l'invention réside également dans la protéine de fusion E2-E1-Sd, et qui comprend l'ectodomaine de E2 du HCV greffée en N-terminal de la quasi-intégralité de la protéine E1, elle même greffée en N-terminal de la protéine S délétée de HBV.
[4]. Selon un aspect particulièrement avantageux de l'invention, le premier et le second peptide constituant la protéine de fusion immunogène sont contigus, et l'extrémité C-terminale du second peptide est liée de façon covalente à l'extrémité N-terminale du premier peptide.
   Avantageusement, selon l'invention, les protéines E1 ou E2 du virus HCV, ou les fragments d'une protéine de fusion de l'invention PIT1-E1, PIT2-E2, E1-E2, ou PIT1-E1-E2 sont liés de façon covalente et contigüe à la protéine S délétée du virus HBV.
   Selon un autre aspect avantageux de l'invention, un peptide de liaison relie le premier et le second peptide constituant la protéine de fusion susmentionnée, le dit peptide de liaison étant constitué de l'acide aminé, ou de 2 acides aminés, ou de 3 acides aminés, ou de 4 acides aminés, ou de 5 acides aminés; sous réserve que ladite protéine de fusion immunogène conserve la capacité à s'assembler en particules sous-virales, en présence de la protéine S sauvage, et les propriétés immunogènes vis-à-vis du virus HCV, ou du virus HBV, ou, des virus HCV et HBV.
[5]. L'invention a également pour objet particulier, une protéine de fusion immunogène susmentionnée, dans laquelle le premier peptide en position C-terminale est constitué :
   - d'une séquence d'acides aminés délimitée par les acides aminés contigus situés dans la région comprise de l'acide aminé en position 23 à celui en position 226 de la protéine S du virus HBV, et particulièrement de l'isolat HBV adw,
      et notamment de la séquence d'acides aminés représentée par la SEQ ID N°2, ladite séquence d'acides aminés conservant la capacité à former des particules sous-virales non-infectieuses immunogènes vis-à-vis du virus humain de l'hépatite B, ou
   - d'une séquence d'acides aminés présentant un pourcentage d'identité d'au moins 91%, notamment d'au moins 93%, particulièrement d'au moins 95%, et plus particulièrement d'au moins 97%, avec ladite séquence d'acides aminés contigus de la protéine S, sous réserve que ladite séquence d'acides aminés soit aussi délétée du domaine transmembranaire situé à l'extrémité N-terminale de la protéine S de HBV, et qu'elle conserve la capacité à former des particules sous-virales non infectieuses, immunogènes vis-à-vis du virus humain de l'hépatite B, ou
   - de la séquence d'acides aminés d'un variant synthétique, dérivé de ladite séquence d'acides aminés de la protéine S, sous réserve que ladite séquence d'acides aminés dudit variant naturel ou synthétique soit aussi délétée du domaine transmembranaire situé à l'extrémité N-terminale de la protéine S de HBV, et qu'elle conserve la capacité à former des particules sous-virales non infectieuses, immunogènes vis-à-vis du virus humain de l'hépatite B.

   Un objet plus particulier de l'invention réside dans la protéine de fusion E1-Sd ou E2-Sd, ou E1-E2-Sd, pour laquelle protéine S délétée est celle de l'isolat HBVadw et a pour séquence la SEQ ID N°2 (cf tableau 1).
[6]. L'invention a également pour objet particulier une protéine de fusion immunogène susmentionnée, dans laquelle le second peptide en position N-terminale est constitué :
   - d'une séquence d'acides aminés du domaine transmembranaire et de l'ectodomaine d'une protéine d'enveloppe d'un isolat du virus de l'hépatite C (HCV) et délimitée par les acides aminés contigus situés dans la région comprise de l'acide aminé en position 192 à celui en position 380 dé la protéine E1 de HCV, et particulièrement de l'isolat HCV-1a, et notamment de la séquence d'acides aminés représentée par la SEQ ID N°4, ou
   - d'une séquence d'acides aminés présentant un pourcentage d'identité d'au moins 75%, notamment d'au moins 80%, particulièrement d'au moins 85%, et plus particulièrement d'au moins 90%, avec ladite séquence d'acides aminés contigus de ladite protéine E1, sous réserve que ladite séquence d'acides aminés conserve les propriétés dudit domaine transmembranaire et les propriétés immunogènes vis-à-vis du virus de l'hépatite C, ou
   - de la séquence d'acides aminés d'un variant synthétique dérivé de ladite séquence d'acides aminés contigus du domaine transmembranaire et de l'ectodomaine de ladite protéine E1, sous réserve que ladite séquence d'acides aminés conserve les propriétés dudit domaine transmembranaire et les propriétés immunogènes vis-à-vis du virus de l'hépatite C.

   L'invention a également pour objet particulier, une protéine de fusion pour laquelle la séquence de protéine E1 est issue de l'isolat HCV-1a, et correspond spécifiquement à la région susmentionnée, telle que notamment les protéines de fusion E1-Sd de SEQ ID N°4 ou E1-E2-Sd, PIT1-E1- E2-Sd (cf tableau 1). [7]. L'invention porte particulièrement sur une protéine de fusion immunogène susmentionnée, dans laquelle le premier et le second peptide sont contigus, ladite protéine de fusion étant constituée par :
   - la séquence d'acides aminés représentée par la SEQ ID N°8, ou
   - une séquence d'acides aminés présentant un pourcentage d'identité d'au moins 88%, notamment d'au moins 90%, particulièrement d'au moins 92%, et plus particulièrement d'au moins 95%, avec SEQ ID N°8, sous réserve que ladite séquence d'acides aminés soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis de HCV et/ou vis-à-vis de HBV humain, ou
   - de la séquence d'acides aminés d'un variant synthétique dérivé de ladite SEQ ID N°8, sous réserve que ladite séquence d'acides aminés dudit variant synthétique soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis de HCV et/ou vis-à-vis de HBV humain.

   L'invention a également pour objet une protéine de fusion E1-Sd pour laquelle la séquence de protéine E1 est issue de l'isolat HCV-1a, et est dotée de la séquence SEQ ID N°8 susmentionnée, ladite séquence correspondant à la SEQ ID N°2 de Sd, greffée en amont de la SEQ ID N°4 de E1.
[7b]. A ce titre, l'invention a plus particulièrement pour objet une protéine de fusion immunogène susmentionnée, comprenant un troisième peptide d'initiation de transfert situé du côté N-terminal du second peptide, ladite protéine de fusion étant représentée par :
   - la SEQ ID N°12, ou
   - une séquence d'acides aminés présentant une homologie d'identité d'au moins 83%, notamment d'au moins 85%, particulièrement d'au moins 90%, et plus particulièrement d'au moins 95%, avec ladite SEQ ID N°12, sous réserve que ladite séquence d'acides aminés soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus de l'hépatite B humain et/ou vis-à-vis du virus de l'hépatite C, ou
   - de la séquence d'acides aminés d'un variant synthétique dérivé de ladite SEQ ID N °12, sous réserve que ladite séquence d'acides aminés dudit variant synthétique soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus de l'hépatite B humain et/ou vis-à-vis du virus de l'hépatite C.

   L'invention a également pour objet, protéine de fusion PIT1-E1-Sd pour laquelle la séquence de protéine E1 est issue de HCV, et particulièrement de l'isolat HCV-1a, et est dotée de la séquence SEQ ID N °12susmentionnée, ladite séquence correspondant à la SEQ ID N°2 de Sd, greffée en N-terminal de la SEQ ID N°4 de E1, elle même greffée en N-terminal de la séquence d'acides aminés du peptide d'initiation de transfert de la protéine E1 (PIT1) compris dans la région constituée de l'acide aminé en position 166 à celui en position 191 de HCV.
   L'insertion d'un peptide d'initiation de transfert en N-terminal de la protéine de fusion E1-Sd susmentionnée a pour avantage particulier d'adresser cette dernière une fois traduite au réticulum endoplasmique, de sorte qu'elle soit correctement glycosylée et que sa conformation tridimentionnelle et/ou que ses qualités antigéniques ne présentent pas d'altération substantielle au regard des protéines sauvages.
[8]. L'invention a également pour objet une protéine de fusion immunogène susmentionnée, dans laquelle le second peptide en position N-terminale est constitué :
   - d'une séquence d'acides aminés du domaine transmembranaire et de l'ectodomaine d'une protéine d'enveloppé d'un isolat du virus de l'hépatite C (HCV) et délimitée par les acides aminés contigus situés de l'acide aminé en position 384 à celui en position 743 de la protéine E2 de HCV, et particulièrement de l'isolat HCV-1a,
      et notamment de la séquence d'acides aminés représentée par la SEQ ID N°6, ou
   - d'une séquence d'acides aminés présentant un pourcentage d'identité d'au moins 78%, notamment d'au moins 80%, particulièrement d'au moins 85%, et plus particulièrement d'au moins 90%, avec ladite séquence d'acides aminés contigus de protéine E2, sous réserve que ladite la séquence d'acides aminés conserve dudit domaine transmembranaire et les propriétés immunogènes vis-à-vis du virus de l'hépatite C, ou
   - de la séquence d'acides aminés d'un variant synthétique, dérivé de ladite séquence d'acides aminés contigus du domaine transmembranaire et de l'ectodomaine de ladite protéine E2, sous réserve que ladite séquence d'acides aminés conserve les propriétés dudit domaine transmembranaire et les propriétés immunogènes vis-à-vis du virus de l'hépatite C,

   L'invention a également pour objet, la protéine de fusion E2-Sd ou E1-E2-Sd, pour laquelle la séquence de protéine E2 est issue du HCV, et particulièrement de l'isolat HCV-1a, et correspond spécifiquement à la région de la séquence susmentionnée de la protéine E2.
[9]. L'invention porte particulièrement sur une protéine de fusion immunogène susmentionnée, dans laquelle le premier et le second peptide sont contigus, ladite protéine de fusion étant constituée par :
   - la séquence d'acides aminés représentée par la SEQ ID N°10, ou
   - une séquence d'acides aminés présentant un pourcentage d'identité d'au moins 86%, notamment d'au moins 88%, particulièrement d'au moins 90%, et plus particulièrement d'au moins 95%, avec SEQ ID N°10, sous réserve que ladite séquence d'acides aminés soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus de l'hépatite B humain et/ou vis-à-vis du virus de l'hépatite C, ou
   - de la séquence d'acides aminés d'un variant synthétique dérivé de ladite SEQ ID N °10,
   sous réserve que ladite séquence d'acides aminés dudit variant synthétique soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus de l'hépatite B humain et/ou vis-à-vis du virus de l'hépatite C.
   L'invention a également pour objet la protéine de fusion E2-Sd pour laquelle la séquence de la protéine E2 est issue de l'isolat HCV-1a, et est dotée de la séquence SEQ ID N°10 susmentionnée, ladite séquence correspondant à la SEQ ID N°2 de Sd, greffée en amont de la SEQ ID N°6 de E2.
[9b]. A ce titre, l'invention a plus particulièrement pour objet une protéine de fusion immunogène susmentionnée (PIT2-E2-Sd), comprenant un troisième peptide d'initiation de transfert situé du côté N-terminal du second peptide, ladite protéine de fusion étant représentée par :
   - la SEQ ID N° 14, ou
   - une séquence d'acides aminés présentant une homologie d'identité d'au moins 82%, notamment d'au moins 85%, particulièrement d'au moins 90%, et plus particulièrement d'au moins 95%, avec ladite SEQ ID N°14, ou
   - de la séquence d'acides aminés d'un variant synthétique dérivé de ladite SEQ ID N ° 14, sous réserve que ladite SEQ ID N ° 14 soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus de l'hépatite B humain et/ou vis-à-vis du virus de l'hépatite C.

L'invention a également pour objet, la protéine de fusion PIT2-E2-Sd pour laquelle la séquence de protéine E2 est issue de l'isolat HCV-1a, et est dotée de la séquence susmentionnée SEQ ID N °14, ladite séquence correspondant à la SEQ ID N°2 de Sd, greffée en N-terminal de la SEQ ID N°6 de E2, elle même greffée en N-terminal de la séquence d'acides aminés du peptide d'initiation de transfert de la protéine E2 (PIT2) compris dans la région constituée de l'acide aminé en position 366 à celui en position 383 de HCV.

L'invention a également pour objet, la protéine de fusion E1-E2-Sd ou PIT1-E1-E2-Sd. L'invention a également pour objet, les protéines de fusion susmentionnées sous forme purifiée.

L'invention concerne également une molécule d'acides nucléiques hybride codant pour l'une quelconque des protéines de fusion susmentionnées.

On entend par le terme « **molécule d'acide nucléiques hybride** », toutes molécules d'acide nucléiques comprenant au moins une séquence codant pour la protéine S délétée de son domaine transmembranaire situé à son extrémité N-terminale d'un isolat du virus humain de l'hépatite B, et au moins une séquence codant pour la quasi-intégralité d'une protéine d'enveloppe d'un isolat du virus de l'hépatite C, ou toute molécule issue d'une molécule définie ci-dessus, et modifiée suite à la dégénérescence naturelle de son code génétique.

Avantageusement, l'invention porte sur une molécule d'acides nucléiques hybride susmentionnée, codant pour une protéine de fusion définie ci-dessus, comprenant les trois séquences d'acides nucléiques suivantes :
a)- du côté 3', une première séquence d'acides nucléiques codant pour la protéine S délétée de son domaine transmembranaire situé à son extrémité N-terminale, d'un isolat du virus humain de l'hépatite B, ou
   - d'une séquence d'acides nucléiques présentant une homologie d'au moins 91%, notamment d'au moins 93%, particulièrement d'au moins 95%, et plus particulièrement d'au moins 97%, avec ladite séquence d'acides nucléiques de la protéine S délétée en N-terminal, sous réserve que la protéine codée par ladite séquence d'acides nucléiques conserve la capacité à former des particules sous-virales non infectieuses, immunogènes vis-à-vis du virus humain de l'hépatite B, ou
   - de la séquence d'acides nucléiques d'un variant synthétique, dérivé d'une séquence d'acides nucléiques de ladite protéine S délétée en N-terminal, sous réserve que la protéine codée par ladite séquence d'acides nucléiques conserve la capacité à former des particules sous-virales non infectieuses, immunogènes vis-à-vis du virus humain de l'hépatite B, et,
b)- du côté 5' de la première séquence, une seconde séquence d'acides nucléiques codant pour le domaine transmembranaire et l'ectodomaine d'au moins une protéine, d'enveloppe E1 ou E2 d'un isolat du virus de l'hépatite C, ou
   - d'une séquence d'acides nucléiques présentant une homologie d'identité d'au moins 78%, notamment d'au moins 80%, particulièrement d'au moins 85%, et plus particulièrement d'au moins 90%, avec ladite séquence d'acides nucléiques codant pour l'une desdites protéines E1 ou E2 d'un isolat du virus de l'hépatite C, sous réserve que la protéine codée par ladite séquence d'acides nucléiques conserve les propriétés, dudit domaine transmembranaire et les propriétés immunogènes vis-à-vis du virus de l'hépatite C,
   - de la séquence d'acides nucléiques variant synthétique, dérivé de ladite séquence d'acides nucléiques d'une des dites protéines E1 ou E2, sous réserve que la protéine codée par ladite séquence d'acides nucléiques conserve les propriétés immunogènes vis-à-vis du virus de l'hépatite C, ladite seconde séquence étant choisie parmi les séquences codant pour la protéine E1, pour la protéine E2 ou pour un peptide de fusion comprenant la protéine E1 et la protéine E2, et,
c)- du côté 5' de la seconde séquence, une troisième séquence d'acides nucléiques codant pour un peptide d'initiation de transfert d'une protéine d'enveloppe E1 ou E2 d'un isolat du virus de l'hépatite C (PIT), ou
   - une séquence d'acides nucléiques d'un variant naturel issue d'un isolat du virus HCV, ou d'un variant synthétique, dérivé de ladite la séquence d'acides nucléiques de la protéine codant pour un peptide d'initiation de transfert (PIT), sous réserve que le peptide d'initiation de transfert codé par ladite la séquence d'acides nucléiques, conserve la capacité à adresser, après traduction, ladite protéine de fusion au réticulum endoplasmique de sorte qu'elle soit correctement glycosylée et que sa conformation tridimentionnelle présente le moins d'altération possible, et/ou que ses qualité antigéniques soient au mieux préservées au regard des protéines sauvages, ou,
   - toute séquence d'acides nucléiques codant pour un peptide capable d'adresser, après traduction, ladite protéine de fusion au réticulum endoplasmique de sorte qu'elle soit correctement glycosylée et que sa conformation tridimentionnelle présente le moins d'altérations possibles, et/ou que ses qualités antigéniques soient au mieux préservées au regard des protéines sauvages.

Avantageusement, la présente invention a pour objet une molécule d'acides nucléiques hybride susmentionnée (notamment les molécules e1-sd ou e2-sd, ou e1-e2-sd, pit1-e1-sd ou pit2-e2-sd, telles que définies dans le tableau 1 ci-dessous), codant pour une protéine de fusion susmentionnée (notamment E1-Sd ou E2-Sd, ou E1-E2-Sd, PIT1-E1-Sd ou PIT2-E2-Sd), comprenant respectivement de son extrémité 5' à son extrémité, 3', une séquence d'acides nucléiques codant pour un peptide d'initiation de transfert (PIT) d'un isolat du virus de l'hépatite C, une séquence d'acides nucléiques codant pour une protéine E1 ou E2 d'un isolat du virus HCV, une séquence d'acides nucléiques codant pour la protéine S délétée d'un isolat du virus HBV.

L'insertion d'une séquence d'acide nucléiques codant pour ledit peptide d'initiation de transfert greffé en N-terminal de la protéine de fusion susmentionnée (notamment E1-Sd ou E2-Sd, ou E1-E2-Sd) a pour avantage particulier que cette dernière une fois traduite est adressée au réticulum endoplasmique, de sorte qu'elle soit correctement glycosylée et que sa conformation tridimentionnelle et/ou que ses qualités antigéniques ne présentent pas d'altération substantielle au regard des protéines sauvages.

La présente invention a également pour objet une molécule d'acides nucléiques hybride susmentionnée ne comprenant que les deux premières séquences d'acides nucléiques susmentionnées et dépourvue de séquence d'acides nucléiques codant pour un peptide d'initiation de transfert.

L'objet de la présente invention porte notamment sur une molécule d'acides nucléiques hybride susmentionnée codant pour une protéine de fusion de l'invention, comprenant au moins :
- la séquence d'acides nucléiques codant pour la protéine S de HBV délétée (Sd), essentiellement constitué de ses trois domaines transmembranaires situés à l'extrémité C-terminale (figures 2A et 3A), ladite protéine S délétée conservant la capacité d'assemblage en particules sous-virales, et conservant les propriétés immunogènes contre le virus HBV humain, et
- la séquence d'acides nucléiques codant pour la quasi-intégralité de la séquence d'une des protéines E1 et E2 (figures 3B et 3C), conservant également les propriétés immunogènes contre le virus HCV,
- la séquence d'acides nucléiques codant pour un peptide d'initiation de transfert de l'invention, conservant la capacité à adresser après traduction ladite protéine de fusion au réticulum endoplasmique de sorte qu'elle soit correctement glycosylée et que sa conformation tridimentionnelle présente le moins d'altérations possibles, et/ou que les qualités antigéniques soient au mieux préservées au regard des protéines sauvages,
de sorte que la protéine de fusion codée par ladite molécule d'acides nucléiques hybride susmentionnée, conserve la capacité à former des particules sous-virales, et les propriétés immunogènes vis-à-vis du virus HBV et/ou HCV, et notamment la propriété d'induire une double immunisation contre les virus HBV et HCV.

On entend par « **pourcentage d'homologie** », le pourcentage déterminé par comparaison directe de deux séquences dé molécules polynucléotidiques, en déterminant le nombre de résidus d'acides nucléiques des deux séquences, puis en le divisant par le nombre de résidus d'acides nucléiques de la séquence la plus longue des deux, et en multipliant le résultat par 100.

Selon un aspect particulièrement avantageux de l'invention, les séquences d'acides nucléiques codant pour les domaines transmembranaires de E1 et/ou de E2, sont délétés d'au moins l'un des neuf derniers acides nucléiques, et préférentiellement des neuf derniers acides nucléiques, situés en position 3', de sorte que les séquences d'acides nucléiques codant pour les domaines transmembranaires de E1 et/ou de E2 correspondent :
- aux fragments, nucléotidiques du génome d'un isolat de HCV, localisés dans les régions comprises :
   * de l'acide nucléique en position 1398 à celui en position 1490 et, avantageusement, de l'acide nucléique en position 1398 à celui en position 1481 en ce qui concerne la séquence d'acides nucléiques codant pour les domaines transmembranaires de E1, ou
   * de l'acide nucléique en position 2493 à celui en position 2579 et, avantageusement, de l'acide nucléique en position 2493 à celui en position 2570 en ce qui concerne la séquence d'acides nucléiques codant pour les domaines transmembranaires de E2, ou
- aux fragments nucléotidiques du génome d'un isolat de HCV présentant un pourcentage d'homologie avec lesdits fragments nucléotidiques sauvages du génome du HCV susmentionnés, d'au moins 91%, en ce qui concerne E1 et d'au moins 90%, en ce qui concerne E2, ou
- aux fragments nucléotidiques issus de variant naturels du virus HCV, et/ou issus de variants synthétiques desdits fragments nucléotidiques sauvages du. génome d'un isolat de HCV.

L'expression « domaines transmembranaires de E1 et/ou de E2 délétés d'au moins l'un des neuf derniers acides nucléiques situés en position 3' » concerne des délétions, de 3 acides nucléiques, ou de 6 acides nucléiques, ou de 9 acides nucléiques situés en position 3' d'un desdits domaines transmembranaires.

Avantageusement, la première et la seconde séquence d'acides nucléiques de la molécule d'acides nucléiques hybride susmentionnée, et codant pour la protéine de fusion immunogène susmentionnée sont contigües, et l'extrémité 5' de la première séquence d'acides nucléiques est liée de façon covalente à l'extrémité 3' de la seconde séquence d'acides nucléiques.

Selon un aspect particulièrement avantageux, la molécule d'acides nucléiques hybride définie ci-dessus correspond par exemple aux molécules suivantes : e1-sd, e2-sd, ou pit1-e1-sd ou pit 2-e2-sd, codant respectivement pour les protéines de fusion suivantes : E1-Sd, E2-Sd, PIT1-E1-Sd ou PIT2-E2-Sd).

Selon un autre aspect avantageux de l'invention, une séquence nucléotidique codant pour un peptide de liaison, relie lesdites première et seconde séquences nucléotidiques susmentionnées, ladite séquence nucléotidique étant constituée
- de 3 acides nucléiques, ou de 6 acides nucléiques, ou de 9 acides nucléiques, ou de 12 acides nucléiques, ou de 15 acides nucléiques, ou
- de toute séquence nucléotidique codant pour tout peptide de liaison ;
sous réserve que la protéine de fusion immunogène codée par la molécule d'acides nucléiques hybride et qui comprend ledit peptide de liaison, lui-même codé par ladite séquence nucléotidique, conserve la capacité à s'assembler en particules sous-virales non infectieuses, en présence dé la protéine S sauvage, et conserve les propriétés immunogènes vis-à-vis du virus HCV, et/ou, du virus HBV.

A ce titre, l'invention a particulièrement pour objet une molécule d'acides nucléiques hybride susmentionnée, dans laquelle la première séquence d'acides nucléiques codant pour la protéine S délétée en N-terminal, de HBV, et particulièrement de l'isolat HBVadw, est située du côté 3' de ladite molécule d'acides nucléiques hybride, et est constituée :
- d'une séquence d'acides nucléiques délimitée par les acides nucléiques contigus situés de la position 1630 à la position 2241 du génome de HBV, et notamment,
- de la séquence d'acides nucléiques représentée par la SEQ ID N°1, ou
- d'une séquence d'acides nucléiques présentant une homologie d'au moins 91 %, notamment d'au moins 93%, particulièrement d'au moins 95%, et plus particulièrement d'au moins 97%, avec ladite séquence d'acides nucléiques codant pour ladite protéine S délétée en N-terminal, sous réserve que la protéine codée par ladite séquence d'acides nucléiques, conserve la capacité à former des particules sous-virales non infectieuses, immunogènes vis-à-vis du virus de l'hépatite B, ou,
- de la séquence d'acides nucléiques d'un variant naturel issue d'un isolat du virus HBV ou d'un variant synthétique, dérivé de ladite séquence d'acides nucléiques codant pour ladite protéine S délétée en N-terminal, sous réserve que la protéine codée par ladite séquence d'acides nucléiques, conserve la capacité à former des particules sous-virales non infectieuses, immunogènes vis-à-vis du virus de l'hépatite B.

Un objet plus particulier de l'invention porte sur une molécule d'acides nucléiques hybride susmentionnée (notamment e1-sd ou e2-sd, ou e1-e2-sd, pit1-e1-sd ou pit2-e2-sd, ou pit1-e1-e2-sd) codant pour une protéine de fusion susmentionnée (notamment E1-Sd ou E2-Sd, ou E1-E2-Sd, PIT1-E1-Sd ou PIT2-E2-Sd, ou PIT1-E1-E2-Sd) pour laquelle ladite séquence d'acides nucléiques codant pour la protéine S délétée du HCV est celle de l'isolat HBVadw, et a pour séquence SEQ ID N°1.

A ce titre encore, l'invention concerne plus particulièrement une molécule d'acides nucléiques hybride susmentionnée, dans laquelle la seconde séquence d'acides nucléiques codant pour le domaine transmembranaire et l'ectodomaine de la protéine E1 de HCV, et particulièrement de l'isolat HCV-1a, est située du côté 5' de ladite molécule d'acides nucléiques hybride, et est constituée :
- d'une séquence d'acides nucléiques délimitée par les acides nucléiques contigus situés de la position 915 à la position 1490, et particulièrement de la position 915 à la position 1481, du génome de HCV, et particulièrement de l'isolat HCV-1a,
   et notamment de la séquence d'acides nucléiques représentée par la SEQ ID N°3, ou
- d'une séquence d'acides nucléiques présentant une homologie d'au moins 75%, notamment d'au moins 80%, particulièrement d'au moins 85%, et plus particulièrement d'au moins 90%, avec ladite séquence d'acides nucléiques de la protéine E1, sous réserve que la protéine codée par ladite séquence d'acides nucléiques conserve les propriétés immunogènes vis-à-vis du virus de l'hépatite C, ou
- de la séquence d'acides nucléiques d'un variant naturel issue d'un isolat du virus HCV ou d'un variant synthétique dérivé de ladite séquence d'acides nucléiques de la protéine E1, sous réserve que la protéine codée par ladite séquence d'acides nucléiques conserve les propriétés immunogènes vis-à-vis du virus de l'hépatite C.
Un objet plus particulier de l'invention porte sur une molécule d'acides nucléiques hybride susmentionnée (notamment e1-sd ou pit1-e1-sd, ou e1-e2-sd, ou pitl-el-e2-sd) codant pour une protéine de fusion (notamment E1-Sd ou PIT1-E1-Sd, ou E1-E2-Sd, ou PIT1-E1-E2-Sd), pour laquelle ladite séquence d'acides nucléiques codant pour la protéine E1 est celle de l'isolat HCV-1a, est la séquence SEQ ID N°3.

L'invention concerne particulièrement une molécule d'acides nucléiques hybride susmentionnée, codant pour une protéine de fusion susmentionnée, comprenant un peptide d'initiation de transfert situé du côté N-terminal, ladite molécule d'acides nucléiques hybride étant représentée par :
- la SEQ ID N°11, ou
- une séquence d'acides nucléiques présentant une homologie d'identité d'au moins 78%, notamment d'au moins 80%, particulièrement d'au moins 85%, et plus particulièrement d'au moins 90%, avec ladite SEQ ID N°11, sous réserve que la protéine codée par ladite séquence d'acides nucléiques soit aussi délétée du domaine transmembranaire situé à l'extrémité N-terminale de la protéine S de HBV, conserve les propriétés du domaine transmembranaire de la protéine d'enveloppe de HCV et qu'elle conserve les propriétés immunogènes vis-à-vis de HCV et/ou de HBV humain, ou
- de la séquence d'acides nucléiques d'un variant synthétique, dérivé de ladite SEQ ID N°11, sous réserve que la protéine codée par ladite séquence d'acides nucléiques, dudit variant synthétique soit aussi délétée du domaine transmembranaire situé à l'extrémité N-terminale de la protéine S de HBV, conserve les propriétés du domaine transmembranaire de la protéine d'enveloppe de HCV et qu'elle conserve les propriétés immunogènes vis-à-vis de HCV et/ou de HBV humain.

La séquence d'acides nucléiques SEQ ID N°11, également nommée pit1-e1-sd, code pour la protéine de fusion PIT1-E1-Sd de SEQ ID N°12 (cf tableau 1).

L'invention a également pour objet, une molécule d'acide nucléiques hybride (notamment pit1-e1-sd de SEQ ID N°11) pour laquelle :
- la séquence d'acide nucléiques codant pour la protéine E1 est issue de HCV, et particulièrement de l'isolat HCV-1a, et est la séquence SEQ ID N °3 susmentionnée et,
- la séquence d'acide nucléiques codant pour la protéine S est issue de HBV, et particulièrement de l'isolat HBV adw, et est la séquence SEQ ID N °1 susmentionnée, ladite SEQ ID N°1 étant greffée en 5' de la SEQ ID N°3 de E1, elle même greffée en 5' de la séquence d'acide nucléiques codant pour le peptide d'initiation de transfert de la protéine E1 (PIT1) compris dans la région constituée de l'acide nucléique en position 837 à celui en position 914 de HCV.

A ce titre, l'objet de l'invention porte plus particulièrement sur une molécule d'acides nucléiques hybride susmentionnée, dans laquelle la seconde séquence d'acides nucléiques codant pour le domaine transmembranaire et l'ectodomaine de la protéine E2 de HCV, et particulièrement de l'isolat HCV-1a, est située du côté 5' de ladite molécule, et est constituée :
- d'une séquence d'acides nucléiques délimitée par les acides nucléiques contigus situés de la position 1491 à la position 2579, et particulièrement de la position 1491 à la position 2570, du génome de HCV, et particulièrement de l'isolat HCV-1a,
   et notamment de la séquence d'acides nucléiques représentée par la SEQ ID N°5, ou
- d'une séquence d'acides nucléiques présentant une homologie d'au moins 78%, notamment d'au moins 80%, particulièrement d'au moins 85%, et plus particulièrement d'au moins 90%, avec ladite séquence d'acides nucléiques de la protéine E2, sous réserve que la protéine codée par ladite séquence d'acides nucléiques conserve les propriétés immunogènes vis-à-vis du virus de l'hépatite C, ou
- de la séquence d'acides nucléiques d'un variant naturel issue d'un isolat du virus HCV ou d'un variant synthétique, dérivé de ladite SEQ ID N°5, sous réserve que la protéine codée par ladite séquence d'acides nucléiques conserve les propriétés immunogènes vis-à-vis du virus de l'hépatite C.
Un objet plus particulier de l'invention porte sur une molécule d'acides nucléiques hybride susmentionnée (notamment e2-sd ou pit2-e2-sd) codant pour une protéine de fusion (notamment E2-Sd ou PIT2-E2-Sd).

L'invention porte particulièrement sur une molécule d'acides nucléiques hybride susmentionnée, codant pour une protéine de fusion de l'invention, comprenant un peptide d'initiation de transfert situé du côté N-terminal, ladite molécule d'acides nucléiques hybride étant représentée par :
- la SEQ ID N°13, ou
- une séquence d'acides nucléiques présentant une homologie d'identité d'au moins 80%, notamment d'au moins 85%, particulièrement d'au moins 90%, et plus particulièrement d'au moins 95%, avec ladite SEQ ID N°13, sous réserve que la protéine codée par ladite séquence d'acides nucléiques soit aussi délétée du domaine transmembranaire situé à l'extrémité N-terminale de la protéine S de HBV, conserve les propriétés du domaine transmembranaire de la protéine d'enveloppe de HCV et qu'elle conserve les propriétés immunogènes vis-à-vis du virus HCV et/ou de HBV humain, ou
- de la séquence d'acides nucléiques dérivé d'un variant synthétique, dérivé de ladite SEQ ID N°13, sous réserve que la protéine codée par ladite séquence d'acides nucléiques dudit variant synthétique soit aussi délétée du domaine transmembranaire situé à l'extrémité N-terminale de la protéine S de HBV, conserve les propriétés du domaine transmembranaire de la protéine d'enveloppe de HCV et qu'elle conserve les propriétés immunogènes vis-à-vis du virus HCV et/ou de HBV humain.

La séquence d'acides nucléiques SEQ ID N°13, également nommée pit2-e2-sd, code pour la protéine de fusion PIT2-E2-Sd de SEQ ID N°14 (cf tableau 1).

L'invention a également pour objet, une molécule d'acide nucléiques hybride (notamment pit2-e2-sd de SEQ ID N°13) pour laquelle :
- la séquence d'acide nucléiques codant pour la protéine E2 est issue de HCV, et particulièrement de l'isolat HCV-1a, et est la séquence SEQ ID N °5 susmentionnée et, pour laquelle
- la séquence d'acide nucléiques codant pour la protéine S est issue de HBV, et particulièrement de l'isolat HBVadw, et est la séquence SEQ ID N°1 susmentionnée, ladite SEQ ID N°1 étant greffée en 5' de la SEQ ID N°5 de E2, elle même greffée en 5' de la séquence d'acide nucléiques codant pour le peptide d'initiation de transfert de la protéine E2 (PIT2) compris dans la région constituée de l'acide nucléique en position 1437 à celui en position 1490 de HCV.

L'invention a également pour objet un vecteur comprenant une molécule d'acides nucléiques hybride susmentionnée codant pour une protéine de fusion susmentionnée, ainsi que les moyens nécessaires pour leur expression liés de façon opérationnelle à ladite molécule d'acides nucléiques hybride.

A titre de vecteurs d'expression qui conviennent aux fins de l'invention, on peut citer par exemple les plasmides, les vecteurs viraux type lentiviraux, Semliki, adenovirus, poxvirus, virus de la vaccine, baculovirus, les vecteurs bactériens du type salmonelle, BCG.

On entend par « **moyen nécessaire à l'expression** » d'une protéine, le terme protéine étant utilisé pour toute molécule d'acides aminés, telle que protéine, protéine de fusion, fragment de protéine, peptide, polyprotéine, polypeptide, etc., tout moyen qui permet d'obtenir la protéine, tel que notamment un promoteur, un terminateur de transcription, une origine de réplication et de préférence un marqueur de sélection. Les moyens nécessaires à l'expression d'un peptide sont liés de façon opérationnelle à la séquence d'acide nucléique codant pour le peptide d'intérêt.

Par « **liés de façon opérationnelle** », on entend une juxtaposition desdits éléments nécessaires à l'expression et du gène codant pour le peptide d'intérêt, lesquels sont en une relation telle que cela leur permet de fonctionner de façon attendue. Par exemple, il peut exister des bases supplémentaires entre le promoteur et le gène d'intérêt tant que leur relation fonctionnelle est préservée.

Les moyens nécessaires à l'expression d'un peptide peuvent être des moyens homologues, c'est-à-dire inclus dans le génome du vecteur utilisé, ou bien être hétérologues. Dans ce dernier cas, lesdits moyens sont clonés avec le peptide d'intérêt à exprimer.

De préférence, le promoteur utilisé dans le vecteur Semliki et le vecteur lentiviral utilisés comme vecteur d'expression, est un promoteur homologue et est un promoteur du cytomégolovirus (CMV).

Des exemples non limitatifs de promoteurs hétérologues comprennent notamment (i) les promoteurs viraux tels que le promoteur SV40 (Virus simien 40), le promoteur du gène de la thimidine-kinase du virus simplex de l'Herpès (TK-HSV-1), le LTR du virus du sarcome de Rous (RSV), le promoteur du cytomégolovirus (CMV) et le promoteur dernier majeur adénoviral (MLP), ainsi que (ii) tout promoteur cellulaire qui contrôle la transcription des gènes codant pour des peptides chez des eucaryotes supérieurs, tel que le promoteur du gène de phosphoglycérate-kinase (PGK) constitutif (Adra et al., 1987, Gene, 60 : 65-74), le promoteur des gènes spécifiques du foie alphal-antitrypsine et FIX et le promoteur SM22 spécifique des cellules du muscle lisse (Moessler et al., 1996, Development, 122 : 2415-2425).

L'invention porte particulièrement sur un vecteur susmentionné, comprenant en outre, un vecteur lentiviral

Un vecteur ventiviral susmentionné, peut être le vecteur pLenti.

Ce dernier a été antérieurement décrit notamment par Naldini, L., Blomer, U., Gallay, P., Ory, D., Mulligan, R., Gage, F. H., Verma, I. M., and Trono, D. (1996). In vivo gene delivery and stable transduction of nondividing cells by a lentiviral vector. Science 272(5259), 263-7.

L'invention a également pour objet un vecteur susmentionné, comprenant en outre, un vecteur viral défectif dérivé du génome du virus de la forêt Semliki.

Ces derniers ont été antérieurement décrits notamment par Schlesinger, S., and T. M. Dubensky, Jr. 1999. Alphavirus vectors for gene expression and vaccines. Curr. Opin. Biotechnol. 10:434-439.

L'invention a notamment pour objet un vecteur susmentionné, dans lequel le vecteur viral défectif dérivé du génome du virus de la forêt Semliki est le vecteur SFV1, ce dernier étant notamment commercialisé par Invitrogen.

A ce titre, l'invention a plus particulièrement pour objet un vecteur susmentionné, dans lequel la molécule d'acides nucléiques hybride est constituée:
- de la séquence d'acides nucléiques représentée par SEQ ID N°11, ou
- d'une séquence d'acides nucléiques présentant une homologie d'au moins 78%, notamment d'au moins 80%, particulièrement d'au moins 85%, et plus particulièrement d'au moins 90%, avec ladite séquence SEQ ID N°11, sous réserve que la protéine codée par ladite séquence d'acides nucléiques code pour une protéine de fusion capable de former des particules sous-virales, non infectieuses et immunogènes vis-à-vis HCV et/ou HBV,
   lequel vecteur est notamment représenté par la SEQ ID N° 15, ou
- de la séquence d'acides nucléiques d'un variant synthétique dérivé de séquence d'acides nucléiques représentée par SEQ ID N°11, sous réserve que la protéine codée par ladite séquence d'acides nucléiques codé pour une protéine de fusion capable de former des particules sous-virales, non infectieuses et immunogènes vis-à-vis HCV et/ou HBV.

L'invention a également pour objet, un vecteur dérivé de pSFV de séquence SEQ ID N° 15, comprenant une molécule d'acide nucléiques hybride (notamment pit1-e1-sd de SEQ ID N°11) codant pour la protéine de fusion susmentionnée (notamment PIT1-E1-Sd de SEQ ID N°12), et pour laquelle :
- la séquence d'acide nucléiques codant pour la protéine E1 est issue de HCV, et particulièrement de l'isolat HCV-1a, et est la séquence SEQ ID N °3 susmentionnée et, pour laquelle
- la séquence d'acide nucléiques codant pour la protéine S est issue de HBV, et particulièrement de l'isolat HBVadw, et est la séquence SEQ ID N °1 susmentionnée, ladite SEQ ID N°1 étant greffée en 5' de la SEQ ID N°3 de E1, elle même greffée en 5' de la séquence d'acide nucléiques codant pour le peptide d'initiation de transfert de la protéine E1 (PIT1) compris dans la région constituée de l'acide nucléique en position 837 à celui en position 914 de HCV.

A ce titre, l'invention a plus particulièrement pour objet l'un des vecteurs susmentionné, dans lequel la molécule d'acides nucléiques hybride est constituée :
- de la séquence d'acides nucléiques représentée par SEQ ID N°13, ou
- d'une séquence d'acides nucléiques présentant une homologie d'au moins 80%, particulièrement d'au moins 85%, plus particulièrement d'au moins 90%, et plus particulièrement d'au moins 95%, avec ladite séquence SEQ ID N°13, sous réserve que ladite séquence d'acides nucléiques code pour un peptide capable de former des particules sous-virales, non infectieuses et immunogènes vis-à-vis du virus de HBV et/ou HCV,
   lequel vecteur est notamment représenté par la SEQ ID N° 16,
- de la séquence d'acides nucléiques d'un variant synthétique, dérivé de ladite SEQ ID N°13, sous réserve que la protéine codée par ladite séquence d'acides nucléiques code pour un peptide capable de former des particules sous-virales, non infectieuses et immunogènes vis-à-vis du virus de HBV et/ou HCV.

L'invention a également pour objet, un vecteur de SEQ ID N° 16, comprenant une molécule d'acide nucléiques hybride (notamment pit1-e1-sd de SEQ ID N°13) codant pour la protéine de fusion susmentionnée (notamment PIT1-E1-Sd de SEQ ID N°14), et pour laquelle :
- la séquence d'acide nucléiques codant pour la protéine E2 est issue de HCV, et particulièrement de l'isolat HCV-1a, et est la séquence SEQ ID N °5 susmentionnée et, pour laquelle
- la séquence d'acide nucléiques codant pour la protéine S est issue de HBV, et particulièrement de l'isolat HBVadw, et est la séquence SEQ ID N°1 susmentionnée,
- ladite SEQ ID N°1 étant greffée en 5' de la SEQ ID N°5 de E2, elle même greffée en 5' de la séquence d'acide nucléiques codant pour le peptide d'initiation de transfert de la protéine E2 (PIT2) compris dans la région constituée de l'acide nucléique en position 1437 à celui en position 1490 de HCV.

L'invention concerne également un vecteur susmentionné, comprenant une molécule d'acides nucléiques hybride constituée dès trois séquences d'acides nucléiques suivantes :
a)- du côté 3', une première séquence d'acides nucléiques codant pour la protéine S délétée de son domaine transmembranaire situé à son extrémité N-terminale (Sd), d'un isolat du virus de l'hépatite B, ou
   - d'une séquence d'acides nucléiques présentant une homologie d'au moins 91 %, notamment d'au moins 93%, particulièrement d'au moins 95%, et plus particulièrement d'au moins 97%, avec ladite séquence d'acides nucléiques de la protéine S délétée en N-terminal, sous réserve que ladite séquence d'acides nucléiques code pour un peptide capable de former des particules sous-virales, non infectieuses et immunogènes vis-à-vis du virus de HBV, ou
   - de la séquence d'acides nucléiques d'un variant naturel issue d'un isolat du virus HCV ou d'un variant synthétique dérivé de ladite séquence d'acides nucléiques codant pour la protéine S délétée (Sd), sous réserve que la protéine codée par ladite séquence d'acides nucléiques code pour un peptide capable de former des particules sous-virales, non infectieuses et immunogènes vis-à-vis du virus de HBV, et,
b)- à l'extrémité 5' de ladite première séquence d'acides nucléiques, une seconde séquence d'acides nucléiques codant pour le domaine transmembranaire et l'ectodomaine d'au moins une protéine d'enveloppe E1 ou E2, d'un isolat du virus de l'hépatite C, ou
   - d'une séquence d'acides nucléiques présentant une homologie d'au moins 78%, notamment d'au moins 80%, particulièrement d'au moins 85%, et plus particulièrement d'au moins 90%, avec ladite séquence d'acides nucléiques codant pour l'une desdites protéines E1 ou E2, d'un isolat du virus de l'hépatite C, sous réserve que la protéine codée par ladite séquence d'acides nucléiques code pour un peptide immunogènes vis-à-vis du virus de l'hépatite C, ou
   - de la séquence d'acides nucléiques d'un variant naturel issue d'un isolat du virus HCV ou d'un variant synthétique dérivé de ladite seconde séquence d'acides nucléiques codant pour une protéine d'enveloppe E1 ou E2, sous réserve que la protéine codée par ladite séquence d'acides nucléiques code pour un peptide immunogènes vis-à-vis du virus de l'hépatite C,
   ladite seconde séquence étant choisie parmi les séquences codant pour la protéine E1, pour la protéine E2 ou pour un peptide de fusion comprenant la protéine E1 et la protéine E2, et,
c)- à l'extrémité 5' de ladite seconde séquence d'acides nucléiques, une troisième séquence d'acides nucléiques codant pour un peptide d'initiation de transfert (PIT) d'un isolat du virus de l'hépatite Ci ou
   - une séquence d'acides nucléiques d'un variant naturel issue d'un isolat du virus HCV, ou d'un variant synthétique, dérivé de ladite la séquence d'acides nucléiques de la protéine codant pour un peptide d'initiation de transfert (PIT), sous réserve que le peptide d'initiation de transfert codé par ladite la séquence d'acides nucléiques, conserve la capacité à adresser, après traduction, ladite protéine de fusion au réticulum endoplasmique de sorte qu'elle soit correctement glycosylée et que sa conformation tridimentionnelle présente le moins d'altération possible, et/ou que ses qualité antigéniques soient au mieux préservées au regard des protéines sauvages, ou,
   - toute séquence d'acides nucléiques codant pour un peptide capable d'adresser, après traduction, ladite protéine de fusion au réticulum endoplasmique de sorte qu'elle soit correctement glycosylée et que sa conformation tridimentionnelle présente le moins d'altérations possibles, et/ou que ses qualités antigéniques soient au mieux préservées au regard des protéines sauvages.

Avantageusement, l'invention a également pour objet un vecteur susmentionné, dans lequel la première et la seconde séquence d'acides nucléiques de la molécule d'acides nucléiques hybride susmentionnée (notamment e1-sd ou e1-e2-sd) et codant pour la protéine de fusion immunogène susmentionnée (notamment E1-Sd ou E2-Sd) sont contigües, et l'extrémité 5' de la première séquence d'acides nucléiques est liée de façon covalente à l'extrémité 3' de la seconde séquence d'acides nucléiques.

Selon un autre aspect avantageux, l'invention concerne le vecteur susmentionné (notamment un vecteur lentiviral, tel que le vecteur pLenti, ou un vecteur viral défectif dérivé du génome du virus de la forêt Semliki, tel que le vecteur pSFV1) pour lequel une séquence nucléotidique, codant pour un peptide de liaison, relie lesdites première et seconde séquences nucléotidiques codant pour la protéine de fusion immunogène susmentionnée (notamment E1-Sd ou E2-Sd), ladite séquence nucléotidique étant constituée
de 3 acides nucléiques, ou de 6 acides nucléiques, ou de 9 acides nucléiques, ou de 12 acides nucléiques, ou de 15 acides nucléiques, sous réserve que ladite séquence nucléotidique, codant pour un peptide de liaison, n'altère pas la capacité de la protéine de fusion immunogène susmentionnée (notamment E1-Sd ou E2-Sd), à s'assembler en particules sous-virales, en présence de la protéine S sauvage, et que cette dernière conserve également les propriétés immunogènes vis-à-vis du virus HCV, et/ou, du virus HBV.

Avantageusement, le vecteur susmentionné comprend la première séquence d'acides nucléiques codant pour la protéine S délétée en N-terminal, de HBV, et notamment de l'isolat HBVadw du virus de l'hépatite B, ladite séquence étant :
- une séquence d'acides nucléiques délimitée par les acides nucléiques contigus situés en positions 1630 à 2241 de HBV, et particulièrement de l'isolat HBVadw,
   et notamment de la séquence d'acides nucléiques représentée par la SEQ ID N°1, ou
- une séquence d'acides nucléiques présentant une homologie d'au moins 91 %, notamment d'au moins 93%, particulièrement d'au moins 95%, et plus particulièrement d'au moins 97%, avec ladite SEQ ID N°1, sous réserve que la protéine codée par ladite séquence d'acides nucléiques conserve la capacité à former des particules sous-virales non infectieuses, immunogènes vis-à-vis du virus de l'hépatite B, ou
- de la séquence d'acides nucléiques d'un variant naturel issue d'un isolat du virus HBV ou d'un variant synthétique dérivé de la SEQ ID N°1, sous réserve que la protéine codée par ladite séquence d'acides nucléiques conserve la capacité à former des particules sous-virales non infectieuses, immunogènes vis-à-vis du virus de l'hépatite B.

Un objet plus particulier de l'invention réside dans le vecteur susmentionné (notamment un vecteur lentiviral, tel que le vecteur pLenti, ou un vecteur viral défectif dérivé du génome du virus de la forêt Semliki, tel que le vecteur pSFV1) comprenant d'une molécule d'acides nucléiques hybride susmentionnée (notamment e1-sd ou e2-sd, ou e1-e2-sd, pit1-e1-sd ou pit2-e2-sd) codant pour une protéine de fusion (notamment E1-Sd ou E2-Sd, ou E1-E2-Sd, PIT1-E1-Sd ou PIT2-E2-Sd) pour laquelle ladite séquence d'acides nucléiques codant pour la protéine S délétée du HBV est celle de l'isolat HBV*adw*, et a pour séquence SEQ ID N°1.

Selon un aspect particulier de l'invention, le vecteur susmentionné comprend la seconde séquence d'acides nucléiques codant pour le domaine transmembranaire et l'ectodomaine de la protéine E1 de HCV, et particulièrement de l'isolat HCV-1a, qui est située du côté 5' de ladite molécule, et qui est constituée :
- d'une séquence d'acides nucléiques délimitée par les acides nucléiques contigus situés de la position 915 à la position 1490, et particulièrement de la position 915 à la position 1481, du génome de HCV, et particulièrement de l'isolat HCV-1a,
   et notamment de la séquence d'acides nucléiques représentée par la SEQ ID N°3, ou
- d'une séquence d'acides nucléiques présentant une homologie d'au moins 75%, notamment d'au moins 80%, particulièrement d'au moins 85%, et plus particulièrement d'au moins 90%, avec ladite séquence d'acides nucléiques codant pour la protéine E1, sous réserve que la protéine codée par ladite séquence d'acides nucléiques conserve les propriétés immunogènes vis-à-vis du virus de l'hépatite C, ou
- de la séquence d'acides nucléiques d'un variant naturel issue d'un isolat du virus HCV ou d'un variant synthétique, dérivé de ladite d'acides nucléiques codant pour ladite protéine E1, sous réserve que la protéine codée par ladite séquence d'acides nucléiques conserve les propriétés immunogènes vis-à-vis du virus de l'hépatite C.

Un objet plus particulier de l'invention réside dans le vecteur susmentionné (notamment un vecteur lentiviral, tel que le vecteur pLenti, ou un vecteur viral défectif dérivé du génome du virus de la forêt Semliki, tel que le vecteur pSFV1) comprenant une molécule d'acides nucléiques hybride susmentionnée (notamment e1-sd, ou e1-e2-sd, pit1-e1-sd) codant pour une protéine de fusion (notamment E1-Sd, ou E1-E2-Sd, PIT1-E1-Sd) pour laquelle ladite séquence d'acides nucléiques codant pour la protéine E1 du HCV est celle de l'isolat HCV-1a, et a pour séquence SEQ ID N°3.

Conformément à un aspect avantageux de l'invention, le vecteur susmentionné comprend la seconde séquence d'acides nucléiques codant pour le domaine transmembranaire et l'ectodomaine de la protéine E2 de HCV, et particulièrement de l'isolat HCV-1a, qui est située du côté 5' de ladite molécule, et qui est constituée :
- d'une séquence d'acides nucléiques délimitée par les acides nucléiques contigus situés de la position 1491 à la position 2579, et particulièrement de la position 1491 à la position 2570, du génome de HCV, et particulièrement de l'isolat HCV-1a,
   et notamment de la séquence d'acides nucléiques représentée par la SEQ ID N°5, ou
- d'une séquence d'acides nucléiques présentant une homologie d'au moins 78%, notamment d'au moins 80%, particulièrement d'au moins 85%, et plus particulièrement d'au moins 90%, avec ladite séquence d'acides nucléiques codant pour la protéine E2, sous réserve que la protéine codée par ladite séquence d'acides nucléiques conserve les propriétés immunogènes vis-à-vis du virus de l'hépatite C, ou
- de la séquence d'acides nucléiques d'un variant naturel, issue d'un isolat du virus HCV,ou d'un variant synthétique dérivé de ladite d'acides nucléiques codant pour ladite protéine E2, sous réserve que la protéine codée par ladite séquence d'acides nucléiques conserve les propriétés immunogènes vis-à-vis du virus de l'hépatite C.

Un objet plus particulier de l'invention réside dans le vecteur susmentionné (notamment un vecteur lentiviral, tel que le vecteur pLenti, ou un vecteur viral défectif dérivé du génome du virus de la forêt Semliki, tel que le vecteur pSFV1) comprenant d'une molécule d'acides nucléiques hybride susmentionnée (notamment e2-sd, ou e1-e2-sd, pit2-e2-sd) codant pour une protéine de fusion (notamment E2-Sd, ou E1-E2-Sd, PIT2-E2-Sd) pour laquelle ladite séquence d'acides nucléiques codant pour la protéine E2 du HCV est celle de l'isolat HCV-1a, et a pour séquence SEQ ID N°5.

L'invention a également pour objet une particule d'enveloppe sous-virale, chimère, immunogène et non infectieuse, comprenant les protéines suivantes :
- la protéine constituée de la protéine S sauvage de l'antigène de surface d'un isolat du virus de l'hépatite B, et,
- au moins une protéine de fusion immunogène susmentionnée.

On entend par le terme « **particule sous-virale chimère** », toute particule sous virale comprenant au moins la protéine S sauvage de HBV et une protéine de fusion immunogène susmentionnée résultant de l'auto-assemblage de la protéine S sauvage, ou de l'assemblage de d'une protéine de fusion susmentionnée, en présence de la protéine S sauvage.

Avantageusement, la protéine de fusion immunogène de la particule sous-virale chimère immunogène susmentionnée, est:
- la séquence d'acides aminés représentée par la SEQ ID N°8, ou
- une séquence d'acides aminés présentant un pourcentage d'identité d'au moins 88%, notamment d'au moins 90%, particulièrement d'au moins 92%, et plus particulièrement d'au moins 95%, avec la dite SEQ ID N°8, sous réserve que ladite séquence d'acides aminés soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis de HCV et/ou vis-à-vis de HBV humain, ou
- de la séquence d'acides aminés d'un variant synthétique, dérivée de ladite SEQ ID N°8, sous réserve que ladite séquence d'acides aminés dudit variant synthétique soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis de HCV et/ou vis-à-vis de HBV humain.

L'invention a également pour objet, une particule sous-virale chimère immunogène susmentionnée comprenant une protéine de fusion susmentionnée, et notamment la protéine de fusion E1-Sd de SEQ ID N°8, où E1-E2-Sd, et pour laquelle la séquence de la protéine E1 est notamment la SEQ ID N°4 susmentionnée, et est notamment greffée en C-terminal de la séquence d'acides aminés de la protéine S délétée (Sd) de séquence SEQ ID N°2.

Selon un aspect particulier de l'invention, la protéine de fusion immunogène de la particule sous-virale chimère immunogène sus-mentionnée, est constituée par :
- la séquence d'acides aminés représentée par la SEQ ID N°10, ou
- une séquence d'acides aminés présentant un pourcentage d'identité d'au moins 86%, notamment d'au moins 88%, particulièrement d'au moins 92%, et plus particulièrement d'au moins 95%, avec SEQ ID N°10, sous réserve que ladite séquence d'acides aminés soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus de l'hépatite B humain et/ou vis-à-vis du virus de l'hépatite C, ou
- de la séquence d'acides aminés d'un variant synthétique, dérivée de la SEQ ID N°10, sous réserve que ladite séquence d'acides aminés dudit variant synthétique soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus de l'hépatite B humain et/ou vis-à-vis du virus de l'hépatite C.

L'invention a également pour objet, une particule sous-virale chimère immunogène susmentionnée comprenant une protéine de fusion susmentionnée, et notamment la protéine de fusion E1-Sd dé SEQ ID N°10, ou E1-E2-Sd, et pour laquelle la séquence de la protéine E1 est notamment la SEQ ID N°6 susmentionnée, et est notamment greffée en C-terminal de la séquence d'acides aminés de la protéine S délétée (Sd) de séquence SEQ ID N°2.

Conformément à un aspect avantageux de l'invention, la particule sous-virale chimère immunogène susmentionné, comprenant les deux protéines de fusion suivantes :
- la séquence d'acides aminés représentée par la SEQ ID N°8, ou
- une séquence d'acides aminés présentant un pourcentage d'idéntité d'au moins 88%, notamment d'au moins 90%, particulièrement d'au moins 92%, et plus particulièrement d'au moins 95%, avec la dite SEQ ID N°8, sous réserve que ladite SEQ ID N°8 code pour une protéine qui soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus de l'hépatite B humain et/ou vis-à-vis du virus de l'hépatite C, ou
- de la séquence d'acides aminés d'un variant synthétique, dérivée de la SEQ ID N°8, sous réserve que ladite SEQ ID N°8 code pour une protéine qui soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus de l'hépatite B humain et/ou vis-à-vis du virus de l'hépatite C, et
- la séquence d'acides aminés représentée par la SEQ ID N°10, ou
- une séquence d'acides aminés présentant un pourcentage d'identité d'au moins 86%, notamment d'au moins 88%, particulièrement d'au moins 90%, et plus particulièrement d'au moins 95%, avec SEQ ID N°10, sous réserve que ladite séquence d'acides aminés soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus de l'hépatite B humain et/ou vis-à-vis du virus de l'hépatite C, ou
- de la séquence d'acides aminés d'un variant synthétique, dérivée de la SEQ ID N°10, sous réserve que ladite séquence d'acides aminés dudit variant synthétique soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus de l'hépatite B humain et/ou vis-à-vis du virus de l'hépatite C.

L'invention a également pour objet, une particule sous-virale chimère immunogène susmentionnée comprenant deux protéines de fusion susmentionnée (notamment E1-Sd de SEQ ID N°8, ou E1-E2-Sd ; et E2-Sd de SEQ ID N°10).

L'invention concerne également une particule sous-virale chimère immunogène susmentionnée, dans laquelle la protéine de fusion immunogène comprend au moins une des protéines d'enveloppe (E1) d'un isolat du virus de l'hépatite C, ladite protéine, étant constituée :
- d'une séquence d'acides aminés correspondant aux résidus d'acides aminés situés dans la région comprenant l'acide aminé en position 192 à celui en position 380 de la protéine sauvage de HCV, et particulièrement de l'isolat HCV-1a, ou
- d'une séquence d'acides aminés présentant un pourcentage d'identité d'au moins 75%, notamment d'au moins 80%, particulièrement d'au moins 85%, et plus particulièrement d'au moins 90%, avec ladite séquence de la protéines E1 sauvage d'un isolat de HCV, sous réserve que ladite séquence d'acides aminés conserve les propriétés dudit domaine transmembranaire et les propriétés immunogènes vis-à-vis du virus de l'hépatite C, ou
- de la séquence d'acides aminés d'un variant naturel issue d'un isolat du virus HCV ou d'un variant synthétique, dérivé de ladite protéine E1, sous réserve que ladite séquence d'acides aminés conserve les propriétés dudit domaine transmembranaire et les propriétés immunogènes vis-à-vis du virus de l'hépatite C.

L'invention a également pour objet, une particule sous-virale chimère immunogène susmentionnée comprenant une protéine de fusion susmentionnée (notamment E1-Sd, ou E1-E2-Sd ou PIT1-E1-E2-Sd, ou PIT2-E2-E1-Sd), pour laquelle la séquence de la protéine E1 est issue de l'isolat HCV-1a.

L'invention a également pour objet, une particule sous-virale chimère immunogène susmentionnée, dans laquelle la protéine de fusion immunogène comprend au moins une des protéines d'enveloppe d'un isolat du virus de l'hépatite C, ladite protéine étant constituée de séquences d'acides aminés choisies parmi les suivantes :
- une séquence d'acides aminés correspondant aux résidus d'acides aminés situés dans la région comprenant l'acide aminé en position 384 à celui en position 743 de la protéine sauvage E2 de HCV, et particulièrement de l'isolat HCV-1a, ou
- une séquence d'acides aminés présentant un pourcentage d'identité d'au moins 78%, notamment d'au moins 80%, particulièrement d'au moins 85%, et plus particulièrement d'au moins 90%, avec ladite séquences de la protéines E2 sauvage dudit isolat HCV, sous réserve que ladite séquence d'acides aminés conserve les propriétés dudit domaine transmembranaire et les propriétés immunogènes vis-à-vis du virus de l'hépatite C, ou
- de la séquence d'acides aminés d'un variant naturel issue d'un isolat du virus HCV ou d'un variant synthétique, dérivé de ladite protéine E2, sous réserve que ladite séquence d'acides aminés conserve les propriétés dudit domaine transmembranaire et les propriétés immunogènes vis-à-vis du virus de l'hépatite C.

L'invention a également pour objet, une particule sous-virale chimère immunogène susmentionnée comprenant une protéine de fusion susmentionnée (notamment E2-Sd, ou E1-E2-Sd ou PIT1-E1-E2-Sd), pour laquelle la séquence de la protéine E2 est issue de l'isolat HCV-1a.

Selon un aspect particulier, l'invention concerne également une particule sous-virale chimère immunogène susmentionnée, comprenant les deux protéines de fusion suivantes :
- la protéine de fusion comprenant la séquence d'acides aminés constituée de l'acide aminé en position 192 à celui en position 383, et particulièrement en position 192 à celui en position 380 de la protéine sauvage E1 de HCV, et particulièrement de l'isolat HCV-1a ; ou
- d'une séquence d'acides aminés présentant un pourcentage d'identité d'au moins 75%, notamment d'au moins 80%, particulièrement d'au moins 85%, et plus particulièrement d'au moins 90%, avec ladite séquence de la protéines E1 sauvage d'un isolat de HCV, sous réserve que ladite séquence d'acides aminés conserve les propriétés dudit domaine transmembranaire et les propriétés immunogènes vis-à-vis du virus de l'hépatite C, ou
- de la séquence d'acides aminés d'un variant naturel issue d'un isolat du virus HCV ou d'un variant synthétique, dérivé de ladite protéine E1, sous réserve que ladite séquence d'acides aminés conserve les propriétés dudit domaine transmembranaire et les propriétés immunogènes vis-à-vis du virus de l'hépatite C, et
- la protéine de fusion comprenant la séquence d'acides aminés constituée de l'acide aminé en position 384 à celui en position 746, et particulièrement en position 384 à celui en position 743de la protéine sauvage E2 de HCV, et particulièrement de l'isolat HCV-1a, ou
- une séquence d'acides aminés présentant un pourcentage d'identité d'au moins 78%, notamment d'au moins 80%, particulièrement d'au moins 85%, et plus particulièrement d'au moins 90%, avec ladite séquences de la protéines E2 sauvage dudit isolat HCV, sous réserve ladite séquence d'acides aminés conserve les propriétés dudit domaine transmembranaire et les propriétés immunogènes vis-à-vis du virus de l'hépatite C, ou
- de la séquence d'acides aminés d'un variant naturel issue d'un isolat du virus HCV ou d'un variant synthétique, dérivé de ladite protéine E2, sous réserve que ladite séquence d'acides aminés conserve les propriétés dudit domaine transmembranaire et les propriétés immunogènes vis-à-vis du virus de l'hépatite C.

L'invention a également pour objet, une particule sous-virale chimère immunogène susmentionnée comprenant deux protéines de fusion susmentionnée (notamment E1-Sd, ou PIT1-E1-Sd ou E2-Sd ou PIT1-E1-Sd ou E1-E2-Sd ou PIT1- E1-E2-Sd).

L'invention a également pour objet une composition immunogène comprenant comme substance active au moins un composé choisi parmi :
- une protéine de fusion susmentionnée,
- une molécule d'acides nucléiques hybride précédemment décrite,
- un vecteur susmentionné comprenant ladite molécule d'acides nucléiques hybride,
- une particule sous-virale chimères décrite ci-dessus,
et, un véhicule pharmaceutiquement acceptable.

Selon un mode de réalisation particulier de l'invention, la composition pharmaceutique contient également un véhicule pharmaceutiquement approprié dont l'homme du métier déterminera facilement la nature et la quantité à utiliser en fonction de paramètres usuels et des constituants de la composition pharmaceutique, la forme pharmaceutique et le mode d'administration souhaités.

Les compositions pharmaceutiques de l'invention sont appropriées pour l'administration orale, sublinguale, sous-cutanée, intramusculaire, intraveineuse, topique, locale, intra-trachéale, intra-nasale, transdermique, rectale, intraoculaire, intra-auliculaire, ledit principe actif pouvant être administré sous forme unitaire d'administration.

Les formes unitaires d'administration peuvent être par exemple des comprimés, des gélules, des granules, des poudres, des solutions injectables ou suspensions orales, des timbres transdermiques (patch), des formes d'administration sublinguale, buccale, intra-trachéale, intraoculaire, intra-nasale, intra-auriculaire, par inhalation, des formes d'administration topique, transdermique, sous-cutanée, intramusculaire ou intraveineuse, des formes d'administration rectale ou des implants. Pour l'administration topique, on peut envisager des crèmes, gels, pommades, lotions ou collyres.

Avantageusement, la composition immunogène susmentionnée, comprend une substance active choisie parmi au moins l'un des trois composés suivants :
a)- une protéine de fusion susmentionnée constituée par :
   * la séquence d'acides aminés représentée par la SEQ ID N°8, ou
   * une séquence d'acides aminés présentant un pourcentage d'identité d'au moins 88%, notamment d'au moins 90%, particulièrement d'au moins 92%, et plus particulièrement d'au moins 95%, avec SEQ ID N°8, sous réserve que ladite séquence d'acides aminés soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus humain de l'hépatite B et/ou vis-à-vis du virus de l'hépatite C, ou
   * de la séquence d'acides aminés d'un variant synthétique, dérivé de la SEQ ID N°8, sous réserve que ladite séquence d'acides aminés dudit variant synthétique soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus humain le l'hépatite B et/ou vis-à-vis du virus de l'hépatite C,
b)- une molécule d'acides nucléiques hybride constituée par :
   * la séquence d'acides nucléiques représentée par SEQ ID N°11, ou
   * d'une séquence d'acides nucléiques présentant une homologie d'au moins 78%, notamment d'au moins 80%, particulièrement d'au moins 85%, et plus particulièrement d'au moins 90%, avec ladite séquence SEQ ID N°11, sous réserve que la protéine codée par ladite séquence d'acides nucléiques soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus humain de l'hépatite B et/ou vis-à-vis du virus de l'hépatite C, ou
   * de la séquence d'acides nucléiques d'un variant synthétique, dérivé de la SEQ ID N°11, sous réserve que la protéine codée par ladite séquence d'acides nucléiques d'un variant synthétique soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus humain de l'hépatite B et/ou vis-à-vis du virus de l'hépatite C, ou un vecteur susmentionné comprenant ladite molécule d'acides nucléiques hybride,
c)- une particule sous-virale chimères, comprenant la protéine de fusion immunogène constituée par :
   * la séquence d'acides aminés représentée par la SEQ ID N°8, ou
   * une séquence d'acides aminés présentant un pourcentage d'identité d'au moins 88%, notamment d'au moins 90%, particulièrement d'au moins 92%, et plus particulièrement d'au moins 95%, avec SEQ ID N°8, sous réserve que ladite séquence d'acides aminés soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis de HBV humain et de HCV, ou
   * de la séquence d'acides aminés d'un variant synthétique, dérivé de la SEQ ID N°8, sous réserve que ladite séquence d'acides aminés dudit variant synthétique soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis de HBV humain et de HCV.

Un objet particulier de l'invention porte sur l'une des trois composés suivants :
a)- la protéine de fusion susmentionnée comprenant la protéine E1 (notamment E1-Sd, PIT1-E1-Sd, E1-E2-Sd, PIT1-E1-E2-Sd), dont on trouve une représentation schématique dans la figure 3B, et qui comprend la quasi-intégralité de la protéine E1 du HCV, greffée en N-terminal de la protéine S délétée de HBV, ou
b)- la molécule d'acides nucléiques hybride susmentionnée comprenant séquence d'acides nucléique codant pour la protéine E1 (notamment e1-sd, pit1-e1-sd, e1-e2-sd, pit1-el-e2-sd), et qui comprend la quasi-intégralité de la séquence d'acides nucléique codant pour la protéine E1 du HCV, greffée en 5' de la séquence d'acides nucléique codant pour la protéine S délétée de HBV, ou
c)- la particule sous-virale susmentionnée comprenant la protéine E1 (notamment E1-Sd, PIT1-E1-Sd, E1-E2-Sd, PIT1-E1-E2-Sd), dont on trouve une représentation schématique dans la figure 3B, et qui comprend la quasi-intégralité de la protéine E1 du HCV, greffée en N-terminal de la protéine S délétée de HBV.

Selon un aspect particulier de l'invention, la composition immunogène susmentionnée, comprend une substance active choisie parmi au moins l'un des composés suivants:
a)- une protéine de fusion constituée par :
   * la séquence d'acides aminés représentée par la SEQ ID N°10, ou
   * une séquence d'acides aminés présentant un pourcentage d'identité d'au moins 86%, notamment d'au moins 88%, particulièrement d'au moins 90%, et plus particulièrement d'au moins 95%, avec SEQ ID N°10, sous réserve que ladite séquence d'acides aminés soit aussi délétée du domaine transmembranaire situé à l'extrémité N-terminale de là protéine S de HBV, conserve les propriétés du domaine transmembranaire de la protéine d'enveloppe de HCV et qu'elle conserve la capacité à former des particules sous-virales, non infectieuses et immunogènes vis-à-vis du virus humain de l'hépatite B et/ou vis-à-vis du virus de l'hépatite C, ou
   de la séquence d'acides aminés d'un variant synthétique, dérivé de la SEQ ID N°10, sous réserve que ladite séquence d'acides aminés dudit variant synthétique soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus humain de l'hépatite B et vis-à-vis du virus de l'hépatite C,
b)- une molécule d'acides nucléiques hybride constituée par :
   * de la séquence d'acides nucléiques représentée par SEQ ID N°13, ou
   * d'une séquence d'acides nucléiques présentant une homologie d'au moins 80%, notamment d'au moins 85%, particulièrement d'au moins 90%, et plus particulièrement d'au moins 95%, avec la dite SEQ ID N°13, sous réserve que la protéine codée par ladite séquence d'acides nucléiques soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus humain de l'hépatite B et/ou vis-à-vis du virus de l'hépatite C, ou
   * de la séquence d'àcides nucléiques d'un variant synthétique, dérivé de la SEQ ID N°13, sous réserve que la protéine codée par ladite séquence d'acides nucléiques dudit variant synthétique soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus humain de l'hépatite B et/ou vis-à-vis du virus de l'hépatite C; ou un vecteur susmentionné comprenant ladite molécule d'acides nucléiques hybride,
c)- une particule sous-virales chimères, comprenant la protéine de fusion immunogène constituée par :
   * la séquence d'acides aminés représentée par la SEQ ID N°10, ou
   * une séquence d'acides aminés présentant un pourcentage d'identité d'au moins 86%, notamment d'au moins 88%, particulièrement d'au moins 90%, et plus particulièrement d'au moins 95%, avec SEQ ID N°10, sous réserve que ladite séquence d'acides aminés soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus humain de l'hépatite B et/ou vis-à-vis du virus de l'hépatite C, ou
   * de la séquence d'acides aminés d'un variant synthétique, dérivé de la SEQ ID N°10, sous réserve que ladite séquence d'acides aminés dudit variant synthétique soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus humain de l'hépatite B et/ou vis-à-vis du virus de l'hépatite C.

Un objet particulier de l'invention porte sur l'une des trois composés suivants :
a)- la protéine de fusion susmentionnée comprenant la protéine E2 (notamment E2-Sd, PIT2-E2-Sd, E1-E2-Sd, PIT1-E1-E2-Sd), dont on trouve une représentation schématique dans la figure 3C, et qui comprend la quasi-intégralité de la protéine E2 du HCV, greffée en N-terminal de la protéine S délétée de HBV, ou
b)- la molécule d'acides nucléiques hybride susmentionnée comprenant séquence d'acides nucléique codant pour la protéine E2 (notamment e2-sd, pit2-e2-sd, e1-e2-sd, pit1-el-e2-sd), et qui comprend la quasi-intégralité de la séquence d'acides nucléique codant pour la protéine E1 du HCV, greffée en 5' de la séquence d'acides nucléique codant pour la protéine S délétée de HBV, ou
c)- la particule sous-virale susmentionnée comprenant la protéine E1 (notamment E1-Sd, PIT-E1-Sd, E1-E2-Sd, PIT-E1-E2-Sd), et qui comprend la quasi-intégralité de la protéine E2 du HCV, greffée en N-terminal de la protéine S délétée de HBV.

Conformément à un aspect avantageux de l'invention, la composition immunogène susmentionnée comprend pour substance active, une particule sous-virale chimère, comprenant au moins les deux protéines de fusion immunogènes constituées par :
- la séquence d'acides aminés représentée par la SEQ ID N°8, ou
- une séquence d'acides aminés présentant un pourcentage d'identité d'au moins 88%, notamment d'au moins 90%, particulièrement d'au moins 92%, et plus particulièrement d'au moins 95%, avec la dite SEQ ID N°8, sous réserve que ladite séquence d'acides aminés soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus humain de l'hépatite B et/ou vis-à-vis du virus de l'hépatite C, ou
- de la séquence d'acides aminés d'un variant synthétique, dérivé de la SEQ ID N°8, sous réserve que ladite séquence d'acides aminés dudit variant synthétique soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus humain de l'hépatite B et/ou vis-à-vis du virus de l'hépatite C, et,
- la séquence d'acides aminés représentée par la SEQ ID N°10, ou
- une séquence d'acides aminés présentant un pourcentage d'identité d'au moins 86%, notamment d'au moins 88%, particulièrement d'au moins 90%, et plus particulièrement d'au moins 95%, avec SEQ ID N°10 de E2-Sd, sous réserve que ladite séquence d'acides aminés soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus humain de l'hépatite B et/ou vis-à-vis du virus de l'hépatite C, ou
- de la séquence d'acides aminés d'un variant synthétique, dérivé de la SEQ ID N°10, sous réserve que ladite séquence d'acides aminés dudit variant synthétique soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus humain de l'hépatite B et/ou vis-à-vis du virus de l'hépatite C.

Un objet particulier de l'invention porte sur la particule sous-virale susmentionnée comprenant deux protéines de fusion susmentionnées, l'une comprenant :
- la protéine E1 du HCV (notamment E1-Sd, PIT1-E1-Sd, E1-E2-Sd, PIT-E1-E2-Sd), et la protéine E2 du HCV, greffée en N-terminal de la protéine S délétée de HBV, et l'autre
- la protéine E2 du HCV (notamment E2-Sd, PIT2-E2-Sd, E1-E2-Sd, PIT-E1-E2-Sd), greffée en N-terminal de la protéine S délétée de HBV.

Selon un aspect particulièrement avantageux de l'invention, la substance active de la composition immunogène susmentionnée, est constituée du mélange comprenant :
- au moins deux protéines de fusion suivante :
   * l'une constituée par :
      - la séquence d'acides aminés représentée par la SEQ ID N°8, ou
      - une séquence d'acides aminés présentant un pourcentage d'identité d'au moins 88%, notamment d'au moins 90%, particulièrement d'au moins 92%, et plus particulièrement d'au moins 95%, avec SEQ ID N°8, sous réserve que ladite séquence d'acides aminés soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus humain de l'hépatite B et/ou vis-à-vis du virus de l'hépatite C, ou
      - de la séquence d'acides aminés d'un variant synthétique, dérivé de la SEQ ID N°8, sous réserve que ladite séquence d'acides aminés dudit variant synthétique soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus humain de l'hépatite B et/ou vis-à-vis du virus de l'hépatite C ;
   * l'autre constituée par :
      - la séquence d'acides aminés représentée par la SEQ ID N°10, ou
      - une séquence d'acides aminés présentant un pourcentage d'identité d'au moins 86%, notamment d'au moins 88%, particulièrement d'au moins 90%, et plus particulièrement d'au moins 95%, avec SEQ ID N°10, sous réserve que ladite séquence d'acides aminés soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus humain de l'hépatite B et/ou vis-à-vis du virus de l'hépatite C, ou
      - de la séquence d'acides aminés d'un variant synthétique, dérivé de la SEQ ID N°10, sous réserve que ladite séquence d'acides aminés dudit variant synthétique soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus humain de l'hépatite B et/ou vis-à-vis du virus de l'hépatite C.
      - au moins deux molécules d'acides nucléiques hybrides suivantes :
   * l'une constituée par :
      - de la séquence d'acides nucléiques représentée par SEQ ID N°11, ou
      - d'une séquence d'acides nucléiques présentant une homologie d'au moins 78%, notamment d'au moins 80%, particulièrement d'au moins 85%, et plus particulièrement d'au moins 90%, avec ladite séquence SEQ ID N°11, sous réserve que la protéine codée par ladite séquence d'acides nucléiques soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis HCV et/ou HBV humain, ou
      - de la séquence d'acides nucléiques d'un variant synthétique dérivé ladite séquence SEQ ID N°11, sous réserve que la protéine codée par ladite séquence d'acides nucléiques dudit variant synthétique soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis HCV et/ou HBV humain, ou un vecteur susmentionné comprenant ladite molécule d'acides nucléiques hybride,
   * l'autre constituée par :
      - de la séquence d'acides nucléiques représentée par SEQ ID N°13, ou
      - d'une séquence d'acides nucléiques présentant une homologie d'au moins 80%, particulièrement d'au moins 85%, plus particulièrement d'au moins 90%, et plus particulièrement d'au moins 95%, avec ladite séquence SEQ ID N°13, sous réserve que la protéine codée par ladite séquence d'acides nucléiques soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis HCV et/ou HBV humain, ou
      - de la séquence d'acides nucléiques d'un variant synthétique, dérivé de ladite séquence SEQ ID N°13, sous réserve que la protéine codée par ladite séquence d'acides nucléiques dudit variant synthétique soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis HCV et/ou HBV humain ou un vecteur susmentionné comprenant ladite molécule d'acides nucléiques hybride;
- au moins deux particules sous-virales chimères suivantes :
   * l'une comprenant la protéine de fusion immunogène constituée par :
      - la séquence d'acides aminés représentée par la SEQ ID N°8, ou
      - une séquence d'acides aminés présentant un pourcentage d'identité d'au moins 88%, notamment d'au moins 90%, particulièrement d'au moins 92%, et plus particulièrement d'au moins 95%, avec la dite SEQ ID N°8, sous réserve que ladite séquence d'acides aminés soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis de HCV et/ou vis-à-vis de HBV humain, ou
      - de la séquence d'acides aminés d'un variant synthétique, dérivé de ladite SEQ ID N°8, sous réserve que ladite séquence d'acides aminés dudit variant synthétique soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis de HCV et/ou vis-à-vis de HBV humain,
   * l'une comprenant la protéine de fusion immunogène constituée par :
      - la séquence d'acides aminés représentée par la SEQ ID N°10, ou
      - une séquence d'acides aminés présentant un pourcentage d'identité d'au moins 86%, notamment d'au moins 88%, particulièrement d'au moins 92%, et plus particulièrement d'au moins 95%, avec SEQ ID N°10, sous réserve que ladite séquence d'acides aminés soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis de HCV. et/ou vis-à-vis de HBV humain, ou
      - de la séquence d'acides aminés a un variant synthétique, dérivé de la SEQ ID N°10,
sous réserve que ladite séquence d'acides aminés dudit variant synthétique soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis de HCV et/ou vis-à-vis de HBV humain.

Un objet particulier de l'invention porte sur le mélange comprenant les composés suivants :
a)- au moins deux protéines de fusion susmentionnées, l'une comprenant la protéine E1 (notamment E1-Sd de séquence SEQ ID N°8, ou PIT1-E1-Sd, ou E1-E2-Sd, ou PIT1-E1-E2-Sd) l'autre comprenant la protéine E2 (notamment E2-Sd de séquence SEQ ID N°10, ou PIT2-E2-Sd, ou E1-E2-Sd, ou PIT1-E1-E2-Sd) du HCV, ces dernières étant greffées en N-terminal de la protéine S délétée de HBV, ou
b)- au moins deux molécules d'acides nucléiques hybrides susmentionnées l'une comprenant la séquence d'acides nucléique codant pour la protéine E1 (notamment pit1-e1-sd de séquence SEQ ID N°11, ou e1-sd, ou e1-e2-Sd, ou pit1-e1-e2-sd), l'autre comprenant la séquence d'acides nucléique codant pour la protéine E1 (notamment pit2-e2-sd de séquence SEQ ID N°13, ou e2-sd, ou e1-e2-Sd, ou pit1-e1-e2-sd), ces dernières étant greffées en 5' de la séquence d'acides nucléique codant pour la protéine S délétée de HBV, ou
c)- au moins deux particules sous-virales susmentionnées, l'une comprenant la protéine E1 (notamment E1-Sd de séquence SEQ ID N°8, ou PIT1-E1-Sd, ou E1-E2-Sd, ou PIT1-E1-E2-Sd) l'autre comprenant la protéine E2 (notamment E2-Sd de séquence SEQ ID N°10, ou PIT2-E2-Sd, ou E1-E2-Sd, ou PIT1-E1-E2-Sd) du HCV, ces dernières étant greffées en N-terminal de la protéine S délétée de HBV.

L'invention a également pour objet l'utilisation d'une composition immunogène susmentionnée, pour la fabrication d'un médicament pour le traitement prophylactique et/ou pour la prévention de l'hépatite C.

La présente invention concerne également l'utilisation d'une composition immunogène susmentionnée, pour la fabrication d'un médicament pour le traitement prophylactique et/ou pour la prévention de l'hépatite B.

Avantageusement l'objet de la présente invention porte sur l'utilisation d'une composition immunogène susmentionnée, pour la fabrication d'un médicament pour le traitement prophylactique et/ou pour la prévention de l'hépatite B et de l'hépatite C.

L'invention a également pour objet une lignée cellulaire qui exprime des particules sous virales chimère, immunogènes non infectieuses précédemment décrites.

A titre d'exemples de microorganisme qui conviennent aux fins de l'invention, on peut citer les levures, telles que celles des familles suivantes : Saccharomyces, Schizosaccharomyces, Kluveromyces, Pichia, Hanseluna, Yarowia, Schwaniomyces, Zygosaccharomyces, Saccharomyces cerevisiae, Saccharomyces carlsbergensis et Kluveromyces lactis étant préférées ; et les bactéries, telles que E. coli et celles des familles suivantes : Lactobacillus, Lactococcus, Salmonella, Strptococcus, Bacillus et Streptomyces.

A titre d'exemples de cellules eucaryotes, on peut citer les cellules provenant d'animaux tels que les mammifères, les reptiles, les insectes et équivalent. Les cellules eucaryotes préférées sont les cellules provenant du hamster chinois (cellules CHO), du singe (cellules COS et Vero), du rein de hamster nain (cellules BHK), du rein de cochon (cellules PK 15) et du rein de lapin (cellules RK13, les lignées cellulaires humaines de l'ostéosacorme (cellules 143 B), les lignées cellulaires humaines HeLa et les lignées cellulaires humaines de l'hépatome (du type cellules Hep G2), ainsi que les lignées cellulaires d'insecte (par exemple de Spodoptera frugiperda).

Avantageusement l'objet de la présente invention porte sur une lignée cellulaire susmentionnée, laquelle est la lignée d'ovaire de hamster chinois nommée CHO.

Ces derniers ont été antérieurement décrits notamment par Michel ML, Pontisso P, Sobczak E, Malpièce Y, Streeck RE, Tiollais P. Synthesis in animal cells of hepatitis B surface antigen particles carrying a receptor for polymerized human serum albumin. Proc Natl Acad Sci U S A. 1984 Dec;81(24):7708-12:

La présente invention concerne également sur une lignée cellulaire susmentionnée, laquelle est une levure, ladite levure pouvant être notamment *Saccharomyces Cerevisae.*

L'invention a également pour objet une lignée cellulaire décrite ci-dessus, laquelle est la lignée cellulaire de rein de hamster nouveau-né (BHK), et est particulièrement la lignée cellulaire de rein de hamster nouveau-né (BHK-21).

Ces derniers ont été antérieurement décrits notamment par Goldman RD, Follett EA. Biréfringent filamentous organelle in BHK-21 cells and its possible role in cell spreading and motility. Science. 1970 Jul 17;169(942):286-8.

Selon un aspect particulier, l'invention porte également sur une méthode de production des particules sous virales chimères, immunogènes non infectieuses susmentionnées, à partir d'une lignée cellulaire susmentionnée, comprenant les étapes suivantes:
1- une étape de TRANSDUCTION des cellules de la lignée cellulaire avec un vecteur lentiviral comprenant une séquence d'acides nucléiques codant pour la protéine S sauvage d'un isolat du virus de l'hépatite B,
2- une étape de CULTURE desdites cellules afin de produire une lignée cellulaire capable d'exprimer les particules sous-virales d'enveloppe sauvage du virus de l'hépatite B.
3- une étape de SELECTION d'un clone présentant une secrétion optimale de particules sous-virales d'enveloppe sauvage du virus de l'hépatite B,
4- une étape de SUR-TRANSDUCTION dudit clone avec un vecteur susmentionné,
5- une étape de CULTURE desdites cellules afin de produire une lignée cellulaire capable d'exprimer les particules sous virales chimères, immunogènes non infectieuses susmentionnées,
6- une étape de SELECTION desdites cellules capables de secréter de manière optimale les particules sous virales chimères, immunogènes non infectieuses susmentionnées,
7- une étape de CULTURE dudit clone pour la production des particules sous virales chimères, immunogènes non infectieuses susmentionnées, et
8- une étape de PURIFICATION des particules sous virales à partir du milieu de culture collecté (centrifugation, utltracentrifugation sur gradients, collecte des fractions positives pour les particules sous-virales chimères, dyalise).

Le tableau 1 fournit une liste des séquences d'acides nucléiques et d'acides aminés décrites dans la présente invention. Par convention, les séquences d'acides nucléiques sont libellées en minuscules et les séquences d'acides aminés sont libellées et majuscules.

**Tableau 1 : LISTE DE SEQUENCES**

| **Acides nucléiques** | **Acides aminés** |
|---|---|
| SEQ ID N° 1 : sd | SEQ ID N° 2 : Sd |
| SEQ ID N° 3 : e1 | SEQ ID N° 4 : E1 |
| SEQ ID N° 5 : e2 | SEQ ID N° 6 : E2 |
| SEQ ID N° 7 : e1-sd | SEQ ID N° 8 : E1-Sd |
| SEQ ID N° 9 : e2-sd | SEQ ID N° 10 : E2-Sd |
| SEQ ID N° 11 : pit1-e1-sd | SEQ.ID N° 12 : PIT1-E1-Sd |
| SEQ ID N° 13 : pit2-e2-sd | SEQ ID N° 14 : PIT2-E2-Sd |
| SEQ ID N° 15 : Vecteur avec pit1-e1-sd | / |
| SEQ ID N° 16 : Vecteur avec pit2-e2-sd | / |

D'autres caractéristiques et avantages de l'invention apparaîtront plus clairement à la lecture de la description suivante d'un mode de réalisation préférentiel, donné à titre de simple exemple illustratif et non limitatif, et des figues annexées, parmi lesquelles :
La **Figure 1** schématise la topologie d'un fragment de la polyprotéine du virus HCV de plus de 3 000 acides aminés. Les clivages effectués par les peptidases cellulaires ( 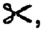 ) aboutissent à la maturation de ladite polyprotéine notamment en protéines structurales (capside, enveloppe E1 et E2). Les nombres indiquent la position des résidus d'acides aminés délimitant les domaines des différentes protéines structurales de la polyprotéine.
Les **Figures 2A et 2B** schématise la typologie transmembranaire de la protéine S sauvage du virus HBV.
   - La **Figure 2A** schématise les 226 résidus d'acides aminés de la protéine S sauvage du virus HBV qui comprend notamment quatre domaines transmembranaires, représentés par des rectangles verticaux gris, et dont les extrémités N- et C-terminales sont orientées vers la lumière du RE. Dans le cas de l'isolat HBVadw, cette protéine d'environ 25 kD comporte 226 résidus d'acides aminés.
   - La **Figure 2B** représente une particule sous-virale obtenue suite à l'auto-assemblage de ladite protéine S sauvage.
Les **Figures 3A à 3C** représentent la co-expression des protéines de fusion E1-Sd (Figure 3B) ou E2-Sd (Figure 3C) qui, en présence de la protéine S sauvage (Figure 3B) s'assemble en particules sous-virales chimères de la présente invention.
   - La **Figure 3A** représente la protéine S sauvage de HBV qui s'auto-assemble en particules sous-virales.
   - La **Figure 3B** représente la protéine de fusion E1-Sd qui résulte de la substitution du domaine transmembranaire situé à l'extrémité N-terminale de la protéine S sauvage par le domaine transmembranaire de la protéine d'enveloppe E1 du virus HCV, représenté par un rectangle vertical noir, et par l'ectodomaine de E1, représenté par des pointillés; puis schématise l'assemblage de ladite protéine de fusion E1-Sd, en présence de la protéine S sauvage, en particules sous-virales S + E1-Sd.
   Selon le mode de réalisation particulier mais non limitatif de l'invention, la protéine de fusion E1-Sd d'environ 50 kD comporte 393 résidus d'acides aminés issus de la protéine E1 et du domaine S. Les résidus 192 à 380 de la protéine complète E1 sont liés à l'extrémité N-terminale de la protéine S délétée constituée des résidus 23 à 226 de la protéine S sauvage. - La **Figure 3C** représente la protéine de fusion E2-Sd qui résulte de la substitution du domaine transmembranaire situé à l'extrémité,N-terminale de la protéine S sauvage par le domaine transmembranaire de la protéine d'enveloppe E2 du virus HCV, représenté par un rectangle vertical blanc, et par l'ectodomaine de E2, représenté par des pointillés; puis schématise l'assemblage de ladite protéine de fusion E2-Sd, en présence de la protéine S sauvage, en particules sous-virales S + E2-Sd.
   Selon le mode de réalisation particulier mais non limitatif de l'invention, la protéine de fusion E2-Sd d'environ 85 kD comporte 564 résidus d'acides aminés issus de la protéine E2 et du domaine S. Les résidus 384 à 743 de la protéine complète E2 sont liés à l'extrémité N-terminale de la protéine S délétée constituée des résidus 23 à 226 de la protéine S sauvage.
Les **Figures 4A à 4E** schématisent la topologie des protéines d'enveloppe S sauvage du HBV, E1 et E2 du HCV, ainsi que les protéines de fusion E1-Sd et E2-Sd.
   - La **Figure 4A** représente la topologie de la protéine d'enveloppe sauvage E1 du HCV qui est constituée d'un domaine transmembranaire unique (rectangle verticale noir) et un large ectodomaine orienté vers la lumière du RE. Dans le cas de l'isolat HCV 1a, cette protéine d'environ 35 kD et comporte 192 résidus d'acides aminés.
   - La **Figure 4B** représente la topologie de la protéine d'enveloppe sauvage E2 du HCV qui est constituée d'un domaine transmembranaire unique (rectangle verticale blanc) et un large ectodomaine orienté vers la lumière du RE. Dans le cas de l'isolat HCV 1a, cette protéine d'environ 70 kD et comporte 363 résidus d'acides aminés.
   - La **Figure 4C** représente la topologie de la protéine d'enveloppe sauvage S du HBV qui comprend notamment quatre domaines transmembranaires (rectangles verticaux gris).
   - La **Figure 4D** représente la séquence d'ADNc de la molécule d'acides nucléiques hybride pit1-e1-sd, constituée de son extrémité 5' à 3' :
   - de la séquence codant pour le peptide d'initiation de transfert PIT1 du virus HCV,
   - la séquence codant pour l'intégralité de l'ectodomaine de la protéine E1 et la séquence codant pour le domaine transmembranaire de la protéine E1 délétée des 3 derniers codons de l'extrémité 3' du virusHCV,
   - de la séquence codant pour la protéine S délétée du virus HBV,
      et son utilisation pour la production de la protéine de fusion E1-Sd de l'invention.
   - La **Figure 4E** représente la séquence d'ADNc de la molécule d'acides nucléique hybride pit2-e2-sd, constituée de son extrémité 5' à 3' :
   - de la séquence codant pour le peptide d'initiation de transfert PIT2 du virus HCV,
   - la séquence codant pour l'intégralité de l'ectodomaine de la protéine E2, la séquence codant pour le domaine transmembranaire de la protéine E2 délétée des 3 derniers codons de l'extrémité 3'-du virus HCV,
   - de la séquence codant pour la protéine S délétée du virus HBV,
      et son utilisation pour la production de la protéine de fusion E2-Sd de l'invention.

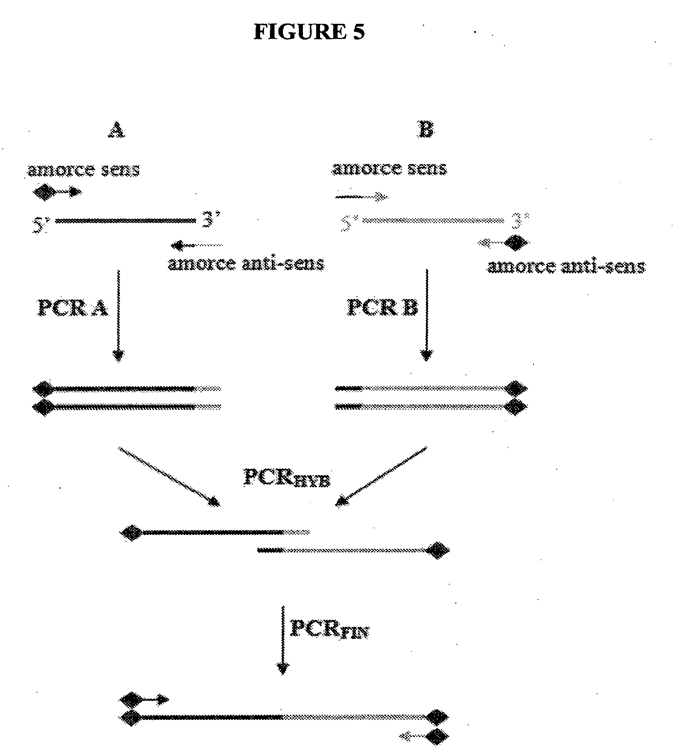

La **Figure 5** schématise le protocole utilisé pour l'obtention des ADNc codant pour les protéines de fusion de l'invention. La séquence codant pour les protéines E1 et E2 est représentée par un trait noir (séquence A). La séquence codant pour la protéine S délétée est représentée par un trait gris (séquence B). Le site de restriction BamHI est représenté par un losange noir. Les amorces sens et anti-sens utilisées pour l'amplification des séquences d'acides nucléiques e1-sd A, e1-sd B, e2-sd A et e2-sd B sont répertoriées dans le tableau x.

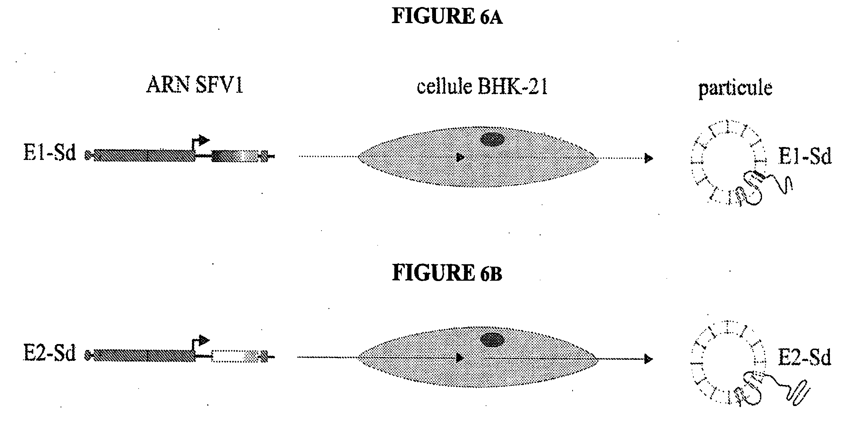

Les **Figures 6A et 6B** proposent une représentation schématique du protocole expérimental de production transitoire d'une des protéines de fusion de l'invention E1-Sd ou E2-Sd en système Semliki / cellules BHK-21.
   - La **Figure 6A** montre que l'ARN dérivé du plasmide pSFV1-E1-Sd est transfectée dans des cellules BHK-21 dans le but d'obtenir la production de de particules sous-virales d'enveloppe chimériques E1-Sd.
   - La **Figure 6B** montre que l'ARN dérivé du plasmide pSFV1-E2-Sd est transfecté dans des cellules BHK-21 dans le but d'obtenir la production de particules sous-virales d'enveloppe chimériques E2-Sd.

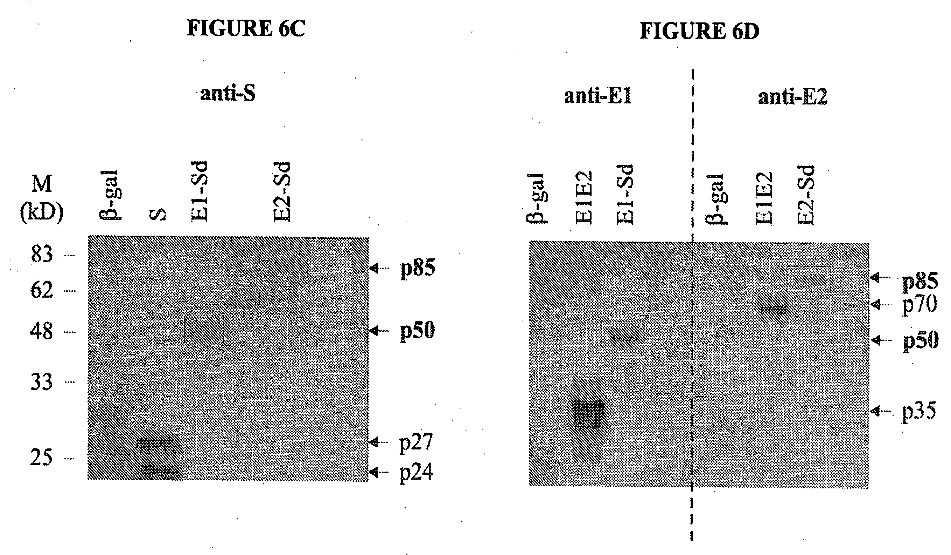

Les **Figures 6C et 6D** montrent les Western-blot révélés soit par un anticorps anti-S (6C), soit par un anticorps anti-E1 et anti-E2 (6D), sur les lysats des cellules transfectées par les ARN dérivés des plasmides pSFV1-E1-Sd et pSFV1-E2-Sd.
   - La **Figure 6C** montre que les protéines de fusion E1-Sd et E2-Sd (bandes encadrées) sont détectées aux tailles de 50 kD et 85 kD respectivement, et correspondant aux tailles théoriques. M : marqueur de poids moléculaire (kD).
   - La **Figure 6D** montre que les protéines de fusion E1-Sd et E2-Sd (bandes encadrées) sont détectées aux tailles de 50 kD et 85 kD respectivement, par l'anticorps anti-E1 et anti-E2 respectivement. M : marqueur de poids moléculaire (kD).

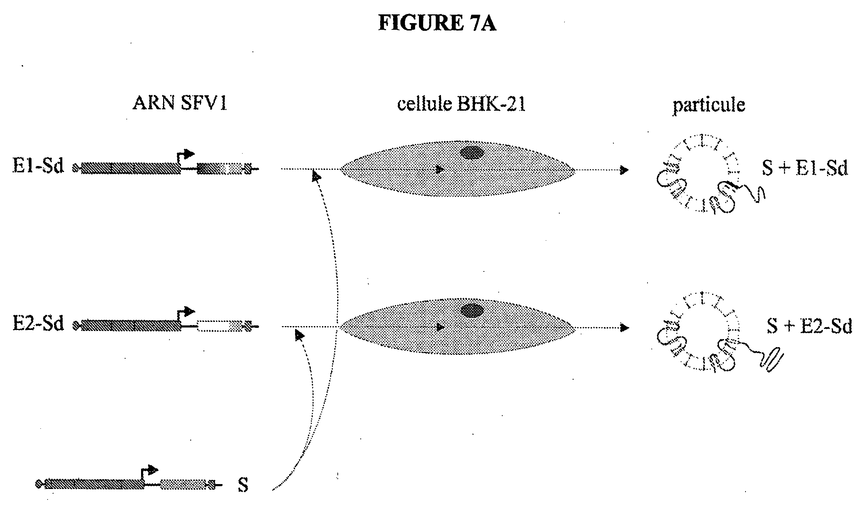

Les **Figure 7A à 7B** montrent la co-production de la protéine S sauvage du HBV et des deux protéines de fusion E1-Sd et E2-Sd en système Semliki / cellules BHK-21.
   Légende : M : marqueur de poids moléculaire (kD) ; les pistes β-gal, E1E2 ou E1E2 et S représentent les lysats cellulaires témoins issus de transfections de cellules BHK-21 par les ARN SFV correspondants.
   - La **Figure 7A** propose une représentation schématique du protocole expérimental. Les ARN dérivés des plasmides pSFV1-E1-Sd et pSFV1-E2-Sd sont co-transfectés individuellement dans des cellules BHK-21 avec l'ARN dérivé du plasmide pSFV1-S dans le but d'obtenir la production de deux types de particules sous-virales d'enveloppe chimériques S+E1-Sd et S+E2-Sd, la première comprenant la protéine S sauvage et la protéine de fusion E1-Sd, la seconde comprenant la protéine S sauvage et la protéine de fusion E2-Sd.

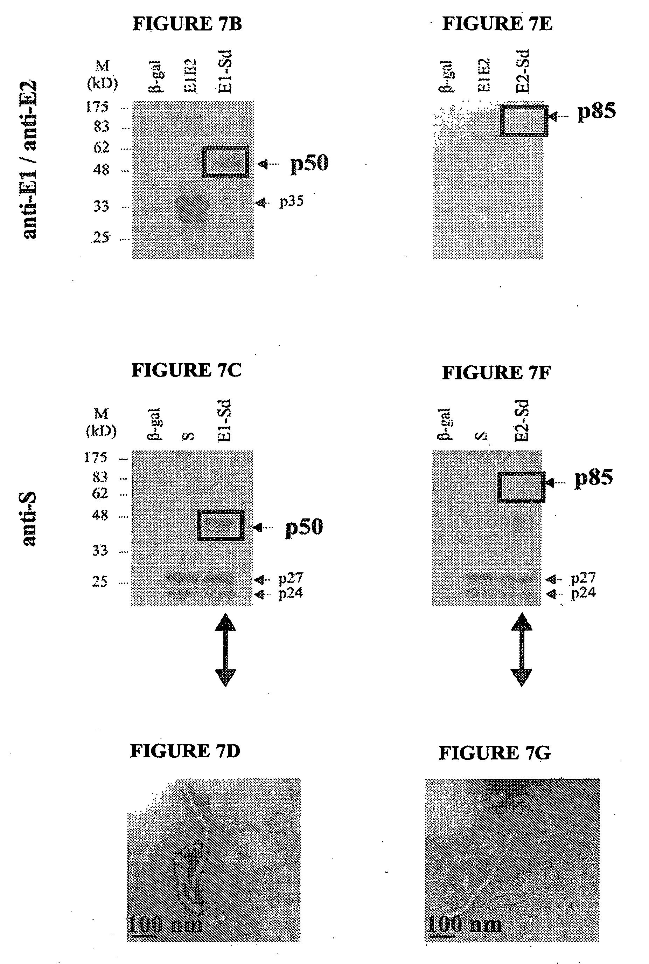

   - La **Figure 7B** montre un Western-blot révélé par un anticorps anti-E1 sur les particules intracellulaires purifiées à partir des cellules transfectées par les ARN dérivés des vecteurs pSFV1-E1-Sd et pSFV1-S. Les résultats montrent que la protéine de fusion E1-Sd (bande encadrée) est détectée à la taille de 50 kD correspondant à la taille théorique.
   - La **Figure 7C** montre un Western-blot révélé par un anticorps anti-S sur les particules intracellulaires purifiées à partir des cellules transfectées par les ARN dérivés des vecteurs pSFV1-E1-Sd et pSFV1-S. Les résultats montrent que la protéine de fusion E1-Sd (bande encadrée) est détectée à la taille de 50 kD correspondant à la taille théorique.
   - La **Figure 7D** montre une micrographie en microscopie électronique à transmission par coloration négative sur les particules intracellulaires purifiées à partir des cellules transfectées par les ARN dérivés des plasmides pSFV1-E1-Sd et pSFV1-S. Barre : 100 nm.
   - La **Figure 7E** montre un Western-blot révélé par un anticorps anti-E2 sur les particules intracellulaires purifiées à partir des cellules transfectées par les ARN dérivés des plasmides pSFV1-E2-Sd et pSFV1-S. Les résultats montrent que la protéine de fusion E2-Sd (bande encadrée) est détectée à la taille de 85 kD correspondant à la taille théorique.
   - La **Figure 7F** montre un Western-blot révélé par un anticorps anti-S sur les particules intracellulaires purifiées à partir des cellules transfectées par les ARN dérivés des plasmides pSFV1-E2-Sd et pSFV1-S. Les résultats montrent que la protéine chimère E2-Sd (bande encadrée) est détectée à la taille de 85 kD correspondant à la taille théorique.
   - La **Figure 7G** montre une micrographie en microscopie électronique à transmission par coloration négative sur les particules intracellulaires purifiées à partir des cellules transfectées par les ARN dérivés des plasmides pSFV1-E2-Sd et pSFV1-S. Barre : 100 nm.

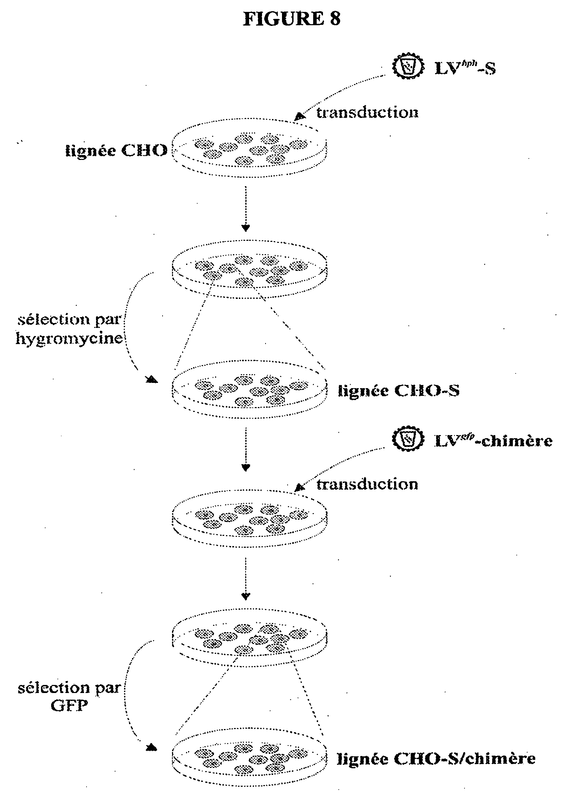

La **Figure 8** représente le protocole d'obtention des lignées clonales CHO produisant 1a protéine S sauvage et les protéines de fusion de l'invention. Elle schématise comment à partir d'une lignée cellulaire CHO, la transduction par un lentivirus (LV) contenant un gène de résistance à l'hygromycine *(hph)* ainsi que le gène codant pour la protéine S sauvage du HBV a permis le développement d'un premier clone cellulaire nommé CHO-S et producteur de la protéine S sauvage. Ce clone a ensuite été sur-transduit par un lentivirus (LV) contenant le gène de la GFP (*gfp*) ainsi que le gène codant pour une des deux protéines de fusion E1-Sd-ou E2-Sd. Cette seconde transduction a permis le développement de deux autres clones cellulaires nommés CHO-S/E1-Sd et-S/E2-Sd..

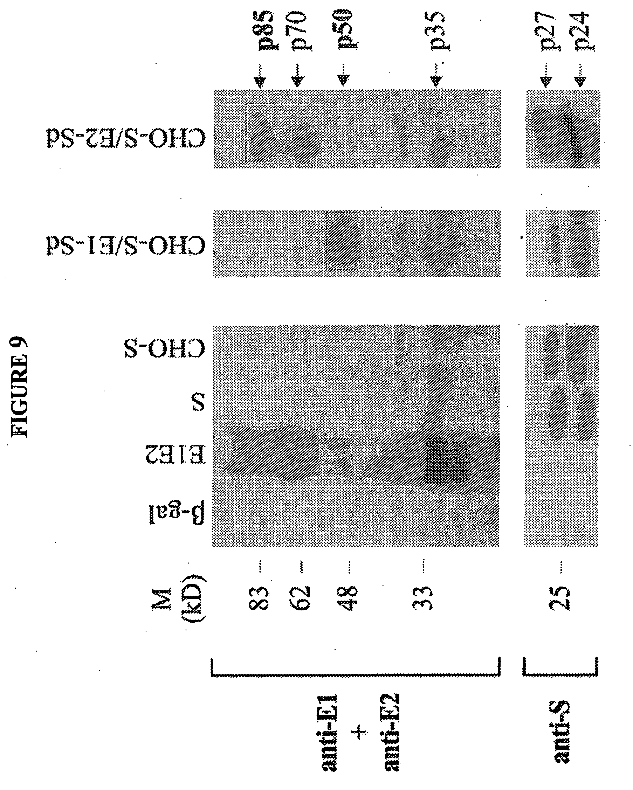

La **Figure 9** montre une analyse par Western-blot de la production intracellulaire la protéine S sauvage et les protéines de fusion de l'invention dans les différents clones stables CHO. Elle montre que les protéines intracellulaires des clones CHO-S, CHO-S/E1-Sd et CHO-S/E2-Sd sont révélés à l'aide d'anticorps anti-E1, anti-E2 et anti-S. Les protéines de fusion recherchées sont indiquées par des cadres noirs. M : marqueur de poids moléculaire (kD) ; pistes β-gal, E1E2 et S : lysats cellulaires témoins issus de transfections de cellules BHK-21 par les ARN SFV correspondants.

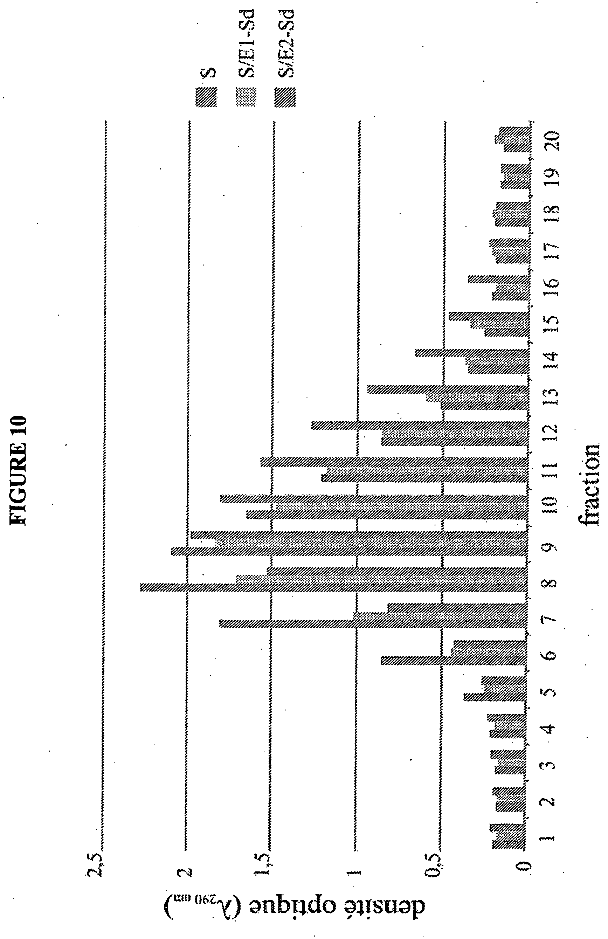

La **Figure 10** représente un histogramme de quantification de la protéine S sauvage par ELISA dans les fractions collectées à partir du gradient de CsCl. L'abscisse montre les différentes fractions collectées, l'ordonnée indique la densité optique à 290 nm de chacune d'elle. A partir des surnageant de culture des clones CHO-S, CHO-S/E1-Sd et CHO-S/E2-Sd, les particules sous-virales S et chimériques sont purifiées par ultracentrifugation en CsCl. Des fractions sont collectées à partir du sommet du gradient (fraction 1) et la quantité de protéine S sauvage à été évaluée par ELISA. L'histogramme noir représente la répartition de la protéine S sauvage dans les fractions du clone CHO-S, l'histogramme gris clair représente la répartition de la protéine S sauvage dans les fractions du clone CHO-S/E1-Sd et l'histogramme gris foncé représente la répartition de la protéine S sauvage dans les fractions du clone CHO-S/E2-Sd.

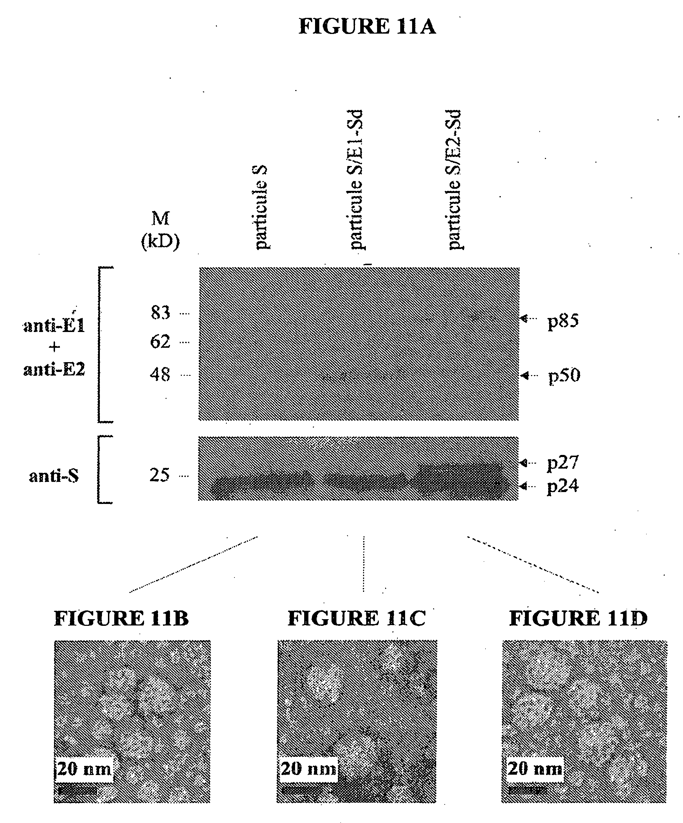

La **Figure 11A** montre que le contenu protéique des particules purifiées par CsCl a été analysé par Western-blot à l'aide d'anticorps anti-E1, anti-E2 et anti-S. La protéine S sauvage est détectée sous ses deux états de glycosylation (24 et 27 kD) dans les trois types de particules sous-virales. Les protéines de fusion E1-Sd ou E2-Sd sont détectées aux tailles correspondant à la taille théorique respectivement de 50 et 85 kD dans leur particule respective. M : marqueur de poids moléculaire (kD).
La **Figure 11B** montre l'analyse en coloration négative par microscopie électronique à transmission de la purification, à partir du surnageant du clone CHO-S, de particules sous virales résultant de l'auto-assemblage de la protéine S sauvage de type sphériques d'environ 20 nm de diamètre. Barre : 20 nm.
La **Figure 11C** montre l'analyse en coloration négative par microscopie électronique à transmission de la purification, à partir du surnageant du clone CHO-S/E1-Sd, de particules sous virales chimère résultant de l'assemblage de la protéine de fusion E1-Sd en présence de la protéine S sauvage. Ces particules de type sphériques font environ 20 nm de diamètre. Barre : 20 nm.
La **Figure 11D** montre l'analyse en coloration négative par microscopie électronique à transmission de la purification, à partir du surnageant du clone CHO-S/E2-Sd, de particules sous virales chimère résultant de l'assemblage de la protéine de fusion E1-Sd en présence de la protéine S sauvage. Ces particules de type sphériques font d'environ 20 nm de diamètre. Barre : 20 nm.

A titre d'exemple non limitatif, on présente ci-après un mode de réalisation particulier de l'invention, pour d'obtention de particules sous-virales d'enveloppe du virus de l'hépatite C (HCV) en système Semliki (cf figures 4 à 7).

Ce mode de réalisation repose sur l'obtention de protéines de fusion immunogènes de l'invention et en particulier des protéines de fusion E1-Sd ou E2-Sd, comprenant d'une part la protéine d'enveloppe S délétée en N-terminal de son domaine transmembranaire (Sd), du virus de l'hépatite B (HBV) et d'autre part la quasi intégralité de l'une des protéines d'enveloppe E1 ou E2 du HCV. Ce mode de réalisation transitoire démontre la capacité d'assemblage de ladite protéine S délétée (Sd), en présence de la protéine S sauvage, permet d'obtenir des particules sous-virales chimères susmentionnées, grâce notamment au haut niveau d'expression du vecteur basé sur les propriétés de réplication du virus de la foret de Semliki (vecteur pSFV), avant de procéder à l'obtention de clones stables et producteurs desdites particules en utilisant des vecteurs lentiviraux.

### Exemple I : Obtention de particules sous-virales chimère d'enveloppe du virus de l'hépatite C en système Semliki (pSFV)

### I.1) Construction des plasmides pSFV1-E1-Sd et pSFV1-E2-Sd.

Le vecteur pSFV1 (Invitrogen) possédant une structure bicistronique de 11033 pb est utilisé pour les constructions suivantes. Ce vecteur est doté d'une séquence promotrice de la SP6-ARN-polymérase, insérée en 5' du premier cistron, afin de pouvoir initier la synthèse d'un ARN complet de polarité positive (ARN nommé 42S(+)) par transcription *in vitro.* Après transfection dans des cellules de mammifère, ces ARN recombinants coiffés *in vitro* s'auto-répliquent en présence de la réplicase nsP1-4 du SFV et servent à la production de protéines d'intérêt par l'intermédiaire d'un ARNm secondaire nommé 26S(+).

L'obtention de l'ADN complémentaire (ADNc) sauvage ou d'une molécule d'acides nucléiques hybride codant pour une protéine de fusion de l'invention s'effectue en réalisant plusieurs séries d'amplifications par réactions de polymérisation en chaîne (PCR). A partir du plasmide pSFV1-E1E2 (consistant en un vecteur SFV contenant la séquence codant pour les deux protéines E1 et E2 du HCV), une première PCR, nommée PCR A, a permis l'amplification des séquences codant pour les protéines d'enveloppe E1 ou E2 du HCV. avec leur domaine transmembranaire et précédées des séquences codant pour leur peptide d'initiation de transfert respectif d'adressage au réticulum endoplasmique. A partir du plasmide pHBV1.5 décrit antérieurement [Patient, R., Hourioux, C., Sizaret, P.Y., Trassard, S., Sureau, C., and Roingeard, P., (2007) Hepatitis B Virus Subviral Envelope Particle Morphogenesis and Intracellular Trafficking J Virol, 81(8), 3842-51], une seconde PCR, nommée PCR B a permis l'amplification de la séquence codant pour la protéine S délétée du HBV. Les parties « HCV » des séquences codant pour les protéines de fusion selon l'invention ont été nommées « A » et les parties « HBV » ont été nommées « B » (figure 5). Les produits des PCR A et B ont été quantifiés et purifiés sur gel pour être ensuite inclus à part égale dans des PCR finales. L'utilisation d'amorces bivalentes HCV-HBV a permis la synthèse de produits de PCR A et B possédant des domaines chevauchants à leur extrémités permettant, en final, d'obtenir les séquences d'ADNc complètes codant pour les protéines de fusion de l'invention.

### I1_{.}1) Amplification des séquences d'ADNc codant pour les protéines de fusion de l'invention (notamment E1-Sd et E2-Sd) :

Les amplifications par PCR s'effectuent dans un milieu réactionnel de 50 µL contenant 50 ng d'ADN, 15 pmol de chaque couple d'amorces (Proligo), 200 µM de chacun des désoxyribonucléotides dNTP (Invitrogen) et 10 unités de Taq-ADN-polymérase (Takara) comportant une activité correctrice de façon à minimiser les erreurs liées aux amplifications successives (figure 5). A l'aide des logiciels « Vector NTI Advance 10 » (Invitrogen) et « Primer premier » (Biosoft International), les amorces « sens » des séquences A et les amorces « anti-sens » des séquences B ont été définies de manière à générer un site BamHI aux extrémités respectives 5' et 3' des fragments d'ADN amplifiés.

Le tableau 2 « **Oligonucléotides utilisés pour l'amplification génique des séquences A et B constitutives des séquences chimères »** ci-dessous indique les séquences d'amorces utilisées.

Les séquences correspondant au HCV y sont représentées en écriture noire, celles correspondant au HBV en écriture noire soulignée, celles correspondant au site de restriction BamHI en écriture italique soulignée, celles correspondant au codon ATG d'initiation de la traduction en écriture grasse, celles correspondant au codon ATT de terminaison de la traduction en écriture grasse.

**Le tableau 2 « Oligonucléotides utilisés pour l'amplification génique des séquences A et B constitutives des séquences chimères »**

| **fragment d'ADNc amplifié** | **couple d'amorce** | **séquence de l'amorce** |
|---|---|---|
| e1-sd A | sens anti-sens | |
| e1-sd B | sens anti-sens | |
| e2-sd A | sens anti-sens | |
| e2-sd B | sens anti-sens | |

Ces réactions d'amplification effectuées en thermocycleur « iCycler » (Biorad) ont consisté en une dénaturation initiale de 5 minutes à 95 °C, suivie de 25 cycles comprenant une dénaturation de 1 minute à 95 °C, une hybridation de 1 minute à 60 °C et une élongation de 1 minute à 69 °C. Les 25 cycles sont suivis d'une élongation finale de 10 minutes à 69 °C. Les fragments amplifiés par les PCR A et B sont purifiés sur gel d'agarose 1 % à l'aide du système « Wizard SV Gel and PCR Clean-Up System » (promena) selon les recommandations du fabricant. Les fragments purifiés des PCR A et B sont ensuite hybridés par une PCR nommée PCRHYB en absence d'amorces pendant 10 cycles puis sont amplifiés par une PCR finale nommée PCRFIN pendant 25 cycles en présence des amorces « sens » des séquences A et les amorces « anti-sens » des séquences B. Les programmes des cycles de ces deux dernières PCR sont similaires à ceux utilisés pour les PCR A et B. Les fragments amplifiés de molécules d'acides nucléiques hybrides de l'invention ainsi obtenus sont purifiés sur gel comme ci-dessus.

### I.1.2) clonage des séquences d'ADNc hybrides dans le vecteur DSFV1

Les produits de PCRFIN purifiés sont clonés dans le vecteur pGEM-T® (« pGEM-T Easy Vector System », Promega) selon les recommandations du fabricant, Les fragments de molécules d'acides nucléiques hybrides de l'invention sont libérés du pGEM-T® par restriction avec l'enzyme BamHI (Biolabs) puis clonés au niveau du site BamHI du plasmide SFV1. Les différents plasmides comprenant les protéines de fusion de l'invention (notamnient les plaamides pSFV1-E1-Sd et pSFV1-E2-Sd) sont amplifiés par transformation bactérienne puis purifiés par maxipréparation d'ADN au phénol/chloroforme. L'orientation de l'insert est vérifiée par restriction enzymatique et l'ensemble des constructions est vérifiée par séquençage.

### I-2) Obtention de lignée cellulaire transfectées transitoirement par les ARN des différentes constructions SFV

Les procédures de culture des cellules de rein de hamster nouveau-né (BHK-21) ainsi que les protocoles de transcription in vitro des plasmides matrices SFV et de transfection des ARN recombinants auto-réplicatifs étaient identiques à ceux décrits antérieurement [Patient, R., Hourioux, C., Sizaret, P.Y., Trassard, S., Sureau, C., and Roingeard, P., (2007) Hepatitis B Virus Subviral Envelope Particle Morphogenesis and Intracellular Trafficking J Virol, 81(8), 3842-51]. La construction pSFV-S, exprimant la protéine S sauvage du HBV et antérieuremnt décrite dans l'article précédent, a été utilisée comme contrôle.

### I-3) Analyse de la production intracellulaire des protéines d'enveloppe sauvages et chimériques

Les procédures d'analyses biochimiques des protéines d'intérêt (notamment S, Sd, E1, E2, E1-Sd, E2-Sd etc..) par immunofluorescence en microscopie confoncale et par Western Blot, les procédures d'analyses ultra-structurales des cellules transfectées en microscopie électronique à transmission ainsi que les procédures de quantification (ELISA) / purification (gradient de sucrose puis chromatographie d'affinité) des particules sous-virales d'enveloppe chimériques HCV-HBV sont celles décrites antérieurement [Patient, R., Hourioux, C., Sizaret, P.Y., Trassard, S., Sureau, C., and Roingeard, P:, (2007) Hepatitis B Virus Subviral Envelope Particle Morphogenesis and Intracellular,Traffcking J Virol, 81(8), 3842-51].

La détection des protéines sauvages E1 et E2 de HCV et des protéines de fusion de l'invention est réalisée à l'aide d'un anticorps monoclonal murin anti-E1 (A4, fourni par le Dr Harry Greenberg, Université de Stanford, Californie) [Dubuisson, J., Hsu, H. H., Cheung, R. C., Greenberg, H. B., Russell, D. G., and Rice, C. M. (1994). Formation and intracellular localization of hepatitis C virus envelope glycoprotein complexes expressed by recombinant vaccinia and Sindbis viruses. J Virol 68(10), 6147-60)] ou anti-E2 (H52, fourni par le Dr Jean Dubuisson, Institut Pasteur de Lille) [Deleersnyder, V., Pillez, A., Wychowski, C., Blight, K., Xu, J., Hahn, Y. S., Rice, C. M., and Dubuisson, J. (1997). Formation of native hepatitis C virus glycoprotein complexes. J Virol 71(1), 697-704].

### I-4 ) Analyse du surnageant de culture.

Après transfection, le surnageant de culture d'environ 10⁷ cellules transfectées est nettoyé par une centrifugation de 10 minutes à 1500 g puis est ultracentrifugé à 4 °C pendant 16 heures à 35.000 rpm à l'aide d'un rotor SW41 (L70 Ultracentrifuge, Beckman). Le culot a est avec 50 µL du tampon de lyse puis analysé par Western-blot.

### I.5 ) Production de protéines de fusion E1-Sd et E2-Sd de l'invention

Seize heures après la transfection par les plasmides pSFV1 comprenant les molécules d'acides nucléiques hybrides de l'invention, pit1-e1-sd, pit2-e2-sd, les cellules BHK-21 ont été lysées puis analysées par Western-blot à l'aide d'anticorps monoclonaux anti-E1 et anti-E2. Après production transitoire en cellule BHK-21, la taille des protéines de fusion E1-Sd et E2-Sd sont d'environ 50 kD pour la protéine E1-Sd et d'environ 85 kD pour la protéine E2-Sd. Ces résultats, corrélés à l'intense immunofluorescence obtenue par la détection desdites protéines de fusion de l'invention avec des anticorps anti-E1, anti-E2 et anti-S, montrent qu'elles sont correctement produites, correctement glycosylées, et donc disposées selon la topologie transmembranaire souhaitée (figure 6A à 6D).

De façon à restaurer la capacité de sécrétion des différentes protéines de fusion de l'invention, des co-transfections sont réalisées en apportant en *trans* la forme sauvage de la protéine S du HBV à chacune des protéines de fusion de l'invention (figure 7A). Seize heures après la transfection les cellules co-tranfectées sont broyées et les particules sous-virales intracellulaires ont été purifiées par gradient de sucrose puis chromatographie d'affinité. comme décrit antérieurement [Patient, R., Hourioux, C., Sizaret, P.Y., Trassard, S., Sureau, C., and Roingeard, P., (2007) Hepatitis B Virus Subviral Envelope Particle Morphogenesis and Intracellular-Trafficking J Virol, 81(8), 3842-51]. Dans tous les cas, les particules sous-virales sont étudiées par microscopie électronique à transmission et par Western-blot en utilisant les anticorps anti-S, anti-E1 ou anti-E2 décrits précédemment dans l'exemple 1.3 (figures 7B à 7G).

Les images de microscopie électronique à transmission montrent que dans toutes ces expériences de co-production de la protéine S sauvage avec une des protéines de fusion de l'invention, il est possible de produire une quantité importante des particules sous-virales, sphériques et filamenteuses. Les analyses en Western-blot montrent que ces particules sous-virales plus ou moins filamenteuses sont riches en protéines de fusion de l'invention.

La mise en oeuvre selon l'exemple 1 de la présente invention en système « Semliki » montre que les protéines de fusion de l'invention contenant la quasi-intégralité des protéines E1 ou E2 du HÇV (leur domaine transmembranaire se substituant à celui situé en N-terminal de la protéine S du HBV) s'assemblent en particules sous-virales chimères de même nature que les particules sous-virales utilisées dans la production de vaccins contre l'hépatite B, facilitant ainsi la purification desdites particules sous-virales chimères de l'invention, et potentiellement, le développement d'une application industrielle d'un vaccin contre le HCV, calquée sur celle du vaccin contre le HBV.

La mise en oeuvre selon l'exemple 1 de la présente invention montre également la production de protéines de fusion comprenant les protéines E1 et/ou E2 non tronquées de HCV. Cette caractéristique peut s'avérer déterminante pour induire une réponse immunitaire neutralisante et cellulaire optimale.

Cependant, en système « Semliki », les particules sous-virales chimères sont produites par transfection transitoire des cellules. En effet, la forte cytotoxicité de ces vecteurs ne permet pas d'obtenir une sécrétion efficace sur le long terme des particules sous-virales chimères. Par ailleurs, la purification desdites particules à partir du broyat des cellules co-transfectées nécessite une mise en oeuvre relativement lourde, mal adaptée à une production industrielle.

A titre d'exemple non limitatif, on présente, en relation avec les figures 8 à 11, un mode de réalisation d'obtention de particules sous-virales d'enveloppe du virus de l'hépatite C à partir de lignées de type CHO exprimant de manière stable la protéine sauvage S du HBV ainsi que la protéine de fusion E1-Sd ou la protéine de fusion E2-Sd. L'obtention de ces clones stables de CHO est rendue possible par l'utilisation de vecteur intégratifs de type lentiviral. Un des atouts de ce système est que la production de protéines recombinantes, telles que les protéines de fusion de l'invention, n'a pas d'effets cytotoxiques majeurs à cours termes tels que ceux rencontrés avec le système SFV.

Un des objectifs de ce mode de réalisation est également d'obtenir un système cellulaire de production de particules sous-virales chimères de l'invention proche de celui utilisé pour la fabrication industrielle du vaccin contre l'hépatite B.

### Exemple II : Obtention de particules sous-virales d'enveltinpe du virus de l'hépatite C en système lentiviral

L'utilisation de vecteurs lentiviraux a permis de développer des clones cellulaires produisant de façon stable la protéine S sauvage du HBV associée à une des protéines de fusion E1-Sd et/ou E2-Sd.

### II-11) Vecteurs plasmidiques lentiviraux pLENTI.

Le plasmide pLENTI*^{hph}* de 9955 pb, comportant le gène de sélection *hph,* et codant pour une protéine de résistance à l'hygromycine comme marqueur de sélection a été utilisé pour les constructions suivantes [Naldini, L., Blomer, U., Gallay, P., Ory, D., Mulligan, R., Gage, F. H., Verma, I. M., and Trono, D. (1996). In vivo gene delivery and stable transduction of nondividing cells by a lentiviral vector. Science 272(5259), 263-7].

Dans un premier temps, la séquence d'acide nucléique codant pour la protéine S sauvage est libérée de pSFV1-S décrit antérieurement [Patient, R., Hourioux, C., Sizaret, P.Y., Trassard, S., Sureau, C., and Roingeard, P., (2007) Hepatitis B Virus Subviral Envelope Particle Morphogenesis and Intracellular Trafficking J Virol, 81(8), 3842-51] par restriction avec l'enzyme *Bam*HI (Biolabs) puis purifié sur gel d'agarose 1% à l'aide du système « Wizard SV Gel and PCR Clean-Up System » (Promega) selon les recommandations du fabricant. Ce fragment purifié a ensuite été cloné au niveau du site *Bam*HI compris dans le site de clonage multiple du plasmide pLENTI*^{hph}* à l'aide d'une T4-ADN-ligase (Biolabs) selon les recommandations du fabricant. Le plasmide pLENTI*^{hph}* -S a enfin été amplifié par transformation bactérienne puis purifié par maxipréparation d'ADN à l'aide du système « Nucleobond PC 500 Kit » (Qiagen) selon les recommandations du fabricant. L'orientation de l'insert a été vérifiée par restriction enzymatique.

De la même façon, les différentes molécules d'acides nucléiques hybrides de l'invention d'ADNc codant pour les protéines de fusion de l'invention (notamment E1-Sd et E2-Sd) sont clonées au niveau du site *Bam*HI du plasmide pLENTI*^{gfp}*. Les plasmides ainsi obtenus (notamment pLENTI*^{gfp}*- E1-Sd et E2-Sd) sont ensuite amplifiés, purifiés et séquencés (cf. II-1).

### II-2 ) Production de lentivirus recombinants.

Vingt-quatre heures avant la transfection des plasmides lentiviraux, des cellules HEK 293T ont été ensemencées à raison de 3.10⁶ cellules par flasque de 75 cm2 (Falcon) en milieux DMEM-glutamax (Invitrogen) supplémenté par 10 % de sérum de voeux fétal décomplémenté (ATGC), 100 UI/mL de pénicilline et 100 µg/mL de streptomycine. Ces cellules ont été cultivées sous 5 % de CO2 et le milieu de culture a été changé 4 heures avant la transfection. Une pmole de chaque plasmide p8.74, pVSV-G et pLENTI (pLENTI*^{hph}*-S, pLENTI*^{gfp}*- E1-Sd, E2-Sd) a été transfectée simultanément dans les cellules HEK 293T à l'aide du système « Calcium Phosphate Transfection Kit » (Invitrogen) selon les recommandations du fabricant. Le milieu de culture est changé 24 heures après la transfection et collecté 48 heures et 72 heures après la transfection. Les milieux collectés sont filtrés à 0,45 µm puis concentrés par ultracentrifugation sur coussin de sucrose 20 % à 4°C pendant 90 minutes à 100.000 g. Le culot contenant les lentivirus recombinants est repris dans 500 µL de tampon phosphate (PBS) et conservé à -80 °C. Des lots de vecteurs lentivirauxs recombinants de l'invention sont générés dont notamment les vecteurs lentivirauxs LVh*^{php}*-S, LV*^{gfp}*-E1-Sd, LV*^{gfp}*-E2-Sd. La titration des unités lentivirales de transduction (TU) de chaque lot est déterminée à partir du dosage de la protéine p24 et à l'aide du système « Innotest HIV Kit » (Innogenetics) selon les recommandations du fabricant.

### II-3 ) Culture cellulaire et transduction.

La lignée utilisée pour la production stable et constitutive des particules sous-virales chimériques HCV-HBV est une lignée d'ovaire de hamster chinois nommée CHO. Cette lignée a déjà été parfaitement validée pour la production de protéines recombinantes d'intérêt médical, et notamment pour le vaccin contre l'hépatite B « GenHevac B Pasteur® » (Sanofi Pasteur MSD). Ces cellules CHO sont cultivées sous 5 % de CO2 en milieux DMEM-F12 (Invitrogen) supplémenté par 10 % de sérum de voeux fétal décomplémenté (ATGC), 100 UI/mL de pénicilline et 100 µg/mL de streptomycine. Vingt-quatre heures avant la transduction, les cellules CHO sont ensemencées dans une plaque 6-puits (Falcon) à raison de 105 cellules par puit. Elles sont ensuite transduites dans du milieu neuf avec une multiplicité d'infection de 2,5 (soit un ratio d'unités de transduction (TU) par cellule de 2,5) en présence de 4 µg/mL de polybrène (Sigma-Aldrich) puis cultivées pendant 24 heures. Le jour suivant, le milieu de transduction est retiré, les cellules obtenues selon la mise en oeuvre de la présente invention sont rincées en tampon PBS puis sont maintenues normalement en culture pendant encore 2 jours.

### II-3.1) Génération d'un clone CHO stable producteur de la protéine S sauvage.

La lignée CHO utilisée est transduite avec le lentivirus LV*^{hph}*-S selon le protocole décrit ci-dessus, et schématisé à la Figure 8. L'efficacité de transduction est vérifiée par immunofluorescence à l'aide d'un anticorps dirigé contre la protéine S sauvage selon un protocole décrit antérieurement [Patient, R., Hourioux, C., Sizaret, P.Y., Trassard, S., Sureau, C., and Roingeard, P., (2007) Hepatitis B Virus Subviral Envelope Particle Morphogenesis and Intracellular Trafficking J Virol, 81(8), 3842-51]. Trois jours après la transduction, les cellules sont trypsinées, ré-ensemencées dans une boîte de culture de 10 cm de diamètre (Falcon) en présence de 1 mg/mL d'hygromycine (Euromedex) et maintenue en culture pendant trois semaines. Les clones cellulaires émergeants sont récupérés par cylindre de clonage (Invitrogen) puis amplifiés en plaque 24-puits (Falcon). Le clone CHO utilisé par la suite est sélectionné après quantification dans le surnageant de culture de la protéine S sauvage par ELISA [Patient, R., Hourioux, C., Sizaret, P.Y., Trassard, S., Sureau, C., and Roingeard, P., (2007) Hepatitis B Virus Subviral Envelope Particle Morphogenesis and Intracellular Trafficking J Virol, 81(8), 3842-51] Ce clone cellulaire a été nommé CHO-S.

### II-3.2) Génération de clones CHO stables producteurs de la protéine S sauvage du virus HBV et des protéines de fusion de l'invention E1-Sd et E2-Sd.

Le clone cellulaire CHO-S est par la suite sur-transduit avec le lentivirus recombinant LV^{gfp} codant pour la protéine GFP comme marqueur de sélection, et comprenant une molécule d'acides nucléiques hybride de l'invention, notamment le vecteur lentivirus LV^{gfp}-PIT1-E1-Sd ou LV^{gfp}-PIT2-E2-Sd selon le protocole décrit ci-dessus au §II-3.1. (Figure 8). De part la présence de la protéine GFP, l'efficacité de transduction est vérifiée à l'aide d'un cytomètre en flux FACScalibur (Becton-Dickinson). Trois jours après la transduction, les cellules sont trypsinées, ré-ensemencées en dilution limite dans une plaque 96-puits (Falcon) à raison de 1 cellule par puit et elles sont maintenues en culture pendant trois semaines. Les clones cellulaires émergeants GFP+ sont récupérés puis amplifiés en grande quantité. Les clones cellulaires ainsi obtenus sont notamment nommés CHO-E1-Sd ou CHO-E2-Sd.

### II-4 ) Analyse de la production intracellulaire des protéines d'enveloppe sauvage et chimériques du HBV et du HCV.

Les procédures d'analyses biochimiques par immunofluorescence et par Western Blot des protéines d'intérêt S, E1-Sd ou E2-Sd de chaque clone cellulaire ont déjà. été décrites précédemment pour l'exemple I au § I-4.

### II-5 ) Puritication et analyse des particules sous-virales chimères issues du surnageant de culture.

Environ 200 mL de surnageant de culture pour chaque clone sélectionné est clarifié par centrifugation à 4 °C pendant 10 minutes à 1500 g. Les protéines sont précipitées par ajout d'une solution de (NH4)2SO4 (pH 7,5 ; 45 % final) puis centrifugation à 4 °C pendant 15 minutes à 10.000 g. Le culot est repris dans un volume minimum de tampon TNE (10 mM Tris/HCl pH 7,5 / 100 mM NaCl / 1 mM EDTA). Après une série de dialyses en tampon TNE, du CsCl est ajouté jusqu'à obtenir une densité d'environ 1,22 g/cm3 puis deux ultracentrifugations isopycniques successives est réalisées à 15 °C pendant 48 heures à 40.000 rpm à l'aide d'un rotor 45Ti (L70 Ultracentrifuge, Beckman). Des fractions sont collectées à partir du sommet du gradient et la protéine S sauvage, est quantifiée par ELISA [Patient, R. Hourioux, C., Sizaret, P.Y., Trassard, S., Sureau, C., and Roingeard, P., (2007) Hepatitis B Virus Subviral Envelope Particle Morphogenesis and Intracellular Trafficking J Virol, 81(8), 3842-51]. Les fractions positives ont été mélangées et dialysées à 4 °C en tampon TNE.

Les préparations purifiées ont enfin été analysées par Western Blot et par microscopie électronique à transmission comme décrit précédemment l'exemple I au § I-4.

### II-6 1 Production intracellulaire de la protéine S et des protéines de fusion.

Suite à leur expansion, les clones cellulaires CHO-S, CHO-E1-Sd et CHO-E2-Sd ont été analysés par immunofluorescence puis Western-blot à l'aide d'anticorps dirigés contre les protéines Sd, E1 ou E2 (cf : exemple I au § I-4 et figure 9). Comme attendu, l'ensemble des clones permet une production intense de la protéine S sauvage du HBV sous ses deux états de glycosylation (p24 et gp27). De même, les clones CHO-E1-Sd et CHO-E2-Sd permettent une production intense et cytoplasmique des protéines de fusion de l'invention de tailles attendues. Ces résultats montrent donc que toutes les protéines de fusion de l'invention sont correctement produites dans la lignée CHO.

### II-7 ) Purification des particules

Un premier essai de purification est réalisé à partir de 200 mL de surnageant de culture du clone CHO-S, CHO-E1-Sd ou CHO-E2-Sd. Ces surnageants, dont la concentration en protéine S sauvage est évaluée par ELISA à environ 100 ng/mL pour les trois clones, sont ultracentrifugés sur un gradient isopycnique de CsCl. Les fractions de chaque gradient formé sont ensuite analysées par une quantification de la protéine S sauvage (figure 10). Pour les trois types de particules sous-virales chimères purifiées (S, E1-Sd et E2-Sd), le pic de concentration de protéine S sauvage a pu être observé dans la fraction n° 9, correspondant à une densité comprise entre 1,17 et 1,18. Les fractions n° 8, 9 et 10 de chaque préparation sont alors rassemblées pour être dialysées puis analysées par Western-blot à l'aide d'anticorps dirigés contre les protéines S sauvage, E1 ou E2 (cf : exemple I au § I-4 et (figure 11A) et par microscopie électronique à transmission en coloration négative (figures 11B à 11D).

Un dépôt correspondant à un µg de protéine S sauvage pour chaque purification est réalisé pour l'analyse en gel d'acrylamide. Ceci permet de montrer que les protéines de fusion de l'invention E1-Sd et E2-Sd sont détectées à la taille attendue et en quantité respectable dans leur préparation respective par les anticorps anti-E1 ou anti-E2. De même, les deux formes de glycosylation attendues de la protéine S sauvage sont détectées dans chaque préparation. L'analyse par coloration négative en microscopie électronique à transmission a permis de confirmer la présence de particules sous-virales d'enveloppe sphériques d'environ 20 nm de diamètre dans les trois préparations étudiées.

### Exemple III : Obtention de particules sous-virales d'enveloppe comprenant les deux protéines de fusion E1-Sd et E2-Sd.

Un nouveau plasmide pLENTI^{g*luc*} comportant le gène de sélection *gluc* de *Gaussia princeps*, au lieu et place du gène *gfp*, est réalisé et code pour une luciférase (GLuc) sécrétée comme marqueur de sélection. Le fragment d'ADNc codant pour la protéine de fusion E1-Sd est cloné au niveau du site *Bam*HI du plasmide pLENTI^{g*luc*}. Le plasmide pLENTI^{g*luc*}- E1-Sd ainsi obtenu est amplifié, purifié et séquencé (cf.II-1).

### III.1. Production de lentivirus recombinants.

Vingt-quatre heures avant la transfection des plasmides lentiviraux, des cellules HEK 293T ont été ensemencées à raison de 3.10⁶ cellules par flasque de 75 cm2 (Falcon) en milieux DMEM-glutamax (Invitrogen) supplémenté par 10 % de sérum de voeux fétal décomplémenté, (ATGC), 100 UI/mL de pénicilline et 100 µg/mL de streptomycine. Ces cellules sont cultivées sous 5 % de CO2 et le milieu de culture est changé 4 heures avant la transfection. Une pmole de chaque plasmide p8.74, pVSV-G et pLENTI^{g*luc*}- E1-Sd est transfectée simultanément dans les cellules HEK 293T à l'aide du système « Calcium Phosphate Transfection Kit » (Invitrogen) selon les recommandations du fabricant. Le milieu de culture est changé 24 heures après la transfection et collecté 48 heures et 72 heures après la transfection. Les milieux collectées, sont filtrés à 0,45 µm puis concentrés par ultracentrifugation sur coussin de sucrose 20 % à 4°C pendant 90 minutes à 100.000 g. Le culot contenant les lentivirus recombinants est repris dans 500 µL de tampon phosphate (PBS) et conservé à-80 °C. Un nouveau lot de lentivirus recombinants est ainsi généré : LV^{g*luc*}- E1-Sd. La titration des unités lentivirales de transduction (TU) de ce lot est déterminée à partir du dosage de la protéine p24 et à l'aide du système « Innotest HIV Kit » (Innogenetics) selon les recommandations du fabricant.

### III.2. Génération d'un clone CHO stable producteur de la protéine S sauvage du virus HBV, des protéine de fusion de l'invention E2-Sd et E1-Sd.

La culture des cellules CHO et la méthode de transduction exposée et utilisée pour l'exemple II est adapté à cet exemple. Après une première transduction avec le lentivirus LV*^{hph}*-S, permettant l'obtention du clone cellulaire CHO-S, une seconde transduction est effectuée avec le lentivirus LV^{gfp}- E2-Sd selon le protocole décrit pour l'exemple II, permettant l'obtention du clone cellulaire CHO-E2-Sd.

Ce dernier est à nouveau transduit avec le lentivirus LV^{g*luc*}-E1-Sd selon le protocole décrit ci-dessus. Trois jours après la transduction, les cellules sont trypsinées et ré-ensemencées en dilution limite dans une plaque 96-puits (Falcon) à raison de 1 cellule par puit. Les cellules sont maintenues en culture pendant trois semaines au bout desquelles le surnageant des clones cellulaires émergeants est récolté. Dans ces surnageants, la présence de la luciférase est détectée par la mesure de son activité enzymatique à l'aide du système *« Gaussia* Luciferase Assay Kit » (Biolabs) selon les recommandations du fabricant. La mesure de la lumière émise par la réaction enzymatique est réalisée à l'aide d'un luminomètre Centro LB 960 (Berthold Technologies). Les clones cellulaires correspondant aux surnageants GLuc+ sont récupérés puis amplifiés en grande quantité. Le clone ainsi obtenu est nommé CHO-S/E2-Sd/E1-Sd.

### Exemple IV : Analyse in vivo de l'activité immunologique des particules sous-virales chimériques

5 à 10 milligrammes de particules sous-virales chimériques pour chaque type de particule (S+E1-Sd, S+E2-Sd, S seule du HBV) sont purifiées à partir du surnageant de culture cellulaire selon la méthode décrite ci-dessus.

Quatre groupes de souris et de lapins sont mis en oeuvre pour une série d'immunisation.

L'immunisation est effectuée par trois injections de 10 microgrammes de l'immunogène selon la méthode classique.

Le premier groupe est immunisé par les particules S+E1-Sd.

Le deuxième groupe est immunisé par les particules S+E2-Sd.

Le troisième groupe est immunisé par les particules HBV S seule.

Le quatrième groupe est immunisé par le mélange de particules sous-virales chimériques S+E1-Sd et de particules sous-virales chimériques S+E2-Sd:

La réponse humorale globale produite chez ces animaux est détectée par l'analyse ELISA et Western blots. Pour les anticorps anti-S, des ELISA commerciaux (Abbott, Roche) permettent de déterminer le nombre d'unités internationales (UI) d'anticorps anti-S, qui sont connus pour avoir des propriétés neutralisantes contre le VHB. Une concentration au moins égales à 10 UI est considérée comme protectrice contre le VHB (Jilg W, et al. Hepatitis B-vaccination: Strategy for booster doses in high risk population groups. Progress in Hepatitis B Immunization. Eds. P. Coursaget et al. Colloque Inserm. 1990; 190: 419-427.) Pour les anticorps anti-E1 et ant-E2, le Western blot est utilisé pour évaluer si une réponse anticorps anti-E1 et anti-E2 est générée. Si c'est le cas, cette analyse est complétée d'une analyse de la présence d'anticorps anti-E1 et anti-E2 neutralisant le virus. L'analyse de la neutralisation de HCV par ces anticorps est mise en oeuvre dans le système JFH-1 (Wakita et al, Nat Med 2005) qui permet de propager une souche de HCV de génotype 2 in vitro, ou par l'utilisation des souches virales chimériques comprenant les protéines structurales du HCV de génotype 1 ou 3.

## Revendications

1. Protéine de fusion immunogène comprenant au moins les deux peptides suivants:
a)- du côté C-terminal, un premier peptide constitué :
- de la séquence d'acides aminés de la protéine S d'un isolat du virus humain de l'hépatite B (HBV), laquelle protéine S est délétée de son domaine transmembranaire situé à son extrémité N-terminale, ladite séquence d'acides aminés conservant la capacité à former des particules sous-virales non-infectieuses immunogènes vis-à-vis du virus humain de l'hépatite B, ou
- d'une séquence d'acides aminés présentant un pourcentage d'identité d'au moins 91%, notamment d'au moins 93%, particulièrement d'au moins 95%, et plus particulièrement d'au moins 97%, avec ladite séquence d'acides aminés de la protéine S délétée en N-terminal, sous réserve que ladite séquence d'acides aminés soit aussi délétée du domaine transmembranaire situé à l'extrémité N-terminale de la protéine S de HBV, et qu'elle conserve la capacité à former des particules sous-virales non infectieuses immunogènes vis-à-vis du virus humain de l'hépatite B, ou,
- de la séquence d'acides aminés d'un variant synthétique dérivé de ladite séquence d'acides aminés de ladite protéine S délétée, sous réserve que ladite séquence d'acides aminés dudit variant synthétique soit aussi délétée du domaine transmembranaire situé à l'extrémité N-terminale de la protéine S de HBV, et qu'elle conserve la capacité à former des particules sous-virales non infectieuses immunogènes vis-à-vis du virus humain de l'hépatite B, et,
b)- du côté N-terminal, un second peptide constitué :
- de la séquence d'acides aminés du domaine transmembranaire et de l'ectodomaine d'au moins une protéine d'enveloppe d'un isolat du virus de l'hépatite C (HCV), ou
- d'une séquence d'acides aminés présentant un pourcentage d'identité d'au moins 78%, notamment d'au moins 80%, particulièrement d'au moins 85%, et plus particulièrement d'au moins 90%, avec ladite séquence d'acides aminés du domaine transmembranaire et de l'ectodomaine d'une protéine d'enveloppe d'un isolat du virus de l'hépatite C, sous réserve que ladite séquence d'acides aminés conserve les propriétés dudit domaine transmembranaire et les propriétés immunogènes vis-à-vis du virus de l'hépatite C , ou
- de la séquence d'acides aminés d'un variant synthétique dérivé de ladite séquence d'acides aminés du domaine transmembranaire et de l'ectodomaine de la protéine E1 ou E2 de HCV, sous réserve que ladite séquence d'acides aminés conserve les propriétés dudit domaine transmembranaire et les propriétés immunogènes vis-à-vis du virus de l'hépatite C, ladite protéine d'enveloppe d'un isolat du virus de l'hépatite C étant choisie parmi la protéine E1, la protéine E2 ou un peptide de fusion comprenant la protéine Elet la protéine E2.

2. Protéine de fusion immunogène selon la revendication 1, dans laquelle le second peptide situé du côté N-terminal, est constitué :
- de la totalité de la séquence d'acides aminés du domaine transmembranaire et de l'ectodomaine de la protéine d'enveloppe E1, ou
- d'une séquence d'acides aminés présentant un pourcentage d'identité d'au moins 75%, notamment d'au moins 80%, particulièrement d'au moins 85%, et plus particulièrement d'au moins 90%, avec ladite séquence d'acides aminés du domaine transmembranaire et de l'ectodomaine de la protéine d'enveloppe E1, sous réserve que ladite séquence d'acides aminés conserve les propriétés dudit domaine transmembranaire et les propriétés immunogènes vis-à-vis du virus de l'hépatite C, ou
- de la séquence d'acides aminés d'un variant synthétique dérivé de ladite protéine E1; sous réserve que ladite séquence d'acides aminés conserve les propriétés dudit domaine transmembranaire et les propriétés immunogènes vis-à-vis du virus de l'hépatite C.

3. Protéine de fusion immunogène selon la revendication 1, dans laquelle le second peptide situé du côté N-terminal, est conslitué :
- de la totalité de la séquence d'acides aminés du domaine transmembranaire et de l'ectodomaine de la protéine d'enveloppe E2, ou
- d'une séquence d'acides aminés présentant un pourcentage d'identité d'au moins 78%, notamment d'au moins 80%, particulièrement d'au moins 85%, et plus particulièrement d'au moins 90%, avec ladite séquence d'acides aminés du domaine transmembranaire et de l'ectodomaine de la protéine d'enveloppe E2, sous réserve que ladite séquence d'acides aminés conserve les propriétés dudit domaine transmembranaire et les propriétés immunogènes vis-à-vis du virus de l'hépatite C, ou
- de la séquence d'acides aminés d'un variant synthétique dérivé de ladite protéine E2, sous réserve que ladite séquence d'acides aminés conserve les propriétés dudit domaine transmembranaire et les propriétés immunogènes vis-à-vis du virus de l'hépatite C.

4. Protéine de fusion immunogène selon l'une quelconque des revendications 1 à 3, dans laquelle le premier et le second peptide constituant la protéine de fusion immunogène sont contigus, et l'extrémité C-terminale du second peptide est liée de façon covalente à l'extrémité N-terminale du premier peptide.

5. Protéine de fusion immunogène selon l'une quelconque des revendications 1. à 4, dans laquelle le premier peptide en position C-terminale est constitué :
- d'une séquence d'acides aminés délimitée par les acides aminés contigus situés dans la région comprise de l'acide aminé en position 23 à celui en position 226 de la protéine S, de HBV, et particulièrement de l'isolat HBVadw,
et notamment de la séquence d'acides aminés représentée par la SEQ ID N°2, ladite séquence d'acides aminés conservant la capacité à former des particules sous-virales non-infectieuses immunogènes vis-à-vis du virus humain de l'hépatite B, ou
- d'une séquence d'acides aminés présentant un pourcentage d'identité d'au moins 91%, notamment d'au moins 93%, particulièrement d'au moins 95%, et plus particulièrement d'au moins 97%, avec ladite séquence d'acides aminés contigus de la protéine S, sous réserve que ladite séquence d'acides aminés soit aussi délétée du domaine transmembranaire situé à l'extrémité N-terminale de la protéine S de HBV, et qu'elle conserve la capacité à former des particules sous-virales non infectieuses, immunogènes vis-à-vis du virus humain de l'hépatite B, ou
- de la séquence d'acides aminés d'un variant synthétique, dérivé de ladite séquence d'acides aminés de ladite protéine S; sous réserve que ladite séquence d'acides aminés dudit variant synthétique soit aussi délétée du domaine transmembranaire situé à l'extrémité N-terminale de la protéine S de HBV, et qu'elle conserve la capacité à former des particules sous-virales non infectieuses, immunogènes vis-à-vis du virus humain de l'hépatite B.

6. Protéine de fusion immunogène selon l'une quelconque des revendications 1 à 5, dans laquelle le second peptide en position N-terminale est constitué :
- d'une séquence d'acides aminés du domaine transmembranaire et de l'ectodomaine d'une protéine d'enveloppe d'un isolat du virus de l'hépatite C (HCV) et délimitée par les acides aminés contigus situés dans la région comprise de l'acide aminé en position 192 à celui en position 380 de la protéine E1 de HCV, et particulièrement de l'isolat HCV-1a,
et notamment de la séquence d'acides aminés représentée par la SEQ ID N°4, ou
- d'une séquence d'acides aminés présentant un pourcentage d'identité d'au moins 75%, notamment d'au moins 80%, particulièrement d'au moins 85%, et plus particulièrement d'au moins 90%, avec ladite séquence d'acides aminés contigus de ladite protéine E1, sous réserve que ladite séquence d'acides aminés conserve les propriétés dudit domaine transmembranaire et les propriétés immunogènes vis-à-vis du virus de l'hépatite C, ou
- de la séquence d'acides aminés d'un variant synthétique dérivé de ladite séquence d'acides aminés contigus du domaine transmembranaire et de l'ectodomaine de ladite protéine E1, sous réserve que ladite séquence d'acides aminés conserve les propriétés dudit domaine transmembranaire et les propriétés immunogènes vis-à-vis du virus de l'hépatite C.

7. Protéine de fusion immunogène selon l'une des revendications 5 ou 6, dans laquelle le premier et le second peptide sont contigus, ladite protéine de fusion étant constituée par :
- la séquence d'acides aminés représentée par la SEQ ID N°8, ou
- une séquence d'acides aminés présentant un pourcentage d'identité d'au moins 88%, notamment d'au moins 90%, particulièrement d'au moins 92%, et plus particulièrement d'au moins 95%, avec SEQ ID N°8, sous réserve que ladite séquence d'acides aminés soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis de HCV et vis-à-vis de HBV humain, ou
- de la séquence d'acides aminés d'un variant synthétique dérivé de ladite SEQ ID N°8, sous réserve que ladite séquence d'acides aminés dudit variant synthétique soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis de HCV et vis-à-vis de HBV humain.

8. Protéine de fusion immunogène selon l'une quelconque des revendications 1 à 5, dans laquelle le second peptide en position N-terminale est constitué :
- d'une séquence d'acides aminés du domaine transmembranaire et de l'ectodomaine d'une protéine d'enveloppe d'un isolat du virus de l'hépatite C (HCV) et délimitée par les acides aminés contigus situés de l'acide aminé en position 384 à celui en position 743 de la protéine E2 de HCV, et particulièrement de l'isolat HCV-1a,
et notamment de la séquence d'acides aminés représentée par la SEQ ID N°6, ou
- d'une séquence d'acides aminés présentant un pourcentage d'identité d'au moins 78%, notamment d'au moins 80%, particulièrement d'au moins 85%, et plus particulièrement d'au moins 90%, avec ladite séquence d'acides aminés contigus de protéine E2, sous réserve que ladite la séquence d'acides aminés conserve les propriétés dudit domaine transmembranaire et les propriétés immunogènes vis-à-vis du virus de l'hépatite C, ou
- de la séquence d'acides aminés d'un variant synthétique dérivé de ladite séquence d'acides aminés contigus du domaine transmembranaire et de l'ectodomaine de ladite protéine E2, sous réserve que ladite séquence d'acides aminés conserve les propriétés dudit domaine transmembranaire et les propriétés immunogènes vis-à-vis du virus de l'hépatite C.

9. Protéine de fusion immunogène selon l'une des revendications 5 ou 8, dans laquelle le premier et le second peptide sont contigus, ladite protéine de fusion étant constituée par :
- la séquence d'acides aminés représentée par la SEQ ID N°10, ou
- une séquence d'acides aminés présentant un pourcentage d'identité d'au moins 86%, notamment d'au moins 88%, particulièrement d'au moins 92%, et plus particulièrement d'au moins 95%, avec SEQ ID N°10, sous réserve que ladite séquence d'acides aminés soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus de l'hépatite B humain et vis-à-vis du virus de l'hépatite C, ou
- de la séquence d'acides aminés d'un variant synthétique dérivé ladite SEQ ID N °10, sous réserve que ladite séquence d'acides aminés dudit variant synthétique soit aussi délétée du domaine transmembranaire situé à l'extrémité N-terminale de la protéine S de HBV, conserve les propriétés du domaine transmembranaire de la protéine d'enveloppe de HCV et qu'elle conserve la capacité à former des particules sous-virales, non infectieuses et immunogènes vis-à-vis du virus de l'hépatite B humain et vis-à-vis du virus de l'hépatite C.

10. Molécule d'acides nucléiques hybride codant pour l'une quelconque des protéines de fusion selon l'une quelconque des revendications 1 à 9.

11. Molécule d'acides nucléiques hybride selon la revendication 10, dans laquelle une première séquence d'acides nucléiques code pour ledit premier peptide, une seconde séquence d'acides nucléiques code pour ledit second peptide, la première et la seconde séquence d'acides nucléiques sont contiguës, et l'extrémité 5' de la première séquence d'acides nucléiques est liée de façon covalente à l'extrémité 3' de la seconde séquence d'acides nucléiques.

12. Molécule d'acides nucléiques hybride selon l'une quelconque des revendications 10 et 11, codant pour une protéine de fusion susmentionnée comprenant un peptide d'initiation de transfert situé du côté N-terminal, ladite molécule d'acides nucléiques hybride étant représentée par :
- la SEQ ID N°11, ou
- une séquence d'acides nucléiques présentant une identité d'au moins 78%, notamment d'au moins 80%, particulièrement d'au moins 85%, et plus particulièrement d'au moins 90%, avec ladite SEQ ID N°11.

13. Molécule d'acides nucléiques hybride selon l'une quelconque des revendications 10 et 11, codant pour une protéine de fusion susmentionnée comprenant un peptide d'initiation de transfert situé du côté N-terminal, ladite molécule d'acides nucléiques hybride étant représentée par :
- la SEQ ID N°13, ou
- une séquence d'acides nucléiques présentant une identité d'au moins 80%, notamment d'au moins 85%, particulièrement d'au moins 90%, et plus particulièrement d'au moins 95%, avec ladite SEQ ID N°13.

14. Vecteur comprenant une molécule d'acides nucléiques hybride selon l'une quelconque des revendications 10 à 13, ainsi que les moyens nécessaires pour leur expression.

15. Vecteur selon la revendication 14, comprenant en outre, un vecteur lentiviral.

16. Vecteur selon l'une des revendications 14 à 15, dans lequel la molécule d'acides nucléiques hybride est une molécule d'acides nucléiques selon la revendication 12 ou 13.

17. Particule d'enveloppe sous-virale, chimère, immunogène et non infectieuse, comprenant les protéines suivantes :
- la protéine constituée de la protéine S sauvage de l'antigène de surface d'un isolat du virus de l'hépatite B, et,
- au moins une protéine de fusion immunogène selon l'une quelconque des revendications 1 à 9.

18. Particule sous-virale chimère immunogène selon la revendication 17, dans laquelle la protéine de fusion immunogène, est constituée par :
- la séquence d'acides aminés représentée par la SFQ ID N°8, ou
- une séquence d'acides aminés présentant un pourcentage d'identité d'au moins 88%, notamment d'au moins 90%, particulièrement d'au moins 92%, et plus particulièrement d'au moins 95%, avec la dite SEQ ID N°8.

19. Particule sous-virale chimère immunogène selon la revendication 17, dans laquelle la protéine de fusion immunogène est constituée par :
- la séquence d'acides aminés représentée par la SEQ ID N°10, ou
- une séquence d'acides aminés présentant un pourcentage d'identité d'au moins 86%, notamment d'au moins 88%, particulièrement d'au moins 92%, et plus particulièrement d'au moins 95%, avec SEQ ID N°10.

20. Particule sous-virale chimère immunogène selon la revendication 17, comprenant les deux protéines de fusion suivantes :
- la séquence d'acides aminés représentée par la SEQ ID N°8, ou
- une séquence d'acides aminés présentant un pourcentage d'identité d'au moins 88%, notamment d'au moins 90%, particulièrement d'au moins 92%, et plus particulièrement d'au moins 95%, avec la dite SEQ ID N°8, et
- la séquence d'acides aminés représentée par la SEQ ID N°10, ou
- une séquence d'acides aminés présentant un pourcentage d'identité d'au moins 86%, notamment d'au moins 88%, particulièrement d'au moins 90%, et plus particulièrement d'au moins 95%, avec SEQ ID N°10.

21. Composition immunogène comprenant comme substance active au moins un élément choisi parmi :
- une protéine de fusion selon l'une quelconque des revendications 1 à 9,
- une molécule d'acides nucléiques hybride selon l'une quelconque des revendications 10 à 13,
- un vecteur selon l'une quelconque des revendications 14 à 16, comprenant ladite molécule d'acides nucléiques hybride,
- une particule sous-virale chimère selon l'une quelconque des revendications 17 à 20, et, un véhicule pharmaceutiquement acceptable.

22. Composition immunogène selon la revendication 21, dans laquelle la substance active est au moins l'un des trois composés suivants :
a)- une protéine de fusion selon la revendication 7,
b)- une molécule d'acides nucléiques hybride selon la revendication 12, ou un vecteur comprenant ladite molécule d'acides nucléiques hybride ;
c)- une particule sous-virale chimère selon la revendication 18,.

23. Composition immunogène selon la revendication 21, dans laquelle la substance active est au moins l'un des composés suivants:
a)- une protéine de fusion selon la revendication 9,
b)- une molécule d'acides nucléiques hybride selon la revendication 13, ou un vecteur comprenant ladite molécule d'acides nucléiques hybride ;
c)- une particule sous-virale chimère, selon la revendication 19.

24. Composition immunogène selon la revendication 21, dans laquelle la substance active est une particule sous-virale chimère, comprenant au moins les deux protéines de fusion immunogènes selon les revendications 7 et 9.

25. Composition immunogène selon la revendication 21, dans laquelle la substance active est constituée du mélange comprenant :
- au moins deux protéines de fusion suivantes :
* l'une selon la revendication 7;
* l'autre selon la revendication 9 ;
- au moins deux molécules d'acides nucléiques hybrides suivantes :
* l'une selon la revendication 12, ou un vecteur comprenant ladite molécule d'acides nucléiques hybride ;
* l'autre selon la revendication 13, ou un vecteur comprenant ladite molécule d'acides nucléiques hybride ;
- au moins deux particules sous-virales chimères suivantes :
* l'une selon la revendication 18,
* l'autre selon la revendication 19.

26. Utilisation d'une composition immunogène de l'une quelconque des revendications 21 à 25, pour la fabrication d'un médicament pour le traitement prophylactique et/ou pour la prévention de l'hépatite C.

27. Utilisation d'une composition immunogène de l'une quelconque des revendications 21 à 25, pour la fabrication d'un médicament pour le traitement prophylactique et/ou pour la prévention de l'hépatite B.

28. Utilisation d'une composition immunogène de l'une quelconque des revendications 21 à 25, pour la fabrication d'un médicament pour le traitement prophylactique et/ou pour la prévention de l'hépatite B et de l'hépatite C.

29. Une lignée cellulaire qui exprime des particules sous virales chimère, immunogènes non infectieuses selon la revendication 17-20, ladite lignée comprenant le vecteur selon les revendications 14-16.

30. Une lignée cellulaire selon la revendication 29, laquelle est la lignée d'ovaire de hamster chinois nommée CHO.

31. Une lignée cellulaire selon la revendication 29, laquelle est une levure, ladite levure pouvant être notamment *Saccharomyces Cerevrsae.*

## Patentansprüche

1. Immunogenes Fusionsprotein, das mindestens die beiden folgenden Peptide umfasst:
a)- auf der C-terminalen Seite ein erstes Peptid, bestehend aus:
- der Aminosäuresequenz des Proteins S eines Isolats des humanen Hepatitis-B-Virus (HBV), wobei das Protein S an seiner Transmembrandomäne, die an seinem N-terminalen Ende liegt, deletiert ist, wobei die Aminosäuresequenz die Fähigkeit behält, subvirale, nicht infektiöse Partikel, in Bezug auf das humane Hepatitis-B-Virus immunogene Partikel zu bilden, oder
- einer Aminosäuresequenz, die eine prozentuale Identität von mindestens 91 %, insbesondere 93 %, bevorzugt mindestens 95 % und stärker bevorzugt mindestens 97 % mit der Aminosäuresequenz des N-terminale deletierten Proteins S aufweist, unter der Bedingung, dass die Aminosäuresequenz auch an der Transmembrandomäne, die am N-terminalen Ende des Proteins S des HBV liegt, deletiert ist, und dass sie die Fähigkeit behält, subvirale, nicht infektiöse, in Bezug auf das humane Hepatitis-B-Virus immunogene Partikel zu bilden, oder,
- der Aminosäuresequenz einer synthetischen Variante, die von der Aminosäuresequenz des deletierten Proteins S abgeleitet ist, unter der Bedingung, dass die Aminosäuresequenz der synthetischen Variante auch an der Transmembrandomäne, die am N-terminalen Ende des Proteins S von HBV liegt, deletiert ist, und dass sie die Fähigkeit behält, subvirale, nicht infektiöse in Bezug auf das humane Hepatitis-B-Virus immunogene Partikel zu bilden, und
b)- auf der N-terminalen Seite ein zweites Peptid, bestehend aus:
- der Aminosäuresequenz der Transmembrandomäne und der Ektodomäne mindestens eines Hüllproteins eines Isolats des Hepatitis-C-Virus (HCV), oder
- einer Aminosäuresequenz, die eine prozentuale Identität von mindestens 78 %, insbesondere mindestens 80 %, bevorzugt mindestens 85 % und stärker bevorzugt mindestens 90 % mit der Aminosäuresequenz der Transmembrandomäne und der Ektodomäne eines Hüllproteins eines Isolats des Hepatitis-C-Virus aufweist, unter der Bedingung, dass die Aminosäuresequenz die Eigenschaften der Transmembrandomäne und die immunogenen Eigenschaften in Bezug auf das Hepatitis-C-Virus behält, oder
- der Aminosäuresequenz einer synthetischen Variante, die von der Aminosäuresequenz der Transmembrandomäne und der Ektodomäne des Proteins E1 oder E2 von HCV abgeleitet ist, unter der Bedingung, dass die Aminosäuresequenz die Eigenschaften der Transmembrandomäne und die immunogenen Eigenschaften in Bezug auf das Hepatitis-C-Virus behält, wobei das Hüllprotein eines Isolats des Hepatitis-C-Virus ausgewählt ist aus dem Protein E1, dem Protein E2 oder einem Fusionspeptid, das das Protein E1 und das Protein E2 umfasst.

2. Immunogenes Fusionsprotein nach Anspruch 1, wobei das zweite Peptid, das auf der N-terminalen Seite liegt, aus Folgendem besteht:
- der gesamten Aminosäuresequenz der Transmembrandomäne und der Ektodomäne des Hüllproteins E1, oder
- einer Aminosäuresequenz, die eine prozentuale Identität von mindestens 75 %, insbesondere mindestens 80 %, bevorzugt mindestens 85 % und stärker bevorzugt mindestens 90 % mit der Aminosäuresequenz der Transmembrandomäne und der Ektodomäne des Hüllproteins E1 aufweist, unter der Bedingung, dass die Aminosäuresequenz die Eigenschaften der Transmembrandomäne und die immunogenen Eigenschaften in Bezug auf das Hepatitis-C-Virus behält, oder
- die Aminosäuresequenz einer synthetischen Variante, die von dem Protein E1 abgeleitet ist; unter der Bedingung, dass die Aminosäuresequenz die Eigenschaften der Transmembrandomäne und die immunogenen Eigenschaften in Bezug auf das Hepatitis-C-Virus behält.

3. Immunogenes Fusionsprotein nach Anspruch 1, wobei das zweite Peptid, das auf der N-terminalen Seite liegt, aus Folgendem besteht:
- der gesamten Aminosäuresequenz der Transmembrandomäne und der Ektodomäne des Hüllproteins E2, oder
- einer Aminosäuresequenz, die eine prozentuale Identität von mindestens 78 %, insbesondere mindestens 80 %, bevorzugt mindestens 85 % und stärker bevorzugt mindestens 90 % mit der Aminosäuresequenz der Transmembrandomäne und der Ektodomäne des Hüllproteins E2 aufweist, unter der Bedingung, dass die Aminosäuresequenz die Eigenschaften der Transmembrandomäne und die immunogenen Eigenschaften in Bezug auf das Hepatitis-C-Virus behält, oder
- die Aminosäuresequenz einer synthetischen Variante, die von dem Protein E2 abgeleitet ist; unter der Bedingung, dass die Aminosäuresequenz die Eigenschaften der Transmembrandomäne und die immunogenen Eigenschaften in Bezug auf das Hepatitis-C-Virus behält.

4. Immunogenes Fusionsprotein nach einem der Ansprüche 1 bis 3, wobei das erste und das zweite Peptid, aus denen das immunogene Fusionsprotein besteht, benachbart sind, und das C-terminale Ende des zweiten Peptids kovalent an das N-terminale Ende des ersten Peptids gebunden ist.

5. Immunogenes Fusionsprotein nach einem der Ansprüche 1 bis 4, wobei das erste Peptid an C-terminaler Position aus Folgendem besteht:
- einer Aminosäuresequenz, die von den benachbarten Aminosäuren begrenzt wird, die in der Region zwischen der Aminosäure an Position 23 bis zu der an Position 226 des Proteins S von HBV und insbesondere des Isolats HBVadw liegen,
und insbesondere der Aminosäuresequenz, die von der SEQ ID Nr. 2 dargestellt wird, wobei die Aminosäuresequenz die Fähigkeit behält, subvirale, nicht infektiöse, in Bezug auf das humane Hepatitis-B-Virus immunogene Partikel zu bilden, oder
- einer Aminosäuresequenz, die eine prozentuale Identität von mindestens 91 %, insbesondere 93 %, bevorzugt mindestens 95 % und stärker bevorzugt mindestens 97 % mit der Sequenz der benachbarten Aminosäuren des Proteins S aufweist, unter der Bedingung, dass die Aminosäuresequenz auch an der Transmembrandomäne, die am N-terminalen Ende des Proteins S des HBV liegt, deletiert ist, und dass sie die Fähigkeit behält, subvirale, nicht infektiöse, in Bezug auf das humane Hepatitis-B-Virus immunogene Partikel zu bilden, oder,
- der Aminosäuresequenz einer synthetischen Variante, die von der Aminosäuresequenz des Proteins S abgeleitet ist, unter der Bedingung, dass die Aminosäuresequenz der synthetischen Variante auch an der Transmembrandomäne, die am N-terminalen Ende des Proteins S von HBV liegt, deletiert ist, und dass sie die Fähigkeit behält, subvirale, nicht infektiöse in Bezug auf das humane Hepatitis-B-Virus immunogene Partikel zu bilden.

6. Immunogenes Fusionsprotein nach einem der Ansprüche 1 bis 5, wobei das zweite Peptid an N-terminaler Position aus Folgendem besteht:
- einer Aminosäuresequenz der Transmembrandomäne und der Ektodomäne eines Hüllproteins eines Isolats des Hepatitis-C-Virus (HCV) und begrenzt von den benachbarten Aminosäuren, die in der Region zwischen der Aminosäure an Position 192 bis zu der an Position 380 des Proteins E1 von HCV und insbesondere des Isolats HCV-1a, liegen, und insbesondere der Aminosäuresequenz, die von der SEQ ID Nr. 4 dargestellt wird, oder
- einer Aminosäuresequenz, die eine prozentuale Identität von mindestens 75 %, insbesondere mindestens 80 %, bevorzugt mindestens 85 % und stärker bevorzugt mindestens 90 % mit der Sequenz der benachbarten Aminosäuren des Proteins E1 aufweist, unter der Bedingung, dass die Aminosäuresequenz die Eigenschaften der Transmembrandomäne und die immunogenen Eigenschaften in Bezug auf das Hepatitis-C-Virus behält, oder
- die Aminosäuresequenz einer synthetischen Variante, die von der Sequenz der benachbarten Aminosäuren der Transmembrandomäne und der Ektodomäne des Proteins E1 abgeleitet ist; unter der Bedingung, dass die Aminosäuresequenz die Eigenschaften der Transmembrandomäne und die immunogenen Eigenschaften in Bezug auf das Hepatitis-C-Virus behält.

7. Immunogenes Fusionsprotein nach einem der Ansprüche 5 oder 6, wobei das erste und das zweite Peptid benachbart sind, wobei das Fusionsprotein aus Folgendem besteht:
- der Aminosäuresequenz, die von der SEQ ID Nr. 8 dargestellt wird, oder
- einer Aminosäuresequenz, die eine prozentuale Identität von mindestens 88 %, insbesondere 90 %, bevorzugt mindestens 92 % und stärker bevorzugt mindestens 95 % mit SEQ IUD Nr. 8 aufweist, unter der Bedingung, dass die Aminosäuresequenz auch an der Transmembrandomäne, die am N-terminalen Ende des Proteins S des HBV liegt, deletiert ist, die Eigenschaften der Transmembrandomäne des Hüllproteins von HCV behält und dass sie die Fähigkeit behält, subvirale, nicht infektiöse, in Bezug auf das HVC und in Bezug auf das humane HBV immunogene Partikel zu bilden, oder,
- der Aminosäuresequenz einer synthetischen Variante, die von der SEQ ID Nr. 8 abgeleitet ist, unter der Bedingung, die Aminosäuresequenz der synthetischen Variante auch an der Transmembrandomäne, die am N-terminalen Ende des Proteins S von HBV liegt, deletiert ist, die Eigenschaften der Transmembrandomäne des Hüllproteins von HCV behält und dass sie die Fähigkeit behält, subvirale, nicht infektiöse und in Bezug auf das HCV und in Bezug auf das humane HBV immunogene Partikel zu bilden.

8. Immunogenes Fusionsprotein nach einem der Ansprüche 1 bis 5, wobei das zweite Peptid an N-terminaler Position aus Folgendem besteht:
- einer Aminosäuresequenz der Transmembrandomäne und der Ektodomäne eines Hüllproteins eines Isolats des Hepatitis-C-Virus (HCV) und begrenzt von den benachbarten Aminosäuren, die zwischen der Aminosäure an Position 384 bis zu der an Position 743 des Proteins E2 von HCV und insbesondere des Isolats HCV-1a, liegen,
und insbesondere der Aminosäuresequenz, die von der SEQ ID Nr. 6 dargestellt wird, oder
- einer Aminosäuresequenz, die eine prozentuale Identität von mindestens 78 %, insbesondere mindestens 80 %, bevorzugt mindestens 85 % und stärker bevorzugt mindestens 90 % mit der Sequenz der benachbarten Aminosäuren des Proteins E2 aufweist, unter der Bedingung, dass die Aminosäuresequenz die Eigenschaften der Transmembrandomäne und die immunogenen Eigenschaften in Bezug auf das Hepatitis-C-Virus behält, oder
- die Aminosäuresequenz einer synthetischen Variante, die von der Sequenz der benachbarten Aminosäuren der Transmembrandomäne und der Ektodomäne des Proteins E2 abgeleitet ist; unter der Bedingung, dass die Aminosäuresequenz die Eigenschaften der Transmembrandomäne und die immunogenen Eigenschaften in Bezug auf das Hepatitis-C-Virus behält.

9. Immunogenes Fusionsprotein nach einem der Ansprüche 5 oder 8, wobei das erste und das zweite Peptid benachbart sind, wobei das Fusionsprotein aus Folgendem besteht:
- der Aminosäuresequenz, die von der SEQ ID Nr. 10 dargestellt wird, oder
- einer Aminosäuresequenz, die eine prozentuale Identität von mindestens 86 %, insbesondere mindestens 88 %, bevorzugt mindestens 92 % und stärker bevorzugt mindestens 95 % mit SEQ ID Nr. 10 aufweist, unter der Bedingung, dass die Aminosäuresequenz auch an der Transmembrandomäne, die am N-terminalen Ende des Proteins S des HBV liegt, deletiert ist, die Eigenschaften der Transmembrandomäne des Hüllproteins von HCV behält und dass sie die Fähigkeit behält, subvirale, nicht infektiöse, in Bezug auf das humane Hepatitis-B-Virus und in Bezug auf das Hepatitis-C-Virus immunogene Partikel zu bildern, oder
- der Aminosäuresequenz einer synthetischen Variante, die von der SEQ ID Nr. 10 abgeleitet ist, unter der Bedingung, die Aminosäuresequenz der synthetischen Variante auch an der Transmembrandomäne, die am N-terminalen Ende des Proteins S von HBV liegt, deletiert ist, die Eigenschaften der Transmembrandomäne des Hüllproteins von HCV behält und dass sie die Fähigkeit behält, subvirale, nicht infektiöse und in Bezug auf das humane Hepatits-B-Virus und in Bezug auf das Hepatitis-C-Virus immunogene Partikel zu bilden.

10. Hybrides Nukleinsäuremolekül, das für eines der Fusionsproteine nach einem der Ansprüche 1 bis 9 kodiert.

11. Hybrides Nukleinsäuremolekül nach Anspruch 10, wobei eine erste Nukleinsäuresequenz für das erste Peptid kodiert, eine zweite Nukleinsäuresequenz für das zweite Peptid kodiert, wobei die erste und die zweite Nukleinsäuresequenz benachbart sind, und das 5'-Ende der ersten Nukleinsäuresequenz kovalent mit dem 3'-Ende der zweiten Nukleinsäuresequenz verbunden ist.

12. Hybrides Nukleinsäuremolekül nach einem der Ansprüche 10 und 11, das für ein oben genanntes Fusionsprotein kodiert, das ein Transferinitiationspeptid umfasst, das auf der N-terminalen Seite liegt, wobei das hybride Nukleinsäuremolekül dargestellt wird durch:
- die SEQ ID Nr. 11, oder
- eine Nukleinsäuresequenz, die eine Identität von mindestens 78 %, insbesondere mindestens 80 %, bevorzugt mindestens 85 % und stärker bevorzugt mindestens 90 % mit der SEQ ID Nr. 11 aufweist.

13. Hybrides Nukleinsäuremolekül nach einem der Ansprüche 10 und 11, das für ein oben genanntes Fusionsprotein kodiert, das ein Transferinitiationspeptid umfasst, das auf der N-terminalen Seite liegt, wobei das hybride Nukleinsäuremolekül dargestellt wird durch:
- die SEQ ID Nr. 13, oder
- eine Nukleinsäuresequenz, die eine Identität von mindestens 80 %, insbesondere mindestens 85 %, bevorzugt mindestens 90 % und stärker bevorzugt mindestens 95 % mit der SEQ ID Nr. 13 aufweist.

14. Vektor, der ein hybrides Nukleinsäuremolekül nach einem der Ansprüche 10 bis 13 sowie die Mittel umfasst, die für deren Expression erforderlich sind.

15. Vektor nach Anspruch 14, der ferner einen Lentivirusvektor umfasst.

16. Vektor nach einem der Ansprüche 14 bis 15, wobei es sich bei dem hybriden Nukleinsäuremolekül um ein Nukleinsäuremolekül nach Anspruch 12 oder 13 handelt.

17. Subviraler, chimärer, immunogener und nicht infektiöser Hüllpartikel, der die folgenden Proteine umfasst:
- das Protein, das aus dem Wildtyp-Protein S des Oberflächenantigens eines Isolats des Hepatitis-B-Virus besteht, und
- mindestens ein immunogenes Fusionsprotein nach einem der Ansprüche 1 bis 9.

18. Subviraler, chimärer, immunogener Partikel nach Anspruch 17, wobei das immunogene Fusionsprotein aus Folgendem besteht:
- der Aminosäuresequenz, die von der SEQ ID Nr. 8 dargestellt wird, oder
- einer Aminosäuresequenz, die eine prozentuale Identität von mindestens 88 %, insbesondere mindestens 90 %, bevorzugt mindestens 92 % und stärker bevorzugt mindestens 95 % mit der SEQ ID Nr. 8 aufweist.

19. Subviraler, chimärer, immunogener Partikel nach Anspruch 17, wobei das immunogene Fusionsprotein aus Folgendem besteht:
- der Aminosäuresequenz, die von der SEQ ID Nr. 10 dargestellt wird, oder
- einer Aminosäuresequenz, die eine prozentuale Identität von mindestens 86 %, insbesondere mindestens 88 %, bevorzugt mindestens 92 % und stärker bevorzugt mindestens 95 % mit der SEQ ID Nr. 10 aufweist.

20. Subviraler, chimärer, immunogener Partikel nach Anspruch 17, der die beiden folgenden Fusionsproteine aufweist:
- die Aminosäuresequenz, die von der SEQ ID Nr. 8 dargestellt wird, oder
- eine Aminosäuresequenz, die eine prozentuale Identität von mindestens 88 %, insbesondere mindestens 90 %, bevorzugt mindestens 92 % und stärker bevorzugt mindestens 95 % mit der SEQ ID Nr. 8 aufweist, und
- die Aminosäuresequenz, die von der SEQ ID Nr. 10 dargestellt wird, oder
- eine Aminosäuresequenz, die eine prozentuale Identität von mindestens 86 %, insbesondere mindestens 88 %, bevorzugt mindestens 90 % und stärker bevorzugt mindestens 95 % mit der SEQ ID Nr. 10 aufweist.

21. Immunogene Zusammensetzung, die als Wirkstoff mindestens ein Element umfasst, ausgewählt aus:
- einem immunogenen Fusionsprotein nach einem der Ansprüche 1 bis 9,
- einem hybriden Nukleinsäuremolekül nach einem der Ansprüche 10 bis 13,
- einem Vektor nach einem der Ansprüche 14 bis 16, der das hybride Nukleinsäuremolekül umfasst,
- einem subviralen, chimären Partikel nach einem der Ansprüche 17 bis 20, und einem pharmazeutisch verträglichen Vehikel.

22. Immunogene Zusammensetzung nach Anspruch 21, wobei der Wirkstoff mindestens eine der drei folgenden Verbindungen ist:
a)- ein Fusionsprotein nach Anspruch 7,
b)- ein hybrides Nukleinsäuremolekül nach Anspruch 12 oder ein Vektor, der das hybride Nukleinsäuremolekül umfasst;
c)- ein subviraler, chimärer Partikel nach Anspruch 18.

23. Immunogene Zusammensetzung nach Anspruch 21, wobei der Wirkstoff mindestens eine der folgenden Verbindungen ist:
a)- ein Fusionsprotein nach Anspruch 9,
b)- ein hybrides Nukleinsäuremolekül nach Anspruch 13 oder ein Vektor, der das hybride Nukleinsäuremolekül umfasst;
c)- ein subviraler, chimärer Partikel nach Anspruch 19.

24. Immunogene Zusammensetzung nach Anspruch 21, wobei es sich bei dem Wirkstoff um einen subviralen, chimären Partikel handelt, der mindestens die beiden immunogenen Fusionsproteine nach den Ansprüchen 7 und 9 umfasst.

25. Immunogene Zusammensetzung nach Anspruch 21, wobei der Wirkstoff aus dem Gemisch besteht, das Folgendes umfasst:
- mindestens zwei der folgenden Fusionsproteine:
* eines nach Anspruch 7;
* das andere nach Anspruch 9;
- mindestens zwei der folgenden hybriden Nukleinsäuremoleküle:
* eines nach Anspruch 12 oder einen Vektor, der das hybride Nukleinsäuremolekül umfasst;
* das andere nach Anspruch 13 oder einen Vektor, der das hybride Nukleinsäuremolekül umfasst;
- mindestens zwei der folgenden subviralen, chimären Partikel:
* einen nach Anspruch 18;
* den anderen nach Anspruch 19.

26. Verwendung einer immunogenen Zusammensetzung nach einem der Ansprüche 21 bis 25 zur Herstellung eines Medikaments zur prophylaktischen Behandlung und/oder zur Prävention von Hepatitis C.

27. Verwendung einer immunogenen Zusammensetzung nach einem der Ansprüche 21 bis 25 zur Herstellung eines Medikaments zur prophylaktischen Behandlung und/oder zur Prävention von Hepatitis B.

28. Verwendung einer immunogenen Zusammensetzung nach einem der Ansprüche 21 bis 25 zur Herstellung eines Medikaments zur prophylaktischen Behandlung und/oder zur Prävention von Hepatitis B und Hepatitis C.

29. Zelllinie, die subvirale, chimäre, immunogene, nicht infektiöse Partikel nach Anspruch 17-20 exprimiert, wobei die Linie den Vektor nach den Ansprüchen 14-16 umfasst.

30. Zelllinie nach Anspruch 29, wobei es sich dabei um die Ovarialzelllinie des chinesischen Hamsters, genannt CHO, handelt.

31. Zellinie nach Anspruch 29, bei der es sich um eine Hefe handelt, wobei die Hefe insbesondere Saccharomyces Cerevisiae sein kann.

## Claims

1. Immunogenic fusion protein comprising at least the tow following peptides:
**a)-** on the C-terminal side, a first peptide constituted :
- by the amino acid sequence of the S protein of an isolate of human hepatitis B virus (HBV), which S protein is deleted of its transmembrane domain located at the N-terminal end thereof, the said amino acid sequence maintaining the ability to form immunogenic non infectious subviral particles with respect to the hepatitis B human virus, or
- by an amino acid sequence having a percentage of identity of at least 91%, especially of at least 93%, particularly of at least 95%, and more particularly of at least 97%, with said amino acid sequence of S protein being deleted on the end, provided that said amino acid sequence is also deleted of the transmembrane domain located at the N-terminal end of the protein S of HBV, and maintains the ability to form immunogenic non infectious subviral particles, with respect to the human hepatitis B virus, or,
- by the amino acid sequence of a synthetic variant derived from said amino acid sequence of said deleted S protein, provided that said amino acid sequence of said synthetic variant is also deleted of the transmembrane domain thereof located at the N-terminal end of the S protein of HBV, and maintains the ability to form immunogenic non infectious subviral particles, with respect to the human hepatitis B virus, and,
**b**)- on the N-terminal side, a second peptide constituted :
- by the amino acid sequence of the transmembrane domain and the ectodomain of at least one envelope protein of a hepatitis C virus (HCV) isolate, or
- by an amino acid sequence having a percentage of identity of at least 78%, especially of at least 80%, particularly of at least 85%, and more particularly of at least 90%, with said amino acid sequence of the transmembrane domain and of the ectodomain of an envelope protein of a hepatitis C virus isolate, provided that said amino acid sequence maintains properties of said transmembrane domain and immunogenic properties with respect to the hepatitis C virus, or
- by the amino acid sequence of a synthetic variant derived from said amino acid sequence of the transmembrane and of the ectodomain protein E1 or E2 of HCV, provided that said amino acid sequence maintains the properties of said transmembrane domain and the immunogenic properties with respect to the hepatitis C virus, said envelope protein of a hepatitis C virus isolate being chosen from the protein E1, the protein E2 or a fusion peptide comprising the protein E1 and the protein E2.

2. Immunogenic fusion protein according to claim 1, in which the second peptide located on the N-terminal side, is constituted :
- by the totality of the amino acid sequence of the transmembrane domain and of the ectodomain of the envelope protein E1, or
- by an amino acid sequence having a percentage of identity of at least 75%, especially of at least 80%, particularly of at least 85%, and more particularly of at least 90%, with said amino acid sequence of the transmembrane domain and of the ectodomain of the envelope protein E1, provided that said amino acid sequence maintains the properties of said transmembrane domain and the immunogenic properties with respect to the hepatitis C virus, or
- by the amino acid sequence of a synthetic variant derived from said protein E1; provided that said amino acid sequence maintains the properties of said transmembrane domain and the immunogenic properties with respect to the hepatitis C virus.

3. Immunogenic fusion protein according to claim 1, in which the second peptide located on the N-terminal side, is constituted:
- by the totality of the amino acid sequence of the transmembrane domain and the ectodomain of the envelope protein E2, or
- by an amino acid sequence having a percentage of identity of at least 78%, especially of at least 80%, particularly of at least 85%, and more particularly of at least 90%, with said amino acid sequence of the transmembrane domain and of the ectodomain of the envelope protein E2, provided that said amino acid sequence maintains the properties of said transmembrane domain and the immunogenic properties with respect to the hepatitis C virus, or
- by the amino acid sequence of a synthetic variant derived from said protein E2, provided that said amino acid sequence maintains the properties of said transmembrane domain and the immunogenic properties with respect to the hepatitis C virus.

4. Immunogenic fusion protein according to any of claims 1 to 3, in which the first and the second peptide constituting the immunogenic fusion protein are contiguous, and the C-terminal end of the second peptide is linked in a covalent manner to the N-terminal end of the first peptide.

5. Immunogenic fusion protein according to any of claims 1 to 4, in which the first peptide in C-terminal position is constitued:
- by an amino acid sequence bounded by the contiguous amino acids located in the region composed from the amino acid in position 23 to that in position 226 of the S protein, of HBV, and particularly the HBVadw isolate,
and especially by the amino acid sequence represented by the SEQ ID N°2, said amino acid sequence maintaining the ability to form immunogenic non infectious subviral particles with respect to the hepatitis B human virus, or
- by an amino acid sequence having a percentage of identity of at least 91%, especially of at least 93%, particularly of at least 95%, and more particularly of at least 97%, with said contiguous amino acid sequence of the S protein, provided that said amino acid sequence is also deleted of the transmembrane domain located at the N-terminal end of the S protein of HBV, and maintains the ability to form immunogenic non infectious subviral particles, vis-à-vis the hepatitis B human virus, or
- by the amino acid sequence of a synthetic variant, derived from said amino acid sequence of said S protein; provided that said amino acid sequence of said synthetic variant is also deleted of the transmembrane domain located at the N-terminal end of the S protein of HBV, and maintains the ability to form immunogenic non infectious subviral particles, with respect to the hepatitis B virus.

6. Immunogenic fusion protein according to any of claims 1 to 5, in which the second peptide in the N-terminal position is constituted:
- by an amino acid sequence of the transmembrane domain and of the ectodomain of the envelope protein of a hepatitis C virus isolate and bounded by the contiguous amino acids located in the region composed from the amino acid in position 192 to that in position 380 of the protein E1 of HCV, and particularly of the HCV-1a isolate,
and especially by the amino acid sequence represented by the SEQ ID N°4, or
- by an amino acid sequence having a percentage of identity of at least 75%, especially of at least 80%, particularly of at least 85%, and more particularly of at least 90%, with said contiguous amino acid sequence of said protein E1, provided that said amino acid sequence maintains the properties of said transmembrane domain and the immunogenic properties with respect to the hepatitis C virus, or
- by the amino acid sequence of a synthetic variant derived from said contiguous amino acid sequence of the transmembrane domain and of the ectodomain of said protein E1, provided that said amino acid sequence maintains the properties of said transmembrane domain and the immunogenic properties with respect to the hepatitis C virus.

7. Immunogenic fusion protein according to one of claims 5 or 6, in which the first and the second peptide are contiguous, said fusion protein being constituted by:
- the amino acid sequence represented by the SEQ ID N°8, or
- an amino acid sequence having a percentage of identity of at least 88%, especially of at least 90%, particularly of at least 92%, and more particularly of at least 95%, with SEQ ID N°8, provided that said amino acid sequence is also deleted of the transmembrane domain located at the N-terminal end of the S protein of HBV, maintains the properties of the transmembrane domain of the envelope protein of HCV and maintains the ability to form immunogenic non infectious subviral particles, with respect to HCV and with respect to human HBV, or
- by the amino acid sequence of a synthetic variant derived from said SEQ ID N°8, provided that said amino acid sequence of said synthetic variant is also deleted of the transmembrane domain located at the N-terminal end of the S protein of HBV, maintains the properties of the transmembrane domain of envelope protein of HCV and maintains the ability to form immunogenic non infectious subviral particles, with respect to HCV and with respect to human HBV.

8. Immunogenic fusion protein according to any of claims 1 to 5, in which the second peptide in N-terminal position is constituted:
- by an amino acid sequence of the transmembrane domain and of the ectodomain of the envelope protein of a hepatitis C virus isolate and bounded by the contiguous amino acids located from the amino acid in position 384 to that in position 743 of the protein E2 of HCV, and particularly the HCV-1a isolate,
and especially by the amino acid sequence represented by the SEQ ID N°6, or
- by an amino acid sequence having a percentage of identity of at least 78%, especially of at least 80%, particularly of at least 85%, and more particularly of at least 90%, with said contiguous amino acid sequence of protein E2, provided that said amino acid sequence maintains the properties of said transmembrane domain and the immunogenic properties with respect to the hepatitis C virus, or
- by the amino acid sequence of a synthetic variant, derived from said contiguous amino acid sequence of the transmembrane domain and of the ectodomain of said protein E2, provided that said amino acid sequence maintains the properties of said transmembrane domain and the immunogenic properties with respect to the hepatitis C virus.

9. Immunogenic fusion protein according to one of claims 5 or 8, in which the first and the second peptide are contiguous, said fusion protein being constituted by:
- the amino acid sequence represented by the SEQ ID N°10, or
- an amino acid sequence having a percentage of identity of at least 86%, especially of at least 88%, particularly of at least 92%, and more particularly of at least 95%, with SEQ ID N°10, provided that said amino acid sequence is also deleted of the transmembrane domain located at the N-terminal end of the S protein of HBV, maintains the properties of the transmembrane domain of the envelope protein of HCV and maintains the ability to form immunogenic non infectious subviral particles, with respect to the hepatitis B human virus and with respect to the hepatitis C virus, or
- by the amino acid sequence of a synthetic variant derived from said SEQ ID N °10, provided that said amino acid sequence of said synthetic variant is also deleted of the transmembrane domain located at the N-terminal end of the S protein of HBV, maintains the properties of the transmembrane domain of the envelope protein of HCV and maintains the ability to form immunogenic non infectious subviral particles, with respect to the hepatitis B human virus and with respect to the hepatitis C virus.

10. Hybrid nucleic acid molecule encoding any one of the fusion proteins according to any of claims 1 to 9.

11. Hybrid nucleic acid molecule according to claim 10, in which a first nucleic acid sequence encodes said first peptide, a second nucleic acid sequence encodes said second peptide, the first and the second nucleic acid sequence are contiguous, and the 5' end of the first nucleic acid sequence is linked in a covalent manner to the 3' end of the second nucleic acid sequence.

12. Hybrid nucleic acid molecule according to any of claims 10 and 11, encoding an aforementioned fusion protein comprising a transfer initiation peptide located on the N-terminal side, said hybrid nucleic acid molecule being represented by:
- the SEQ ID N°11, or
- a nucleic acid sequence having an identity of at least 78%, especially of at least 80%, particularly of at least 85%, and more particularly of at least 90%, with said SEQ ID N°11.

13. Hybrid nucleic acid molecule according to any of claims 10 and 11, encoding for an aforementioned fusion protein comprising a transfer initiation peptide located on the N-terminal side, said hybrid nucleic acid molecule being represented by:
- the SEQ ID N°13, or
- a nucleic acid sequence having an identity of at least 80%, especially of at least 85%, particularly of at least 90%, and more particularly of at least 95%, with said SEQ ID N°13.

14. Vector comprising a hybrid nucleic acid molecule according to any of claims 10 to 13, as well as the necessary means for their expression.

15. Vector according to claim 14, further comprising a lentiviral vector.

16. Vector according to one of claims 14 to 15, in which the hybrid nucleic acid molecule is a nucleic acid molecule according to claim 12 or 13.

17. Subviral, chimeric, immunogenic and non infectious envelope particle, comprising the following proteins:
- the protein constituted by the wild-type S protein of the surface antigen of a hepatitis B virus (HBV) isolate, and,
- at least one immunogenic fusion protein according to any of claims 1 to 9.

18. Immunogenic chimeric subviral particle according to claim 17, in which the immunogenic fusion protein is constituted by:
- the amino acid sequence represented by the SEQ ID N°8, or
- an amino acid sequence having a percentage of identity of at least 88%, especially of at least 90%, particularly of at least 92%, and more particularly of at least 95%, with said SEQ ID N°8.

19. Immunogenic chimeric subviral particle according to claim 17, in which the immunogenic fusion protein is constituted by:
- the amino acid sequence represented by the SEQ ID N°10, or
- an amino acid sequence having a percentage of identity of at least 86%, especially of at least 88%, particularly of at least 92%, and more particularly of at least 95%, with SEQ ID N°10.

20. Immunogenic chimeric subviral particle according to claim 17, comprising the two following fusion proteins:
- the amino acid sequence represented by the SEQ ID N°8, or
- an amino acid sequence having a percentage of identity of at least 88%, especially of at least 90%, particularly of at least 92%, and more particularly of at least 95%, with said SEQ ID N°8, and
- the amino acid sequence represented by the SEQ ID N°10, or
- an amino acid sequence having a percentage of identity of at least 86%, especially of at least 88%, particularly of at least 90%, and more particularly of at least 95%, with SEQ ID N°10.

21. Immunogenic composition comprising as active substance at least one element chosen from:
- a fusion protein according to any of claims 1 to 9,
- a hybrid nucleic acid molecule according to any of claims 10 to 13,
- a vector according to any of claims 14 to 16, comprising said hybrid nucleic acid molecule,
- a chimeric subviral particle according to any of claims 17 to 20,
and, a pharmaceutically acceptable vehicle.

22. Immunogenic composition according to claim 21, in which the active substance is at least one of the following three compounds:
a)- a fusion protein according to claim 7;
b)- a hybrid nucleic acid molecule according to claim 12, or a vector comprising said hybrid nucleic acid molecule;
c)- a chimeric subviral particle according to claim 18.

23. Immunogenic composition according to claim 21, in which the active substance is at least one of the following compounds:
a)- a fusion protein according to claim 9;
b)- a hybrid nucleic acid molecule according to claim 13, or a vector comprising said hybrid nucleic acid molecule;
c)- a chimeric subviral particle, according to claim 19.

24. Immunogenic composition according to claim 21, in which the active substance is a chimeric subviral particle, comprising at least the two immunogenic fusion proteins according to claim 7 and 9.

25. Immunogenic composition according to claim 21, in which the active substance is constituted by the mixture comprising:
- at least two of the following fusion proteins:
* one according to claim 7;
* the other according to claim 9;
- at least two of the following hybrid nucleic acid molecules:
* one according to claim 12, or a vector comprising said hybrid nucleic acid molecule;
* the other according to claim 13, or a vector comprising said hybrid nucleic acid molecule;
- at least two of the following chimeric subviral particles:
* one according to claim 18;
* the other according to claim 19.

26. Use of an immunogenic composition of any of claims 21 to 25, for the manufacture of a medicine for prophylactic treatment and/or for the prevention of hepatitis C.

27. Use of an immunogenic composition of any of claims 21 to 25, for the manufacture of a medicine for the prophylactic treatment and/or for the prevention of hepatitis B.

28. Use of an immunogenic composition of any of claims 21 to 25, for the manufacture of a medicine for the prophylactic treatment and/or for the prevention of hepatitis B and of hepatitis C.

29. A cell line that expresses chimeric, immunogenic non infectious subviral particles of claims 17 to 20, said line comprising the vector according to claims 14 to 16.

30. A cell line according to claim 29, which is the Chinese hamster ovary line named CHO.

31. A cell line according to claim 29, which is yeast, said yeast being in particular *Saccharomyces cerevisae.*


### LISTE DE SEQUENCES

<110> UNIVERSITE FRANCOIS RABELAIS DE TOURS Roingeard, Philippe Hourioux, Christophe Patient, Romuald
<120> NOUVELLES PROTEINES DE FUSION ET LEUR APPLICATION COMME VACCINS CONTRE L'HEPATITE C
<130> IFB 08 TOU VACC
<160> 16
<170> PatentIn version 3.5
<210> 1
   <211> 612
   <212> DNA
   <213> Hepatitis B virus
<220>
   <221> CDS
   <222> (1)..(612)
<400> 1
   aca aga atc ctc aca ata ccg cag agt cta gac tcg tgg tgg act tct      48
   Thr Arg Ile Leu Thr Ile Pro Gln Ser Leu Asp Ser Trp Trp Thr Ser
   1               5                   10                  15
   ctc aat ttt cta ggg gga tct ccc gtg tgt ctt ggc caa aat tcg cag      96
   Leu Asn Phe Leu Gly Gly Ser Pro Val Cys Leu Gly Gln Asn Ser Gln
               20                  25                  30
   tcc cca acc tcc aat cac tca cca acc tcc tgt cct cca att tgt cct      144
   Ser Pro Thr Ser Asn His Ser Pro Thr Ser Cys Pro Pro Ile Cys Pro
           35                  40                  45
   ggt tat cgc tgg atg tgt ctg cgg cgt ttt atc ata ttc ctc ttc atc      192
   Gly Tyr Arg Trp Met Cys Leu Arg Arg Phe Ile Ile Phe Leu Phe Ile
       50                  55                  60
   ctg ctg cta tgc ctc atc ttc tta ttg gtt ctt ctg gat tat caa ggt      240
   Leu Leu Leu Cys Leu Ile Phe Leu Leu Val Leu Leu Asp Tyr Gln Gly
   65                  70                  75                  80
   atg ttg ccc gtt tgt cct cta att cca gga tca aca aca acc agt acg      288
   Met Leu Pro Val Cys Pro Leu Ile Pro Gly Ser Thr Thr Thr Ser Thr
                   85                  90                  95
   gga cca tgc aaa acc tgc acg act cct gct caa ggc aac tct atg ttt      336
   Gly Pro Cys Lys Thr Cys Thr Thr Pro Ala Gln Gly Asn Ser Met Phe
               100                 105                 110
   ccc tca tgt tgc tgt aca aaa cct acg gat gga aat tgc acc tgt att      384
   Pro Ser Cys Cys Cys Thr Lys Pro Thr Asp Gly Asn Cys Thr Cys Ile
           115                 120                 125
   ccc atc cca tcg tcc tgg gct ttc gca aaa tac cta tgg gag tgg gcc      432
   Pro Ile Pro Ser Ser Trp Ala Phe Ala Lys Tyr Leu Trp Glu Trp Ala
       130                 135                 140
   tca gtc cgt ttc tct tgg ctc agt tta cta gtg cca ttt gtt cag tgg      480
   Ser Val Arg Phe Ser Trp Leu Ser Leu Leu Val Pro Phe Val Gln Trp
   145                 150                 155                 160
   ttc gta ggg ctt tcc ccc act gtt tgg ctt tca gct ata tgg atg atg      528
   Phe Val Gly Leu Ser Pro Thr Val Trp Leu Ser Ala Ile Trp Met Met
                   165                 170                 175
   tgg tat tgg ggg cca agt ctg tac agc atc gtg agt ccc ttt ata ccg      576
   Trp Tyr Trp Gly Pro Ser Leu Tyr Ser Ile Val Ser Pro Phe Ile Pro
               180                 185                 190
   ctg tta cca att ttc ttt tgt ctc tgg gta tac att                      612
   Leu Leu Pro Ile Phe Phe Cys Leu Trp Val Tyr Ile
           195                 200
<210> 2
   <211> 204
   <212> PRT
   <213> Hepatitis B virus
<400> 2
   Thr Arg Ile Leu Thr Ile Pro Gln Ser Leu Asp Ser Trp Trp Thr Ser
   1 5 10 15
   Leu Asn Phe Leu Gly Gly Ser Pro Val Cys Leu Gly Gln Asn Ser Gln
   20 25 30
   Ser Pro Thr Ser Asn His Ser Pro Thr Ser Cys Pro Pro Ile Cys Pro
   35 40 45
   Gly Tyr Arg Trp Met Cys Leu Arg Arg Phe Ile Ile Phe Leu Phe Ile
   50 55 60
   Leu Leu Leu Cys Leu Ile Phe Leu Leu Val Leu Leu Asp Tyr Gln Gly
   65 70 75 80
   Met Leu Pro Val Cys Pro Leu Ile Pro Gly Ser Thr Thr Thr Ser Thr
   85 90 95
   Gly Pro Cys Lys Thr Cys Thr Thr Pro Ala Gln Gly Asn Ser Met Phe
   100 105 110
   Pro Ser Cys Cys Cys Thr Lys Pro Thr Asp Gly Asn Cys Thr Cys Ile
   115 120 125
   Pro Ile Pro Ser Ser Trp Ala Phe Ala Lys Tyr Leu Trp Glu Trp Ala
   130 135 140
   Ser Val Arg Phe Ser Trp Leu Ser Leu Leu Val Pro Phe Val Gln Trp
   145 150 155 160
   Phe Val Gly Leu Ser Pro Thr Val Trp Leu Ser Ala Ile Trp Met Met
   165 170 175
   Trp Tyr Trp Gly Pro Ser Leu Tyr Ser Ile Val Ser Pro Phe Ile Pro
   180 185 190
   Leu Leu Pro Ile Phe Phe Cys Leu Trp Val Tyr Ile
   195 200
<210> 3
   <211> 576
   <212> DNA
   <213> Hepatitis C virus
<220>
   <221> CDS
   <222> (1)..(576)
<400> 3
   tac caa gta cgc aac tcc tcg ggc ctt tac cat gtc acc aat gat tgc      48
   Tyr Gln Val Arg Asn Ser Ser Gly Leu Tyr His Val Thr Asn Asp Cys
   1               5                   10                  15
   cct aac tcg agt att gtg tac gag acg gcc gat acc atc cta cac tct      96
   Pro Asn Ser Ser Ile Val Tyr Glu Thr Ala Asp Thr Ile Leu His Ser
               20                  25                  30
   ccg ggg tgt gtc cct tgc gtt cgc gag ggc aat gcc tca aaa tgt tgg      144
   Pro Gly Cys Val Pro Cys Val Arg Glu Gly Asn Ala Ser Lys Cys Trp
           35                  40                  45
   gtg gcg gtg gcc cct aca gtc gcc acc aga gac ggc aag ctc ccc aca      192
   Val Ala Val Ala Pro Thr Val Ala Thr Arg Asp Gly Lys Leu Pro Thr
       50                  55                  60
   acg cag ctt cga cgt cac atc gat ctg ctc gtc agg agc gcc acc ctc      240
   Thr Gln Leu Arg Arg His Ile Asp Leu Leu Val Arg Ser Ala Thr Leu
   65                  70                  75                  80
   tgc tcg gcc ctc tat gtg ggg gac ttg tgc ggg tcc gtc ttc ctc gtc      288
   Cys Ser Ala Leu Tyr Val Gly Asp Leu Cys Gly Ser Val Phe Leu Val
                   85                  90                  95
   ggt caa ctg ttc acc ttc tcc ccc agg cgc cac tgg aca acg caa gac      336
   Gly Gln Leu Phe Thr Phe Ser Pro Arg Arg His Trp Thr Thr Gln Asp
               100                 105                 110
   tgc aac tgt tcc atg tac ccc ggc cat ata acg ggt cac cgt atg gca      384
   Cys Asn Cys Ser Met Tyr Pro Gly His Ile Thr Gly His Arg Met Ala
           115                 120                 125
   tgg gac atg atg atg aac tgg tcc cct acg aca gcg ctg gta gta gct      432
   Trp Asp Met Met Met Asn Trp Ser Pro Thr Thr Ala Leu Val Val Ala
       130                 135                 140
   cag ctg ctc agg gtc ccg caa gcc atc ttg gac atg atc gct ggt gcc      480
   Gln Leu Leu Arg Val Pro Gln Ala Ile Leu Asp Met Ile Ala Gly Ala
   145                 150                 155                 160
   cac tgg gga gtc cta gcg ggc ata gcg tat ttc tcc atg gtg ggg aac      528
   His Trp Gly Val Leu Ala Gly Ile Ala Tyr Phe Ser Met Val Gly Asn
                   165                 170                 175
   tgg gcg aag gtc ctg gtg gtg ctg ttg ctg ttc gcc agc gtc gat gcg      576
   Trp Ala Lys Val Leu Val Val Leu Leu Leu Phe Ala Ser Val Asp Ala
               180                 185                 190
<210> 4
   <211> 192
   <212> PRT
   <213> Hepatitis C virus
<400> 4
   Tyr Gln Val Arg Asn Ser Ser Gly Leu Tyr His Val Thr Asn Asp Cys
   1               5                   10                  15
   Pro Asn Ser Ser Ile Val Tyr Glu Thr Ala Asp Thr Ile Leu His Ser
               20                  25                  30
   Pro Gly Cys Val Pro Cys Val Arg Glu Gly Asn Ala Ser Lys Cys Trp
           35                  40                  45
   Val Ala Val Ala Pro Thr Val Ala Thr Arg Asp Gly Lys Leu Pro Thr
       50                  55                  60
   Thr Gln Leu Arg Arg His Ile Asp Leu Leu Val Arg Ser Ala Thr Leu
   65                  70                  75                  80
   Cys Ser Ala Leu Tyr Val Gly Asp Leu Cys Gly Ser Val Phe Leu Val
                   85                  90                  95
   Gly Gln Leu Phe Thr Phe Ser Pro Arg Arg His Trp Thr Thr Gln Asp
               100                 105                 110
   Cys Asn Cys Ser Met Tyr Pro Gly His Ile Thr Gly His Arg Met Ala
           115                 120                 125
   Trp Asp Met Met Met Asn Trp Ser Pro Thr Thr Ala Leu Val Val Ala
       130                 135                 140
   Gln Leu Leu Arg Val Pro Gln Ala Ile Leu Asp Met Ile Ala Gly Ala
   145                 150                 155                 160
   His Trp Gly Val Leu Ala Gly Ile Ala Tyr Phe Ser Met Val Gly Asn
                   165                 170                 175
   Trp Ala Lys Val Leu Val Val Leu Leu Leu Phe Ala Ser Val Asp Ala
               180                 185                 190
<210> 5
   <211> 1089
   <212> DNA
   <213> Hepatitis C virus
<220>
   <221> CDS
   <222> (1)..(1089)
<400> 5
   gaa acc tac acc acc ggg ggg agt att gcc aaa acc gtg caa gga ttc        48
   Glu Thr Tyr Thr Thr Gly Gly Ser Ile Ala Lys Thr Val Gln Gly Phe
   1               5                   10                  15
   acc agt ttt ttt acc cca ggc gcc aag cag gac atc cag ctg atc aac        96
   Thr Ser Phe Phe Thr Pro Gly Ala Lys Gln Asp Ile Gln Leu Ile Asn
               20                  25                  30
   acc aac ggc agt tgg cac atc aat cgc acg gcc ttg aac tgt aat gcg        144
   Thr Asn Gly Ser Trp His Ile Asn Arg Thr Ala Leu Asn Cys Asn Ala
           35                  40                  45
   agc ctc gaa acc ggc tgg ata gcg ggg ctc ttc tac tac aac aaa ttc        192
   Ser Leu Glu Thr Gly Trp Ile Ala Gly Leu Phe Tyr Tyr Asn Lys Phe
       50                  55                  60
   aac tcc tca ggc tgc ccc gag agg atg gcc agc tgc aaa ccc ctt gcc        240
   Asn Ser Ser Gly Cys Pro Glu Arg Met Ala Ser Cys Lys Pro Leu Ala
   65                  70                  75                  80
   tat ttc gcc caa ggc tgg ggc cct atc agc cat gtc aac gga agc ggc        288
   Tyr Phe Ala Gln Gly Trp Gly Pro Ile Ser His Val Asn Gly Ser Gly
                   85                  90                  95
   ccc gaa cag cgc ccc tac tgc tgg cac tac gcc cca agg cct tgt ggt        336
   Pro Glu Gln Arg Pro Tyr Cys Trp His Tyr Ala Pro Arg Pro Cys Gly
               100                 105                 110
   atc gtg tca gca cag aca gta tgt ggc cca gtg tat tgt ttc act cct        384
   Ile Val Ser Ala Gln Thr Val Cys Gly Pro Val Tyr Cys Phe Thr Pro
           115                 120                 125
   agc ccc gtg gtg gtg ggg acg acc gac aag ttg ggc gcg cct acc tac        432
   Ser Pro Val Val Val Gly Thr Thr Asp Lys Leu Gly Ala Pro Thr Tyr
       130                 135                 140
   aac tgg ggt gag aat gat acg gac gtc ttc gtc ctc aat aac acc agg        480
   Asn Trp Gly Glu Asn Asp Thr Asp Val Phe Val Leu Asn Asn Thr Arg
   145                 150                 155                 160
   cca ccg ttg ggc aat tgg ttc ggt tgc acc tgg atg aac tca tct gga      528
   Pro Pro Leu Gly Asn Trp Phe Gly Cys Thr Trp Met Asn Ser Ser Gly
                   165                 170                 175
   ttc acc aaa gtg tgc gga gcg cct ccc tgt gcc atc gga gga gtg ggc      576
   Phe Thr Lys Val Cys Gly Ala Pro Pro Cys Ala Ile Gly Gly Val Gly
               180                 185                 190
   aat aac acc ttg cgc tgt ccc act gac tgt ttc cgc aag cat ccg gaa      624
   Asn Asn Thr Leu Arg Cys Pro Thr Asp Cys Phe Arg Lys His Pro Glu
           195                 200                 205
   gct aca tac tct cga tgt ggc tcc ggt ccc tgg atc acg ccc agg tgc      672
   Ala Thr Tyr Ser Arg Cys Gly Ser Gly Pro Trp Ile Thr Pro Arg Cys
       210                 215                 220
   ctg gtc gac tat cct tat agg ctc tgg cat tat cct tgc act gtc aac      720
   Leu Val Asp Tyr Pro Tyr Arg Leu Trp His Tyr Pro Cys Thr Val Asn
   225                 230                 235                 240
   tac acc ctg ttt aaa atc agg atg tac gtg gga ggg gtc gag cac agg      768
   Tyr Thr Leu Phe Lys Ile Arg Met Tyr Val Gly Gly Val Glu His Arg
                   245                 250                 255
   cta caa gct gct tgc aac tgg acg cgg ggc gag cgt tgt gat ctg gac      816
   Leu Gln Ala Ala Cys Asn Trp Thr Arg Gly Glu Arg Cys Asp Leu Asp
               260                 265                 270
   gac agg gac agg tcc gag ctc agc ccg ctg ctg ctg tcc acc acg cag      864
   Asp Arg Asp Arg Ser Glu Leu Ser Pro Leu Leu Leu Ser Thr Thr Gln
           275                 280                 285
   tgg cag gtc ctt ccg tgt tct ttc acg acc ttg cca gcc ttg acc acc      912
   Trp Gln Val Leu Pro Cys Ser Phe Thr Thr Leu Pro Ala Leu Thr Thr
       290                 295                 300
   ggc ctc atc cac ctc cat cag aac atc gtg gac gtg caa tat ttg tac      960
   Gly Leu Ile His Leu His Gln Asn Ile Val Asp Val Gln Tyr Leu Tyr
   305                 310                 315                 320
   ggg gtg ggg tca agc att gtg tcc tgg gcc atc aag tgg gag tac gtc     1008
   Gly Val Gly Ser Ser Ile Val Ser Trp Ala Ile Lys Trp Glu Tyr Val
                   325                 330                 335
   att ctc ttg ttt ctc ctg ctt gca gac gcg cgc atc tgc tcc tgc ttg     1056
   Ile Leu Leu Phe Leu Leu Leu Ala Asp Ala Arg Ile Cys Ser Cys Leu
               340                 345                 350
   tgg atg atg cta ctc ata tcc caa gcg gag gcg                         1089
   Trp Met Met Leu Leu Ile Ser Gln Ala Glu Ala
           355                 360
<210> 6
   <211> 363
   <212> PRT
   <213> Hepatitis C virus
<400> 6
   Glu Thr Tyr Thr Thr Gly Gly Ser Ile Ala Lys Thr Val Gln Gly Phe
   1               5                   10                  15
   Thr Ser Phe Phe Thr Pro Gly Ala Lys Gln Asp Ile Gln Leu Ile Asn
               20                  25                  30
   Thr Asn Gly Ser Trp His Ile Asn Arg Thr Ala Leu Asn Cys Asn Ala
           35                  40                  45
   Ser Leu Glu Thr Gly Trp Ile Ala Gly Leu Phe Tyr Tyr Asn Lys Phe
       50                  55                  60
   Asn Ser Ser Gly Cys Pro Glu Arg Met Ala Ser Cys Lys Pro Leu Ala
   65                  70                  75                  80
   Tyr Phe Ala Gln Gly Trp Gly Pro Ile Ser His Val Asn Gly Ser Gly
                   85                  90                  95
   Pro Glu Gln Arg Pro Tyr Cys Trp His Tyr Ala Pro Arg Pro Cys Gly
               100                 105                 110
   Ile Val Ser Ala Gln Thr Val Cys Gly Pro Val Tyr Cys Phe Thr Pro
           115                 120                 125
   Ser Pro Val Val Val Gly Thr Thr Asp Lys Leu Gly Ala Pro Thr Tyr
       130                 135                 140
   Asn Trp Gly Glu Asn Asp Thr Asp Val Phe Val Leu Asn Asn Thr Arg
   145                 150                 155                 160
   Pro Pro Leu Gly Asn Trp Phe Gly Cys Thr Trp Met Asn Ser Ser Gly
                   165                 170                 175
   Phe Thr Lys Val Cys Gly Ala Pro Pro Cys Ala Ile Gly Gly Val Gly
               180                 185                 190
   Asn Asn Thr Leu Arg Cys Pro Thr Asp Cys Phe Arg Lys His Pro Glu
           195                 200                 205
   Ala Thr Tyr Ser Arg Cys Gly Ser Gly Pro Trp Ile Thr Pro Arg Cys
       210                 215                 220
   Leu Val Asp Tyr Pro Tyr Arg Leu Trp His Tyr Pro Cys Thr Val Asn
   225                 230                 235                 240
   Tyr Thr Leu Phe Lys Ile Arg Met Tyr Val Gly Gly Val Glu His Arg
                   245                 250                 255
   Leu Gln Ala Ala Cys Asn Trp Thr Arg Gly Glu Arg Cys Asp Leu Asp
               260                 265                 270
   Asp Arg Asp Arg Ser Glu Leu Ser Pro Leu Leu Leu Ser Thr Thr Gln
           275                 280                 285
   Trp Gln Val Leu Pro Cys Ser Phe Thr Thr Leu Pro Ala Leu Thr Thr
       290                 295                 300
   Gly Leu Ile His Leu His Gln Asn Ile Val Asp Val Gln Tyr Leu Tyr
   305                 310                 315                 320
   Gly Val Gly Ser Ser Ile Val Ser Trp Ala Ile Lys Trp Glu Tyr Val
                   325                 330                 335
   Ile Leu Leu Phe Leu Leu Leu Ala Asp Ala Arg Ile Cys Ser Cys Leu
               340                 345                 350
   Trp Met Met Leu Leu Ile Ser Gln Ala Glu Ala
           355                 360
<210> 7
   <211> 1182
   <212> DNA
   <213> Artificial Sequence
<220>
   <223> dérivée de HCV et HBV
<220>
   <221> CDS
   <222> (1)..(1182)
<400> 7
   tac caa gta cgc aac tcc tcg ggc ctt tac cat gtc acc aat gat tgc      48
   Tyr Gln Val Arg Asn Ser Ser Gly Leu Tyr His Val Thr Asn Asp Cys
   1               5                   10                  15
   cct aac tcg agt att gtg tac gag acg gcc gat acc atc cta cac tct      96
   Pro Asn Ser Ser Ile Val Tyr Glu Thr Ala Asp Thr Ile Leu His Ser
               20                  25                  30
   ccg ggg tgt gtc cct tgc gtt cgc gag ggc aat gcc tca aaa tgt tgg     144
   Pro Gly Cys Val Pro Cys Val Arg Glu Gly Asn Ala Ser Lys Cys Trp
           35                  40                  45
   gtg gcg gtg gcc cct aca gtc gcc acc aga gac ggc aag ctc ccc aca     192
   Val Ala Val Ala Pro Thr Val Ala Thr Arg Asp Gly Lys Leu Pro Thr
       50                  55                  60
   acg cag ctt cga cgt cac atc gat ctg ctc gtc agg agc gcc acc ctc     240
   Thr Gln Leu Arg Arg His Ile Asp Leu Leu Val Arg Ser Ala Thr Leu
   65                  70                  75                  80
   tgc tcg gcc ctc tat gtg ggg gac ttg tgc ggg tcc gtc ttc ctc gtc      288
   Cys Ser Ala Leu Tyr Val Gly Asp Leu Cys Gly Ser Val Phe Leu Val
                   85                  90                  95
   ggt caa ctg ttc acc ttc tcc ccc agg cgc cac tgg aca acg caa gac      336
   Gly Gln Leu Phe Thr Phe Ser Pro Arg Arg His Trp Thr Thr Gln Asp
               100                 105                 110
   tgc aac tgt tcc atg tac ccc ggc cat ata acg ggt cac cgt atg gca      384
   Cys Asn Cys Ser Met Tyr Pro Gly His Ile Thr Gly His Arg Met Ala
           115                 120                 125
   tgg gac atg atg atg aac tgg tcc cct acg aca gcg ctg gta gta gct      432
   Trp Asp Met Met Met Asn Trp Ser Pro Thr Thr Ala Leu Val Val Ala
       130                 135                 140
   cag ctg ctc agg gtc ccg caa gcc atc ttg gac atg atc gct ggt gcc      480
   Gln Leu Leu Arg Val Pro Gln Ala Ile Leu Asp Met Ile Ala Gly Ala
   145                 150                 155                 160
   cac tgg gga gtc cta gcg ggc ata gcg tat ttc tcc atg gtg ggg aac      528
   His Trp Gly Val Leu Ala Gly Ile Ala Tyr Phe Ser Met Val Gly Asn
                   165                 170                 175
   tgg gcg aag gtc ctg gtg gtg ctg ttg ctg ttc gcc agc aca aga atc      576
   Trp Ala Lys Val Leu Val Val Leu Leu Leu Phe Ala Ser Thr Arg Ile
               180                 185                 190
   ctc aca ata ccg cag agt cta gac tcg tgg tgg act tct ctc aat ttt      624
   Leu Thr Ile Pro Gln Ser Leu Asp Ser Trp Trp Thr Ser Leu Asn Phe
           195                 200                 205
   cta ggg gga tct ccc gtg tgt ctt ggc caa aat tcg cag tcc cca acc      672
   Leu Gly Gly Ser Pro Val Cys Leu Gly Gln Asn Ser Gln Ser Pro Thr
       210                 215                 220
   tcc aat cac tca cca acc tcc tgt cct cca att tgt cct ggt tat cgc      720
   Ser Asn His Ser Pro Thr Ser Cys Pro Pro Ile Cys Pro Gly Tyr Arg
   225                 230                 235                 240
   tgg atg tgt ctg cgg cgt ttt atc ata ttc ctc ttc atc ctg ctg cta      768
   Trp Met Cys Leu Arg Arg Phe Ile Ile Phe Leu Phe Ile Leu Leu Leu
                   245                 250                 255
   tgc ctc atc ttc tta ttg gtt ctt ctg gat tat caa ggt atg ttg ccc      816
   Cys Leu Ile Phe Leu Leu Val Leu Leu Asp Tyr Gln Gly Met Leu Pro
               260                 265                 270
   gtt tgt cct cta att cca gga tca aca aca acc agt acg gga cca tgc      864
   Val Cys Pro Leu Ile Pro Gly Ser Thr Thr Thr Ser Thr Gly Pro Cys
           275                 280                 285
   aaa acc tgc acg act cct gct caa ggc aac tct atg ttt ccc tca tgt      912
   Lys Thr Cys Thr Thr Pro Ala Gln Gly Asn Ser Met Phe Pro Ser Cys
       290                 295                 300
   tgc tgt aca aaa cct acg gat gga aat tgc acc tgt att ccc atc cca      960
   Cys Cys Thr Lys Pro Thr Asp Gly Asn Cys Thr Cys Ile Pro Ile Pro
   305                 310                 315                     320
   tcg tcc tgg gct ttc gca aaa tac cta tgg gag tgg gcc tca gtc cgt      1008
   Ser Ser Trp Ala Phe Ala Lys Tyr Leu Trp Glu Trp Ala Ser Val Arg
                   325                     330                 335
   ttc tct tgg ctc agt tta cta gtg cca ttt gtt cag tgg ttc gta ggg      1056
   Phe Ser Trp Leu Ser Leu Leu Val Pro Phe Val Gln Trp Phe Val Gly
               340                 345                 350
   ctt tcc ccc act gtt tgg ctt tca gct ata tgg atg atg tgg tat tgg      1104
   Leu Ser Pro Thr Val Trp Leu Ser Ala Ile Trp Met Met Trp Tyr Trp
           355                 360                 365
   ggg cca agt ctg tac agc atc gtg agt ccc ttt ata ccg ctg tta cca      1152
   Gly Pro Ser Leu Tyr Ser Ile Val Ser Pro Phe Ile Pro Leu Leu Pro
       370                 375                 380
   att ttc ttt tgt ctc tgg gta tac att taa                              1182
   Ile Phe Phe Cys Leu Trp Val Tyr Ile
   385                 390
<210> 8
   <211> 393
   <212> PRT
   <213> Artificial Sequence
<220>
   <223> Synthetic Construct
<400> 8
   Tyr Gln Val Arg Asn Ser Ser Gly Leu Tyr His Val Thr Asn Asp Cys
   1               5                   10                  15
   Pro Asn Ser Ser Ile Val Tyr Glu Thr Ala Asp Thr Ile Leu His Ser
               20                  25                  30
   Pro Gly Cys Val Pro Cys Val Arg Glu Gly Asn Ala Ser Lys Cys Trp
           35                  40                  45
   Val Ala Val Ala Pro Thr Val Ala Thr Arg Asp Gly Lys Leu Pro Thr
       50                  55                  60
   Thr Gln Leu Arg Arg His Ile Asp Leu Leu Val Arg Ser Ala Thr Leu
   65                  70                  75                  80
   Cys Ser Ala Leu Tyr Val Gly Asp Leu Cys Gly Ser Val Phe Leu Val
                   85                  90                  95
   Gly Gln Leu Phe Thr Phe Ser Pro Arg Arg His Trp Thr Thr Gln Asp
               100                 105                 110
   Cys Asn Cys Ser Met Tyr Pro Gly His Ile Thr Gly His Arg Met Ala
   115 120 125
   Trp Asp Met Met Met Asn Trp Ser Pro Thr Thr Ala Leu Val Val Ala
   130 135 140
   Gln Leu Leu Arg Val Pro Gln Ala Ile Leu Asp Met Ile Ala Gly Ala
   145 150 155 160
   His Trp Gly Val Leu Ala Gly Ile Ala Tyr Phe Ser Met Val Gly Asn
   165 170 175
   Trp Ala Lys Val Leu Val Val Leu Leu Leu Phe Ala Ser Thr Arg Ile
   180 185 190
   Leu Thr Ile Pro Gln Ser Leu Asp Ser Trp Trp Thr Ser Leu Asn Phe
   195 200 205
   Leu Gly Gly Ser Pro Val Cys Leu Gly Gln Asn Ser Gln Ser Pro Thr
   210 215 220
   Ser Asn His Ser Pro Thr Ser Cys Pro Pro Ile Cys Pro Gly Tyr Arg
   225 230 235 240
   Trp Met Cys Leu Arg Arg Phe Ile Ile Phe Leu Phe Ile Leu Leu Leu
   245 250 255
   Cys Leu Ile Phe Leu Leu Val Leu Leu Asp Tyr Gln Gly Met Leu Pro
   260 265 270
   Val Cys Pro Leu Ile Pro Gly Ser Thr Thr Thr Ser Thr Gly Pro Cys
   275 280 285
   Lys Thr Cys Thr Thr Pro Ala Gln Gly Asn Ser Met Phe Pro Ser Cys
   290 295 300
   Cys Cys Thr Lys Pro Thr Asp Gly Asn Cys Thr Cys Ile Pro Ile Pro
   305 310 315 320
   Ser Ser Trp Ala Phe Ala Lys Tyr Leu Trp Glu Trp Ala Ser Val Arg
   325 330 335
   Phe Ser Trp Leu Ser Leu Leu Val Pro Phe Val Gln Trp Phe Val Gly
   340 345 350
   Leu Ser Pro Thr Val Trp Leu Ser Ala Ile Trp Met Met Trp Tyr Trp
               355                 360                 365
   Gly Pro Ser Leu Tyr Ser Ile Val Ser Pro Phe Ile Pro Leu Leu Pro
       370                 375                 380
   Ile Phe Phe Cys Leu Trp Val Tyr Ile
   385                 390
<210> 9
   <211> 1695
   <212> DNA
   <213> Artificial Sequence
<220>
   <223> dérivée de HCV et HBV
<220>
   <221> CDS
   <222> (1)..(1695)
<400> 9
   gaa acc tac acc acc ggg ggg agt att gcc aaa acc gtg caa gga ttc      48
   Glu Thr Tyr Thr Thr Gly Gly Ser Ile Ala Lys Thr Val Gln Gly Phe
   1               5                   10                  15
   acc agt ttt ttt acc cca ggc gcc aag cag gac atc cag ctg atc aac      96
   Thr Ser Phe Phe Thr Pro Gly Ala Lys Gln Asp Ile Gln Leu Ile Asn
               20                  25                  30
   acc aac ggc agt tgg cac atc aat cgc acg gcc ttg aac tgt aat gcg     144
   Thr Asn Gly Ser Trp His Ile Asn Arg Thr Ala Leu Asn Cys Asn Ala
           35                  40                  45
   agc ctc gaa acc ggc tgg ata gcg ggg ctc ttc tac tac aac aaa ttc     192
   Ser Leu Glu Thr Gly Trp Ile Ala Gly Leu Phe Tyr Tyr Asn Lys Phe
       50                  55                  60
   aac tcc tca ggc tgc ccc gag agg atg gcc agc tgc aaa ccc ctt gcc     240
   Asn Ser Ser Gly Cys Pro Glu Arg Met Ala Ser Cys Lys Pro Leu Ala
   65                  70                  75                  80
   tat ttc gcc caa ggc tgg ggc cct atc agc cat gtc aac gga agc ggc     288
   Tyr Phe Ala Gln Gly Trp Gly Pro Ile Ser His Val Asn Gly Ser Gly
                   85                  90                  95
   ccc gaa cag cgc ccc tac tgc tgg cac tac gcc cca agg cct tgt ggt     336
   Pro Glu Gln Arg Pro Tyr Cys Trp His Tyr Ala Pro Arg Pro Cys Gly
               100                 105                 110
   atc gtg tca gca cag aca gta tgt ggc cca gtg tat tgt ttc act cct     384
   Ile Val Ser Ala Gln Thr Val Cys Gly Pro Val Tyr Cys Phe Thr Pro
           115                 120                 125
   agc ccc gtg gtg gtg ggg acg acc gac aag ttg ggc gcg cct acc tac     432
   Ser Pro Val Val Val Gly Thr Thr Asp Lys Leu Gly Ala Pro Thr Tyr
       130                 135                 140
   aac tgg ggt gag aat gat acg gac gtc ttc gtc ctc aat aac acc agg      480
   Asn Trp Gly Glu Asn Asp Thr Asp Val Phe Val Leu Asn Asn Thr Arg
   145                 150                 155                 160
   cca ccg ttg ggc aat tgg ttc ggt tgc acc tgg atg aac tca tct gga      528
   Pro Pro Leu Gly Asn Trp Phe Gly Cys Thr Trp Met Asn Ser Ser Gly
                   165                 170                 175
   ttc acc aaa gtg tgc gga gcg cct ccc tgt gcc atc gga gga gtg ggc      576
   Phe Thr Lys Val Cys Gly Ala Pro Pro Cys Ala Ile Gly Gly Val Gly
               180                 185                 190
   aat aac acc ttg cgc tgt ccc act gac tgt ttc cgc aag cat ccg gaa      624
   Asn Asn Thr Leu Arg Cys Pro Thr Asp Cys Phe Arg Lys His Pro Glu
           195                 200                 205
   gct aca tac tct cga tgt ggc tcc ggt ccc tgg atc acg ccc agg tgc      672
   Ala Thr Tyr Ser Arg Cys Gly Ser Gly Pro Trp Ile Thr Pro Arg Cys
       210                 215                 220
   ctg gtc gac tat cct tat agg ctc tgg cat tat cct tgc act gtc aac      720
   Leu Val Asp Tyr Pro Tyr Arg Leu Trp His Tyr Pro Cys Thr Val Asn
   225                 230                 235                 240
   tac acc ctg ttt aaa atc agg atg tac gtg gga ggg gtc gag cac agg      768
   Tyr Thr Leu Phe Lys Ile Arg Met Tyr Val Gly Gly Val Glu His Arg
                   245                 250                 255
   cta caa gct gct tgc aac tgg acg cgg ggc gag cgt tgt gat ctg gac      816
   Leu Gln Ala Ala Cys Asn Trp Thr Arg Gly Glu Arg Cys Asp Leu Asp
               260                 265                 270
   gac agg gac agg tcc gag ctc agc ccg ctg ctg ctg tcc acc acg cag      864
   Asp Arg Asp Arg Ser Glu Leu Ser Pro Leu Leu Leu Ser Thr Thr Gln
           275                 280                 285
   tgg cag gtc ctt ccg tgt tct ttc acg acc ttg cca gcc ttg acc acc      912
   Trp Gln Val Leu Pro Cys Ser Phe Thr Thr Leu Pro Ala Leu Thr Thr
       290                 295                 300
   ggc ctc atc cac ctc cat cag aac atc gtg gac gtg caa tat ttg tac      960
   Gly Leu Ile His Leu His Gln Asn Ile Val Asp Val Gln Tyr Leu Tyr
   305                 310                 315                 320
   ggg gtg ggg tca agc att gtg tcc tgg gcc atc aag tgg gag tac gtc     1008
   Gly Val Gly Ser Ser Ile Val Ser Trp Ala Ile Lys Trp Glu Tyr Val
                   325                 330                 335
   att ctc ttg ttt ctc ctg ctt gca gac gcg cgc atc tgc tcc tgc ttg     1056
   Ile Leu Leu Phe Leu Leu Leu Ala Asp Ala Arg Ile Cys Ser Cys Leu
               340                 345                 350
   tgg atg atg cta ctc ata tcc caa aca aga atc ctc aca ata ccg cag     1104
   Trp Met Met Leu Leu Ile Ser Gln Thr Arg Ile Leu Thr Ile Pro Gln
           355                 360                 365
   agt cta gac tcg tgg tgg act tct ctc aat ttt cta ggg gga tct ccc     1152
   Ser Leu Asp Ser Trp Trp Thr Ser Leu Asn Phe Leu Gly Gly Ser Pro
       370                 375                 380
   gtg tgt ctt ggc caa aat tcg cag tcc cca acc tcc aat cac tca cca     1200
   Val Cys Leu Gly Gln Asn Ser Gln Ser Pro Thr Ser Asn His Ser Pro
   385                 390                 395                 400
   acc tcc tgt cct cca att tgt cct ggt tat cgc tgg atg tgt ctg cgg      1248
   Thr Ser Cys Pro Pro Ile Cys Pro Gly Tyr Arg Trp Met Cys Leu Arg
                   405                 410                 415
   cgt ttt atc ata ttc ctc ttc atc ctg ctg cta tgc ctc atc ttc tta      1296
   Arg Phe Ile Ile Phe Leu Phe Ile Leu Leu Leu Cys Leu Ile Phe Leu
               420                 425                 430
   ttg gtt ctt ctg gat tat caa ggt atg ttg ccc gtt tgt cct cta att      1344
   Leu Val Leu Leu Asp Tyr Gln Gly Met Leu Pro Val Cys Pro Leu Ile
           435                 440                 445
   cca gga tca aca aca acc agt acg gga cca tgc aaa acc tgc acg act      1392
   Pro Gly Ser Thr Thr Thr Ser Thr Gly Pro Cys Lys Thr Cys Thr Thr
       450                 455                 460
   cct gct caa ggc aac tct atg ttt ccc tca tgt tgc tgt aca aaa cct      1440
   Pro Ala Gln Gly Asn Ser Met Phe Pro Ser Cys Cys Cys Thr Lys Pro
   465                 470                 475                 480
   acg gat gga aat tgc acc tgt att ccc atc cca tcg tcc tgg gct ttc      1488
   Thr Asp Gly Asn Cys Thr Cys Ile Pro Ile Pro Ser Ser Trp Ala Phe
                   485                 490                 495
   gca aaa tac cta tgg gag tgg gcc tca gtc cgt ttc tct tgg ctc agt      1536
   Ala Lys Tyr Leu Trp Glu Trp Ala Ser Val Arg Phe Ser Trp Leu Ser
               500                 505                 510
   tta cta gtg cca ttt gtt cag tgg ttc gta ggg ctt tcc ccc act gtt      1584
   Leu Leu Val Pro Phe Val Gln Trp Phe Val Gly Leu Ser Pro Thr Val
           515                 520                 525
   tgg ctt tca gct ata tgg atg atg tgg tat tgg ggg cca agt ctg tac      1632
   Trp Leu Ser Ala Ile Trp Met Met Trp Tyr Trp Gly Pro Ser Leu Tyr
       530                 535                 540
   agc atc gtg agt ccc ttt ata ccg ctg tta cca att ttc ttt tgt ctc      1680
   Ser Ile Val Ser Pro Phe Ile Pro Leu Leu Pro Ile Phe Phe Cys Leu
   545                 550                 555                 560
   tgg gta tac att taa                                                  1695
   Trp Val Tyr Ile
<210> 10
   <211> 564
   <212> PRT
   <213> Artificial Sequence
<220>
   <223> Synthetic Construct
<400> 10
   Glu Thr Tyr Thr Thr Gly Gly Ser Ile Ala Lys Thr Val Gln Gly Phe
   1               5                   10                  15
   Thr Ser Phe Phe Thr Pro Gly Ala Lys Gln Asp Ile Gln Leu Ile Asn
   20 25 30
   Thr Asn Gly Ser Trp His Ile Asn Arg Thr Ala Leu Asn Cys Asn Ala
   35 40 45
   Ser Leu Glu Thr Gly Trp Ile Ala Gly Leu Phe Tyr Tyr Asn Lys Phe
   50 55 60
   Asn Ser Ser Gly Cys Pro Glu Arg Met Ala Ser Cys Lys Pro Leu Ala
   65 70 75 80
   Tyr Phe Ala Gln Gly Trp Gly Pro Ile Ser His Val Asn Gly Ser Gly
   85 90 95
   Pro Glu Gln Arg Pro Tyr Cys Trp His Tyr Ala Pro Arg Pro Cys Gly
   100 105 110
   Ile Val Ser Ala Gln Thr Val Cys Gly Pro Val Tyr Cys Phe Thr Pro
   115 120 125
   Ser Pro Val Val Val Gly Thr Thr Asp Lys Leu Gly Ala Pro Thr Tyr
   130 135 140
   Asn Trp Gly Glu Asn Asp Thr Asp Val Phe Val Leu Asn Asn Thr Arg
   145 150 155 160
   Pro Pro Leu Gly Asn Trp Phe Gly Cys Thr Trp Met Asn Ser Ser Gly
   165 170 175
   Phe Thr Lys Val Cys Gly Ala Pro Pro Cys Ala Ile Gly Gly Val Gly
   180 185 190
   Asn Asn Thr Leu Arg Cys Pro Thr Asp Cys Phe Arg Lys His Pro Glu
   195 200 205
   Ala Thr Tyr Ser Arg Cys Gly Ser Gly Pro Trp Ile Thr Pro Arg Cys
   210 215 220
   Leu Val Asp Tyr Pro Tyr Arg Leu Trp His Tyr Pro Cys Thr Val Asn
   225 230 235 240
   Tyr Thr Leu Phe Lys Ile Arg Met Tyr Val Gly Gly Val Glu His Arg
   245 250 255
   Leu Gln Ala Ala Cys Asn Trp Thr Arg Gly Glu Arg Cys Asp Leu Asp
   260 265 270
   Asp Arg Asp Arg Ser Glu Leu Ser Pro Leu Leu Leu Ser Thr Thr Gln
   275 280 285
   Trp Gln Val Leu Pro Cys Ser Phe Thr Thr Leu Pro Ala Leu Thr Thr
   290 295 300
   Gly Leu Ile His Leu His Gln Asn Ile Val Asp Val Gln Tyr Leu Tyr
   305 310 315 320
   Gly Val Gly Ser Ser Ile Val Ser Trp Ala Ile Lys Trp Glu Tyr Val
   325 330 335
   Ile Leu Leu Phe Leu Leu Leu Ala Asp Ala Arg Ile Cys Ser Cys Leu
   340 345 350
   Trp Met Met Leu Leu Ile Ser Gln Thr Arg Ile Leu Thr Ile Pro Gln
   355 360 365
   Ser Leu Asp Ser Trp Trp Thr Ser Leu Asn Phe Leu Gly Gly Ser Pro
   370 375 380
   Val Cys Leu Gly Gln Asn Ser Gln Ser Pro Thr Ser Asn His Ser Pro
   385 390 395 400
   Thr Ser Cys Pro Pro Ile Cys Pro Gly Tyr Arg Trp Met Cys Leu Arg
   405 410 415
   Arg Phe Ile Ile Phe Leu Phe Ile Leu Leu Leu Cys Leu Ile Phe Leu
   420 425 430
   Leu Val Leu Leu Asp Tyr Gln Gly Met Leu Pro Val Cys Pro Leu Ile
   435 440 445
   Pro Gly Ser Thr Thr Thr Ser Thr Gly Pro Cys Lys Thr Cys Thr Thr
   450 455 460
   Pro Ala Gln Gly Asn Ser Met Phe Pro Ser Cys Cys Cys Thr Lys Pro
   465 470 475 480
   Thr Asp Gly Asn Cys Thr Cys Ile Pro Ile Pro Ser Ser Trp Ala Phe
   485 490 495
   Ala Lys Tyr Leu Trp Glu Trp Ala Ser Val Arg Phe Ser Trp Leu Ser
   500 505 510
   Leu Leu Val Pro Phe Val Gln Trp Phe Val Gly Leu Ser Pro Thr Val
   515 520 525
   Trp Leu Ser Ala Ile Trp Met Met Trp Tyr Trp Gly Pro Ser Leu Tyr
   530 535 540
   Ser Ile Val Ser Pro Phe Ile Pro Leu Leu Pro Ile Phe Phe Cys Leu
   545 550 555 560
   Trp Val Tyr Ile
<210> 11
   <211> 1263
   <212> DNA
   <213> Artificial Sequence
<220>
   <223> dérivée de HCV et HBV
<220>
   <221> CDS
   <222> (1)..(1263)
<400> 11
   atg aca ggg aac ctt cct ggt tgc tct ttc tct atc ttc ctt ctg gcc      48
   Met Thr Gly Asn Leu Pro Gly Cys Ser Phe Ser Ile Phe Leu Leu Ala
   1               5                   10                  15
   ctg ctc tct tgc ctg act gtg ccc gcg tca gcc tac caa gta cgc aac      96
   Leu Leu Ser Cys Leu Thr Val Pro Ala Ser Ala Tyr Gln Val Arg Asn
               20                  25                  30
   tcc tcg ggc ctt tac cat gtc acc aat gat tgc cct aac tcg agt att     144
   Ser Ser Gly Leu Tyr His Val Thr Asn Asp Cys Pro Asn Ser Ser Ile
           35                  40                  45
   gtg tac gag acg gcc gat acc atc cta cac tct ccg ggg tgt gtc cct     192
   Val Tyr Glu Thr Ala Asp Thr Ile Leu His Ser Pro Gly Cys Val Pro
       50                  55                  60
   tgc gtt cgc gag ggc aat gcc tca aaa tgt tgg gtg gcg gtg gcc cct     240
   Cys Val Arg Glu Gly Asn Ala Ser Lys Cys Trp Val Ala Val Ala Pro
   65                  70                  75                  80
   aca gtc gcc acc aga gac ggc aag ctc ccc aca acg cag ctt cga cgt     288
   Thr Val Ala Thr Arg Asp Gly Lys Leu Pro Thr Thr Gln Leu Arg Arg
                   85                  90                  95
   cac atc gat ctg ctc gtc agg agc gcc acc ctc tgc tcg gcc ctc tat     336
   His Ile Asp Leu Leu Val Arg Ser Ala Thr Leu Cys Ser Ala Leu Tyr
               100                 105                 110
   gtg ggg gac ttg tgc ggg tcc gtc ttc ctc gtc ggt caa ctg ttc acc      384
   Val Gly Asp Leu Cys Gly Ser Val Phe Leu Val Gly Gln Leu Phe Thr
           115                 120                 125
   ttc tcc ccc agg cgc cac tgg aca acg caa gac tgc aac tgt tcc atg      432
   Phe Ser Pro Arg Arg His Trp Thr Thr Gln Asp Cys Asn Cys Ser Met
       130                 135                 140
   tac ccc ggc cat ata acg ggt cac cgt atg gca tgg gac atg atg atg      480
   Tyr Pro Gly His Ile Thr Gly His Arg Met Ala Trp Asp Met Met Met
   145                 150                 155                 160
   aac tgg tcc cct acg aca gcg ctg gta gta gct cag ctg ctc agg gtc      528
   Asn Trp Ser Pro Thr Thr Ala Leu Val Val Ala Gln Leu Leu Arg Val
                   165                 170                 175
   ccg caa gcc atc ttg gac atg atc gct ggt gcc cac tgg gga gtc cta      576
   Pro Gln Ala Ile Leu Asp Met Ile Ala Gly Ala His Trp Gly Val Leu
               180                 185                 190
   gcg ggc ata gcg tat ttc tcc atg gtg ggg aac tgg gcg aag gtc ctg      624
   Ala Gly Ile Ala Tyr Phe Ser Met Val Gly Asn Trp Ala Lys Val Leu
           195                 200                 205
   gtg gtg ctg ttg ctg ttc gcc agc aca aga atc ctc aca ata ccg cag      672
   Val Val Leu Leu Leu Phe Ala Ser Thr Arg Ile Leu Thr Ile Pro Gln
       210                 215                 220
   agt cta gac tcg tgg tgg act tct ctc aat ttt cta ggg gga tct ccc      720
   Ser Leu Asp Ser Trp Trp Thr Ser Leu Asn Phe Leu Gly Gly Ser Pro
   225                 230                 235                 240
   gtg tgt ctt ggc caa aat tcg cag tcc cca acc tcc aat cac tca cca      768
   Val Cys Leu Gly Gln Asn Ser Gln Ser Pro Thr Ser Asn His Ser Pro
                   245                 250                 255
   acc tcc tgt cct cca att tgt cct ggt tat cgc tgg atg tgt ctg cgg      816
   Thr Ser Cys Pro Pro Ile Cys Pro Gly Tyr Arg Trp Met Cys Leu Arg
               260                 265                 270
   cgt ttt atc ata ttc ctc ttc atc ctg ctg cta tgc ctc atc ttc tta      864
   Arg Phe Ile Ile Phe Leu Phe Ile Leu Leu Leu Cys Leu Ile Phe Leu
           275                 280                 285
   ttg gtt ctt ctg gat tat caa ggt atg ttg ccc gtt tgt cct cta att      912
   Leu Val Leu Leu Asp Tyr Gln Gly Met Leu Pro Val Cys Pro Leu Ile
       290                 295                 300
   cca gga tca aca aca acc agt acg gga cca tgc aaa acc tgc acg act      960
   Pro Gly Ser Thr Thr Thr Ser Thr Gly Pro Cys Lys Thr Cys Thr Thr
   305                 310                 315                 320
   cct gct caa ggc aac tct atg ttt ccc tca tgt tgc tgt aca aaa cct     1008
   Pro Ala Gln Gly Asn Ser Met Phe Pro Ser Cys Cys Cys Thr Lys Pro
                   325                 330                 335
   acg gat gga aat tgc acc tgt att ccc atc cca tcg tcc tgg gct ttc     1056
   Thr Asp Gly Asn Cys Thr Cys Ile Pro Ile Pro Ser Ser Trp Ala Phe
               340                 345                 350
   gca aaa tac cta tgg gag tgg gcc tca gtc cgt ttc tct tgg ctc agt     1104
   Ala Lys Tyr Leu Trp Glu Trp Ala Ser Val Arg Phe Ser Trp Leu Ser
   355                     360                 365
   tta cta gtg cca ttt gtt cag tgg ttc gta ggg ctt tcc ccc act gtt      1152
   Leu Leu Val Pro Phe Val Gln Trp Phe Val Gly Leu Ser Pro Thr Val
       370                 375                 380
   tgg ctt tca gct ata tgg atg atg tgg tat tgg ggg cca agt ctg tac      1200
   Trp Leu Ser Ala Ile Trp Met Met Trp Tyr Trp Gly Pro Ser Leu Tyr
   385                 390                 395                 400
   agc atc gtg agt ccc ttt ata ccg ctg tta cca att ttc ttt tgt ctc      1248
   Ser Ile Val Ser Pro Phe Ile Pro Leu Leu Pro Ile Phe Phe Cys Leu
                   405                 410                 415
   tgg gta tac att taa                                                  1263
   Trp Val Tyr Ile
               420
<210> 12
   <211> 420
   <212> PRT
   <213> Artificial Sequence
<220>
   <223> Synthetic Construct
<400> 12
   Met Thr Gly Asn Leu Pro Gly Cys Ser Phe Ser Ile Phe Leu Leu Ala
   1               5                   10                  15
   Leu Leu Ser Cys Leu Thr Val Pro Ala Ser Ala Tyr Gln Val Arg Asn
               20                  25                  30
   Ser Ser Gly Leu Tyr His Val Thr Asn Asp Cys Pro Asn Ser Ser Ile
           35                  40                  45
   Val Tyr Glu Thr Ala Asp Thr Ile Leu His Ser Pro Gly Cys Val Pro
       50                  55                  60
   Cys Val Arg Glu Gly Asn Ala Ser Lys Cys Trp Val Ala Val Ala Pro
   65                  70                  75                  80
   Thr Val Ala Thr Arg Asp Gly Lys Leu Pro Thr Thr Gln Leu Arg Arg
                   85                  90                  95
   His Ile Asp Leu Leu Val Arg Ser Ala Thr Leu Cys Ser Ala Leu Tyr
               100                 105                 110
   Val Gly Asp Leu Cys Gly Ser Val Phe Leu Val Gly Gln Leu Phe Thr
           115                 120                 125
   Phe Ser Pro Arg Arg His Trp Thr Thr Gln Asp Cys Asn Cys Ser Met
       130                 135                 140
   Tyr Pro Gly His Ile Thr Gly His Arg Met Ala Trp Asp Met Met Met
   145                 150                 155                 160
   Asn Trp Ser Pro Thr Thr Ala Leu Val Val Ala Gln Leu Leu Arg Val
                   165                 170                 175
   Pro Gln Ala Ile Leu Asp Met Ile Ala Gly Ala His Trp Gly Val Leu
               180                 185                 190
   Ala Gly Ile Ala Tyr Phe Ser Met Val Gly Asn Trp Ala Lys Val Leu
           195                 200                 205
   Val Val Leu Leu Leu Phe Ala Ser Thr Arg Ile Leu Thr Ile Pro Gln
       210                 215                 220
   Ser Leu Asp Ser Trp Trp Thr Ser Leu Asn Phe Leu Gly Gly Ser Pro
   225                 230                 235                 240
   Val Cys Leu Gly Gln Asn Ser Gln Ser Pro Thr Ser Asn His Ser Pro
                   245                 250                 255
   Thr Ser Cys Pro Pro Ile Cys Pro Gly Tyr Arg Trp Met Cys Leu Arg
               260                 265                 270
   Arg Phe Ile Ile Phe Leu Phe Ile Leu Leu Leu Cys Leu Ile Phe Leu
           275                 280                 285
   Leu Val Leu Leu Asp Tyr Gln Gly Met Leu Pro Val Cys Pro Leu Ile
       290                 295                 300
   Pro Gly Ser Thr Thr Thr Ser Thr Gly Pro Cys Lys Thr Cys Thr Thr
   305                 310                 315                 320
   Pro Ala Gln Gly Asn Ser Met Phe Pro Ser Cys Cys Cys Thr Lys Pro
                   325                 330                 335
   Thr Asp Gly Asn Cys Thr Cys Ile Pro Ile Pro Ser Ser Trp Ala Phe
               340                 345                 350
   Ala Lys Tyr Leu Trp Glu Trp Ala Ser Val Arg Phe Ser Trp Leu Ser
           355                 360                 365
   Leu Leu Val Pro Phe Val Gln Trp Phe Val Gly Leu Ser Pro Thr Val
       370                 375                 380
   Trp Leu Ser Ala Ile Trp Met Met Trp Tyr Trp Gly Pro Ser Leu Tyr
   385                 390                 395                 400
   Ser Ile Val Ser Pro Phe Ile Pro Leu Leu Pro Ile Phe Phe Cys Leu
                   405                 410                 415
   Trp Val Tyr Ile
               420
<210> 13
   <211> 1752
   <212> DNA
   <213> Artificial Sequence
<220>
   <223> dérivée de HCV et HBV
<220>
   <221> CDS
   <222> (1)..(1752)
<400> 13
   atg ggg aac tgg gcg aag gtc ctg gtg gtg ctg ttg ctg ttc gcc agc      48
   Met Gly Asn Trp Ala Lys Val Leu Val Val Leu Leu Leu Phe Ala Ser
   1               5                   10                  15
   gtc gat gcg gaa acc tac acc acc ggg ggg agt att gcc aaa acc gtg      96
   Val Asp Ala Glu Thr Tyr Thr Thr Gly Gly Ser Ile Ala Lys Thr Val
               20                  25                  30
   caa gga ttc acc agt ttt ttt acc cca ggc gcc aag cag gac atc cag     144
   Gln Gly Phe Thr Ser Phe Phe Thr Pro Gly Ala Lys Gln Asp Ile Gln
           35                  40                  45
   ctg atc aac acc aac ggc agt tgg cac atc aat cgc acg gcc ttg aac     192
   Leu Ile Asn Thr Asn Gly Ser Trp His Ile Asn Arg Thr Ala Leu Asn
       50                  55                  60
   tgt aat gcg agc ctc gaa acc ggc tgg ata gcg ggg ctc ttc tac tac     240
   Cys Asn Ala Ser Leu Glu Thr Gly Trp Ile Ala Gly Leu Phe Tyr Tyr
   65                  70                  75                  80
   aac aaa ttc aac tcc tca ggc tgc ccc gag agg atg gcc agc tgc aaa     288
   Asn Lys Phe Asn Ser Ser Gly Cys Pro Glu Arg Met Ala Ser Cys Lys
                   85                  90                  95
   ccc ctt gcc tat ttc gcc caa ggc tgg ggc cct atc agc cat gtc aac     336
   Pro Leu Ala Tyr Phe Ala Gln Gly Trp Gly Pro Ile Ser His Val Asn
               100                 105                 110
   gga agc ggc ccc gaa cag cgc ccc tac tgc tgg cac tac gcc cca agg     384
   Gly Ser Gly Pro Glu Gln Arg Pro Tyr Cys Trp His Tyr Ala Pro Arg
           115                 120                 125
   cct tgt ggt atc gtg tca gca cag aca gta tgt ggc cca gtg tat tgt      432
   Pro Cys Gly Ile Val Ser Ala Gln Thr Val Cys Gly Pro Val Tyr Cys
       130                 135                 140
   ttc act cct agc ccc gtg gtg gtg ggg acg acc gac aag ttg ggc gcg      480
   Phe Thr Pro Ser Pro Val Val Val Gly Thr Thr Asp Lys Leu Gly Ala
   145                 150                 155                 160
   cct acc tac aac tgg ggt gag aat gat acg gac gtc ttc gtc ctc aat      528
   Pro Thr Tyr Asn Trp Gly Glu Asn Asp Thr Asp Val Phe Val Leu Asn
                   165                 170                 175
   aac acc agg cca ccg ttg ggc aat tgg ttc ggt tgc acc tgg atg aac      576
   Asn Thr Arg Pro Pro Leu Gly Asn Trp Phe Gly Cys Thr Trp Met Asn
               180                 185                 190
   tca tct gga ttc acc aaa gtg tgc gga gcg cct ccc tgt gcc atc gga      624
   Ser Ser Gly Phe Thr Lys Val Cys Gly Ala Pro Pro Cys Ala Ile Gly
           195                 200                 205
   gga gtg ggc aat aac acc ttg cgc tgt ccc act gac tgt ttc cgc aag      672
   Gly Val Gly Asn Asn Thr Leu Arg Cys Pro Thr Asp Cys Phe Arg Lys
       210                 215                 220
   cat ccg gaa gct aca tac tct cga tgt ggc tcc ggt ccc tgg atc acg      720
   His Pro Glu Ala Thr Tyr Ser Arg Cys Gly Ser Gly Pro Trp Ile Thr
   225                 230                 235                 240
   ccc agg tgc ctg gtc gac tat cct tat agg ctc tgg cat tat cct tgc      768
   Pro Arg Cys Leu Val Asp Tyr Pro Tyr Arg Leu Trp His Tyr Pro Cys
                   245                 250                 255
   act gtc aac tac acc ctg ttt aaa atc agg atg tac gtg gga ggg gtc      816
   Thr Val Asn Tyr Thr Leu Phe Lys Ile Arg Met Tyr Val Gly Gly Val
               260                 265                 270
   gag cac agg cta caa gct gct tgc aac tgg acg cgg ggc gag cgt tgt      864
   Glu His Arg Leu Gln Ala Ala Cys Asn Trp Thr Arg Gly Glu Arg Cys
           275                 280                 285
   gat ctg gac gac agg gac agg tcc gag ctc agc ccg ctg ctg ctg tcc      912
   Asp Leu Asp Asp Arg Asp Arg Ser Glu Leu Ser Pro Leu Leu Leu Ser
       290                 295                 300
   acc acg cag tgg cag gtc ctt ccg tgt tct ttc acg acc ttg cca gcc      960
   Thr Thr Gln Trp Gln Val Leu Pro Cys Ser Phe Thr Thr Leu Pro Ala
   305                 310                 315                 320
   ttg acc acc ggc ctc atc cac ctc cat cag aac atc gtg gac gtg caa     1008
   Leu Thr Thr Gly Leu Ile His Leu His Gln Asn Ile Val Asp Val Gln
                   325                 330                 335
   tat ttg tac ggg gtg ggg tca agc att gtg tcc tgg gcc atc aag tgg     1056
   Tyr Leu Tyr Gly Val Gly Ser Ser Ile Val Ser Trp Ala Ile Lys Trp
               340                 345                 350
   gag tac gtc att ctc ttg ttt ctc ctg ctt gca gac gcg cgc atc tgc     1104
   Glu Tyr Val Ile Leu Leu Phe Leu Leu Leu Ala Asp Ala Arg Ile Cys
           355                 360                 365
   tcc tgc ttg tgg atg atg cta ctc ata tcc caa aca aga atc ctc aca      1152
   Ser Cys Leu Trp Met Met Leu Leu Ile Ser Gln Thr Arg Ile Leu Thr
       370                 375                 380
   ata ccg cag agt cta gac tcg tgg tgg act tct ctc aat ttt cta ggg      1200
   Ile Pro Gln Ser Leu Asp Ser Trp Trp Thr Ser Leu Asn Phe Leu Gly
   385                 390                 395                 400
   gga tct ccc gtg tgt ctt ggc caa aat tcg cag tcc cca acc tcc aat      1248
   Gly Ser Pro Val Cys Leu Gly Gln Asn Ser Gln Ser Pro Thr Ser Asn
                   405                 410                 415
   cac tca cca acc tcc tgt cct cca att tgt cct ggt tat cgc tgg atg      1296
   His Ser Pro Thr Ser Cys Pro Pro Ile Cys Pro Gly Tyr Arg Trp Met
               420                 425                 430
   tgt ctg cgg cgt ttt atc ata ttc ctc ttc atc ctg ctg cta tgc ctc      1344
   Cys Leu Arg Arg Phe Ile Ile Phe Leu Phe Ile Leu Leu Leu Cys Leu
           435                 440                 445
   atc ttc tta ttg gtt ctt ctg gat tat caa ggt atg ttg ccc gtt tgt      1392
   Ile Phe Leu Leu Val Leu Leu Asp Tyr Gln Gly Met Leu Pro Val Cys
       450                 455                 460
   cct cta att cca gga tca aca aca acc agt acg gga cca tgc aaa acc      1440
   Pro Leu Ile Pro Gly Ser Thr Thr Thr Ser Thr Gly Pro Cys Lys Thr
   465                 470                 475                 480
   tgc acg act cct gct caa ggc aac tct atg ttt ccc tca tgt tgc tgt      1488
   Cys Thr Thr Pro Ala Gln Gly Asn Ser Met Phe Pro Ser Cys Cys Cys
                   485                 490                 495
   aca aaa cct acg gat gga aat tgc acc tgt att ccc atc cca tcg tcc      1536
   Thr Lys Pro Thr Asp Gly Asn Cys Thr Cys Ile Pro Ile Pro Ser Ser
               500                 505                 510
   tgg gct ttc gca aaa tac cta tgg gag tgg gcc tca gtc cgt ttc tct      1584
   Trp Ala Phe Ala Lys Tyr Leu Trp Glu Trp Ala Ser Val Arg Phe Ser
           515                 520                 525
   tgg ctc agt tta cta gtg cca ttt gtt cag tgg ttc gta ggg ctt tcc      1632
   Trp Leu Ser Leu Leu Val Pro Phe Val Gln Trp Phe Val Gly Leu Ser
       530                 535                 540
   ccc act gtt tgg ctt tca gct ata tgg atg atg tgg tat tgg ggg cca      1680
   Pro Thr Val Trp Leu Ser Ala Ile Trp Met Met Trp Tyr Trp Gly Pro
   545                 550                 555                 560
   agt ctg tac agc atc gtg agt ccc ttt ata ccg ctg tta cca att ttc      1728
   Ser Leu Tyr Ser Ile Val Ser Pro Phe Ile Pro Leu Leu Pro Ile Phe
                   565                 570                 575
   ttt tgt ctc tgg gta tac att taa                                      1752
   Phe Cys Leu Trp Val Tyr Ile
               580
<210> 14
   <211> 583
   <212> PRT
   <213> Artificial Sequence
<220>
   <223> Synthetic Construct
<400> 14
   Met Gly Asn Trp Ala Lys Val Leu Val Val Leu Leu Leu Phe Ala Ser
   1               5                   10                  15
   Val Asp Ala Glu Thr Tyr Thr Thr Gly Gly Ser Ile Ala Lys Thr Val
               20                  25                  30
   Gln Gly Phe Thr Ser Phe Phe Thr Pro Gly Ala Lys Gln Asp Ile Gln
           35                  40                  45
   Leu Ile Asn Thr Asn Gly Ser Trp His Ile Asn Arg Thr Ala Leu Asn
       50                  55                  60
   Cys Asn Ala Ser Leu Glu Thr Gly Trp Ile Ala Gly Leu Phe Tyr Tyr
   65                  70                  75                  80
   Asn Lys Phe Asn Ser Ser Gly Cys Pro Glu Arg Met Ala Ser Cys Lys
                   85                  90                  95
   Pro Leu Ala Tyr Phe Ala Gln Gly Trp Gly Pro Ile Ser His Val Asn
               100                 105                 110
   Gly Ser Gly Pro Glu Gln Arg Pro Tyr Cys Trp His Tyr Ala Pro Arg
           115                 120                 125
   Pro Cys Gly Ile Val Ser Ala Gln Thr Val Cys Gly Pro Val Tyr Cys
       130                 135                 140
   Phe Thr Pro Ser Pro Val Val Val Gly Thr Thr Asp Lys Leu Gly Ala
   145                 150                 155                 160
   Pro Thr Tyr Asn Trp Gly Glu Asn Asp Thr Asp Val Phe Val Leu Asn
                   165                 170                 175
   Asn Thr Arg Pro Pro Leu Gly Asn Trp Phe Gly Cys Thr Trp Met Asn
               180                 185                 190
   Ser Ser Gly Phe Thr Lys Val Cys Gly Ala Pro Pro Cys Ala Ile Gly
           195                 200                 205
   Gly Val Gly Asn Asn Thr Leu Arg Cys Pro Thr Asp Cys Phe Arg Lys
       210                 215                 220
   His Pro Glu Ala Thr Tyr Ser Arg Cys Gly Ser Gly Pro Trp Ile Thr
   225 230 235 240
   Pro Arg Cys Leu Val Asp Tyr Pro Tyr Arg Leu Trp His Tyr Pro Cys
   245 250 255
   Thr Val Asn Tyr Thr Leu Phe Lys Ile Arg Met Tyr Val Gly Gly Val
   260 265 270
   Glu His Arg Leu Gln Ala Ala Cys Asn Trp Thr Arg Gly Glu Arg Cys
   275 280 285
   Asp Leu Asp Asp Arg Asp Arg Ser Glu Leu Ser Pro Leu Leu Leu Ser
   290 295 300
   Thr Thr Gln Trp Gln Val Leu Pro Cys Ser Phe Thr Thr Leu Pro Ala
   305 310 315 320
   Leu Thr Thr Gly Leu Ile His Leu His Gln Asn Ile Val Asp Val Gln
   325 330 335
   Tyr Leu Tyr Gly Val Gly Ser Ser Ile Val Ser Trp Ala Ile Lys Trp
   340 345 350
   Glu Tyr Val Ile Leu Leu Phe Leu Leu Leu Ala Asp Ala Arg Ile Cys
   355 360 365
   Ser Cys Leu Trp Met Met Leu Leu Ile Ser Gln Thr Arg Ile Leu Thr
   370 375 380
   Ile Pro Gln Ser Leu Asp Ser Trp Trp Thr Ser Leu Asn Phe Leu Gly
   385 390 395 400
   Gly Ser Pro Val Cys Leu Gly Gln Asn Ser Gln Ser Pro Thr Ser Asn
   405 410 415
   His Ser Pro Thr Ser Cys Pro Pro Ile Cys Pro Gly Tyr Arg Trp Met
   420 425 430
   Cys Leu Arg Arg Phe Ile Ile Phe Leu Phe Ile Leu Leu Leu Cys Leu
   435 440 445
   Ile Phe Leu Leu Val Leu Leu Asp Tyr Gln Gly Met Leu Pro Val Cys
   450 455 460
   Pro Leu Ile Pro Gly Ser Thr Thr Thr Ser Thr Gly Pro Cys Lys Thr
   465 470 475 480
   Cys Thr Thr Pro Ala Gln Gly Asn Ser Met Phe Pro Ser Cys Cys Cys
   485 490 495
   Thr Lys Pro Thr Asp Gly Asn Cys Thr Cys Ile Pro Ile Pro Ser Ser
   500 505 510
   Trp Ala Phe Ala Lys Tyr Leu Trp Glu Trp Ala Ser Val Arg Phe Ser
   515 520 525
   Trp Leu Ser Leu Leu Val Pro Phe Val Gln Trp Phe Val Gly Leu Ser
   530 535 540
   Pro Thr Val Trp Leu Ser Ala Ile Trp Met Met Trp Tyr Trp Gly Pro
   545 550 555 560
   Ser Leu Tyr Ser Ile Val Ser Pro Phe Ile Pro Leu Leu Pro Ile Phe
   565 570 575
   Phe Cys Leu Trp Val Tyr Ile
   580
<210> 15
   <211> 10855
   <212> DNA
   <213> Artificial Sequence
<220>
   <223> Lentivirus contenant des séquences dérivées de HCV et HBV
<400> 15
   ttaattccgt gtattctata gtgtcaccta aatcgtatgt gtatgataca taaggttatg 60
   tattaattgt agccgcgttc taacgacaat atgtacaagc ctaattgtgt agcatctggc 120
   ttactgaagc agaccctatc atctctctcg taaactgccg tcagagtcgg tttggttgga 180
   cgaaccttct gagtttctgg taacgccgtc ccgcacccgg aaatggtcag cgaaccaatc 240
   agcagggtca tcgctagcca gatcctctac gccggacgca tcgtggccgg catcaccggc 300
   gccacaggtg cggttgctgg cgcctatatc gccgacatca ccgatgggga agatcgggct 360
   cgccacttcg ggctcatgag cgcttgtttc ggcgtgggta tggtggcagg ccccgtggcc 420
   gggggactgt tgggcgccat ctccttgcat gcaccattcc ttgcggcggc ggtgctcaac 480
   ggcctcaacc tactactggg ctgcttccta atgcaggagt cgcataaggg agagcgtcga 540
   atggtgcact ctcagtacaa tctgctctga tgccgcatag ttaagccagc cccgacaccc 600
   gccaacaccc gctgacgcgc cctgacgggc ttgtctgctc ccggcatccg cttacagaca    660
   agctgtgacc gtctccggga gctgcatgtg tcagaggttt tcaccgtcat caccgaaacg    720
   cgcgagacga aagggcctcg tgatacgcct atttttatag gttaatgtca tgataataat    780
   ggtttcttag acgtcaggtg gcacttttcg gggaaatgtg cgcggaaccc ctatttgttt    840
   atttttctaa atacattcaa atatgtatcc gctcatgaga caataaccct gataaatgct    900
   tcaataatat tgaaaaagga agagtatgag tattcaacat ttccgtgtcg cccttattcc    960
   cttttttgcg gcattttgcc ttcctgtttt tgctcaccca gaaacgctgg tgaaagtaaa   1020
   agatgctgaa gatcagttgg gtgcacgagt gggttacatc gaactggatc tcaacagcgg   1080
   taagatcctt gagagttttc gccccgaaga acgttttcca atgatgagca cttttaaagt   1140
   tctgctatgt ggcgcggtat tatcccgtat tgacgccggg caagagcaac tcggtcgccg   1200
   catacactat tctcagaatg acttggttga gtactcacca gtcacagaaa agcatcttac   1260
   ggatggcatg acagtaagag aattatgcag tgctgccata accatgagtg ataacactgc   1320
   ggccaactta cttctgacaa cgatcggagg accgaaggag ctaaccgctt ttttgcacaa   1380
   catgggggat catgtaactc gccttgatcg ttgggaaccg gagctgaatg aagccatacc   1440
   aaacgacgag cgtgacacca cgatgcctgt agcaatggca acaacgttgc gcaaactatt   1500
   aactggcgaa ctacttactc tagcttcccg gcaacaatta atagactgga tggaggcgga   1560
   taaagttgca ggaccacttc tgcgctcggc ccttccggct ggctggttta ttgctgataa   1620
   atctggagcc ggtgagcgtg ggtctcgcgg tatcattgca gcactggggc cagatggtaa   1680
   gccctcccgt atcgtagtta tctacacgac ggggagtcag gcaactatgg atgaacgaaa   1740
   tagacagatc gctgagatag gtgcctcact gattaagcat tggtaactgt cagaccaagt   1800
   ttactcatat atactttaga ttgatttaaa acttcatttt taatttaaaa ggatctaggt   1860
   gaagatcctt tttgataatc tcatgaccaa aatcccttaa cgtgagtttt cgttccactg   1920
   agcgtcagac cccgtagaaa agatcaaagg atcttcttga gatccttttt ttctgcgcgt   1980
   aatctgctgc ttgcaaacaa aaaaaccacc gctaccagcg gtggtttgtt tgccggatca   2040
   agagctacca actctttttc cgaaggtaac tggcttcagc agagcgcaga taccaaatac   2100
   tgtccttcta gtgtagccgt agttaggcca ccacttcaag aactctgtag caccgcctac   2160
   atacctcgct ctgctaatcc tgttaccagt ggctgctgcc agtggcgata agtcgtgtct   2220
   taccgggttg gactcaagac gatagttacc ggataaggcg cagcggtcgg gctgaacggg   2280
   gggttcgtgc acacagccca gcttggagcg aacgacctac accgaactga gatacctaca   2340
   gcgtgagcat tgagaaagcg ccacgcttcc cgaagggaga aaggcggaca ggtatccggt   2400
   aagcggcagg gtcggaacag gagagcgcac gagggagctt ccagggggaa acgcctggta   2460
   tctttatagt cctgtcgggt ttcgccacct ctgacttgag cgtcgatttt tgtgatgctc 2520
   gtcagggggg cggagcctat ggaaaaacgc cagcaacgcg gcctttttac ggttcctggc 2580
   cttttgctgg ccttttgctc acatgttctt tcctgcgtta tcccctgatt ctgtggataa 2640
   ccgtattacc gcctttgagt gagctgatac cgctcgccgc agccgaacga ccgagcgcag 2700
   cgagtcagtg agcgaggaag cggaagagcg cccaatacgc aaaccgcctc tccccgcgcg 2760
   ttggccgatt cattaatgca gctgtggaat gtgtgtcagt tagggtgtgg aaagtcccca 2820
   ggctccccag caggcagaag tatgcaaagc atgcatctca attagtcagc aaccaggtgt 2880
   ggaaagtccc caggctcccc agcaggcaga agtatgcaaa gcatgcatct caattagtca 2940
   gcaaccatag tcccgcccct aactccgccc atcccgcccc taactccgcc cagttccgcc 3000
   cattctccgc cccatggctg actaattttt tttatttatg cagaggccga ggccgcctcg 3060
   gcctctgagc tattccagaa gtagtgagga ggcttttttg gaggcctagg cttttgcaaa 3120
   aagcttggac acaagacagg cttgcgagat atgtttgaga ataccacttt atcccgcgtc 3180
   agggagaggc agtgcgtaaa aagacgcgga ctcatgtgaa atactggttt ttagtgcgcc 3240
   agatctctat aatctcgcgc aacctatttt cccctcgaac actttttaag ccgtagataa 3300
   acaggctggg acacttcaca tgagcgaaaa atacatcgtc acctgggaca tgttgcagat 3360
   ccatgcacgt aaactcgcaa gccgactgat gccttctgaa caatggaaag gcattattgc 3420
   cgtaagccgt ggcggtctgt accgggtgcg ttactggcgc gtgaactggg tattcgtcat 3480
   gtcgataccg tttgtatttc cagctacgat cacgacaacc agcgcgagct taaagtgctg 3540
   aaacgcgcag aaggcgatgg cgaaggcttc atcgttattg atgacctggt ggataccggt 3600
   ggtactgcgg ttgcgattcg tgaaatgtat ccaaaagcgc actttgtcac catcttcgca 3660
   aaaccggctg gtcgtccgct ggttgatgac tatgttgttg atatcccgca agatacctgg 3720
   attgaacagc cgtgggatat gggcgtcgta ttcgtcccgc caatctccgg tcgctaatct 3780
   tttcaacgcc tggcactgcc gggcgttgtt ctttttaact tcaggcgggt tacaatagtt 3840
   tccagtaagt attctggagg ctgcatccat gacacaggca aacctgagcg aaaccctgtt 3900
   caaaccccgc tttaaacatc ctgaaacctc gacgctagtc cgccgcttta atcacggcgc 3960
   acaaccgcct gtgcagtcgg cccttgatgg taaaaccatc cctcactggt atcgcatgat 4020
   taaccgtctg atgtggatct ggcgcggcat tgacccacgc gaaatcctcg acgtccaggc 4080
   acgtattgtg atgagcgatg ccgaacgtac cgacgatgat ttatacgata cggtgattgg 4140
   ctaccgtggc ggcaactgga tttatgagtg ggccccggat ctttgtgaag gaaccttact 4200
   tctgtggtgt gacataattg gacaaactac ctacagagat ttaaagctct aaggtaaata 4260
   taaaattttt aagtgtataa tgtgttaaac tactgattct aattgtttgt gtattttaga 4320
   ttccaaccta tggaactgat gaatgggagc agtggtggaa tgcctttaat gaggaaaacc 4380
   tgttttgctc agaagaaatg ccatctagtg atgatgaggc tactgctgac tctcaacatt 4440
   ctactcctcc aaaaaagaag agaaaggtag aagaccccaa ggactttcct tcagaattgc 4500
   taagtttttt gagtcatgct gtgtttagta atagaactct tgcttgcttt gctatttaca 4560
   ccacaaagga aaaagctgca ctgctataca agaaaattat ggaaaaatat tctgtaacct 4620
   ttataagtag gcataacagt tataatcata acatactgtt ttttcttact ccacacaggc 4680
   atagagtgtc tgctattaat aactatgctc aaaaattgtg tacctttagc tttttaattt 4740
   gtaaaggggt taataaggaa tatttgatgt atagtgcctt gactagagat cataatcagc 4800
   cataccacat ttgtagaggt tttacttgct ttaaaaaacc tcccacacct ccccctgaac 4860
   ctgaaacata aaatgaatgc aattgttgtt gttaacttgt ttattgcagc ttataatggt 4920
   tacaaataaa gcaatagcat cacaaatttc acaaataaag catttttttc actgcattct 4980
   agttgtggtt tgtccaaact catcaatgta tcttatcatg tctggatcaa ctggataact 5040
   caagctaacc aaaatcatcc caaacttccc accccatacc ctattaccac tgccaattac 5100
   ctagtggttt catttactct aaacctgtga ttcctctgaa ttattttcat tttaaagaaa 5160
   ttgtatttgt taaatatgta ctacaaactt agtagttgga agggctaatt cactcccaaa 5220
   gaagacaaga tatccttgat ctgtggatct accacacaca aggctacttc cctgattagc 5280
   agaactacac accagggcca ggggtcagat atccactgac ctttggatgg tgctacaagc 5340
   tagtaccagt tgagccagat aaggtagaag aggccaataa aggagagaac accagcttgt 5400
   tacaccctgt gagcctgcat gggatggatg acccggagag agaagtgtta gagtggaggt 5460
   ttgacagccg cctagcattt catcacgtgg cccgagagct gcatccggag tacttcaaga 5520
   actgctgata tcgagcttgc tacaagggac tttccgctgg ggactttcca gggaggcgtg 5580
   gcctgggcgg gactggggag tggcgagccc tcagatcctg catataagca gctgcttttt 5640
   gcctgtactg ggtctctctg gttagaccag atctgagcct gggagctctc tggctaacta 5700
   gggaacccac tgcttaagcc tcaataaagc ttgccttgag tgcttcaagt agtgtgtgcc 5760
   cgtctgttgt gtgactctgg taactagaga tccctcagac ccttttagtc agtgtggaaa 5820
   atctctagca gtggcgcccg aacagggact tgaaagcgaa agggaaacca gaggagctct 5880
   ctcgacgcag gactcggctt gctgaagcgc gcacggcaag aggcgagggg cggcgactgg 5940
   tgagtacgcc aaaaattttg actagcggag gctagaagga gagagatggg tgcgagagcg 6000
   tcagtattaa gcgggggaga attagatcgc gatgggaaaa aattcggtta aggccagggg 6060
   gaaagaaaaa atataaatta aaacatatag tatgggcaag cagggagcta gaacgattcg 6120
   cagttaatcc tggcctgtta gaaacatcag aaggctgtag acaaatactg ggacagctac     6180
   aaccatccct tcagacagga tcagaagaac ttagatcatt atataataca gtagcaaccc     6240
   tctattgtgt gcatcaaagg atagagataa aagacaccaa ggaagcttta gacaagatag     6300
   aggaagagca aaacaaaagt aagaccaccg cacagcaagc ggccgctgat cttcagacct     6360
   ggaggaggag atatgaggga caattggaga agtgaattat ataaatataa agtagtaaaa     6420
   attgaaccat taggagtagc acccaccaag gcaaagagaa gagtggtgca gagagaaaaa     6480
   agagcagtgg gaataggagc tttgttcctt gggttcttgg gagcagcagg aagcactatg     6540
   ggcgcagcgt caatgacgct gacggtacag gccagacaat tattgtctgg tatagtgcag     6600
   cagcagaaca atttgctgag ggctattgag gcgcaacagc atctgttgca actcacagtc     6660
   tggggcatca agcagctcca ggcaagaatc ctggctgtgg aaagatacct aaaggatcaa     6720
   cagctcctgg ggatttgggg ttgctctgga aaactcattt gcaccactgc tgtgccttgg     6780
   aatgctagtt ggagtaataa atctctggaa cagatttgga atcacacgac ctggatggag     6840
   tgggacagag aaattaacaa ttacacaagc ttaatacact ccttaattga agaatcgcaa     6900
   aaccagcaag aaaagaatga acaagaatta ttggaattag ataaatgggc aagtttgtgg     6960
   aattggttta acataacaaa ttggctgtgg tatataaaat tattcataat gatagtagga     7020
   ggcttggtag gtttaagaat agtttttgct gtactttcta tagtgaatag agttaggcag     7080
   ggatattcac cattatcgtt tcagacccac ctcccaaccc cgaggggacc cgacaggccc     7140
   gaaggaatag aagaagaagg tggagagaga gacagagaca gatccattcg attagtgaac     7200
   ggatctcgac ggtatcgccg aattcacaaa tggcagtatt catccacaat tttaaaagaa     7260
   aaggggggat tggggggtac agtgcagggg aaagaatagt agacataata gcaacagaca     7320
   tacaaactaa agaattacaa aaacaaatta caaaaattca aaattttcgg gtttattaca     7380
   gggacagcag agatccactt tatcgataag cttgggagtt ccgcgttaca taacttacgg     7440
   taaatggccc gcctggctga ccgcccaacg acccccgccc attgacgtca ataatgacgt     7500
   atgttcccat agtaacgcca atagggactt tccattgacg tcaatgggtg gagtatttac     7560
   ggtaaactgc ccacttggca gtacatcaag tgtatcatat gccaagtacg ccccctattg     7620
   acgtcaatga cggtaaatgg cccgcctggc attatgccca gtacatgacc ttacgggact     7680
   ttcctacttg gcagtacatc tacgtattag tcatcgctat taccatggtg atgcggtttt     7740
   ggcagtacac caatgggcgt ggatagcggt ttgactcacg gggatttcca agtctccacc     7800
   ccattgacgt caatgggagt ttgttttggc accaaaatca acgggacttt ccaaaatgtc     7860
   gtaacaactc cgccccattg acgcaaatgg gcggtaggcg tgtacggtgg gaggtctata     7920
   taagcagagc tcgtttagtg aaccgtcaga tcgcctggag acgccatcca cgctgttttg 7980
   acctccatag aagacaccga ctctagctag aggatccatg taccaagtac gcaactcctc 8040
   gggcctttac catgtcacca atgattgccc taactcgagt attgtgtacg agacggccga 8100
   taccatccta cactctccgg ggtgtgtccc ttgcgttcgc gagggcaatg cctcaaaatg 8160
   ttgggtggcg gtggccccta cagtcgccac cagagacggc aagctcccca caacgcagct 8220
   tcgacgtcac atcgatctgc tcgtcaggag cgccaccctc tgctcggccc tctatgtggg 8280
   ggacttgtgc gggtccgtct tcctcgtcgg tcaactgttc accttctccc ccaggcgcca 8340
   ctggacaacg caagactgca actgttccat gtaccccggc catataacgg gtcaccgtat 8400
   ggcatgggac atgatgatga actggtcccc tacgacagcg ctggtagtag ctcagctgct 8460
   cagggtcccg caagccatct tggacatgat cgctggtgcc cactggggag tcctagcggg 8520
   catagcgtat ttctccatgg tggggaactg ggcgaaggtc ctggtggtgc tgttgctgtt 8580
   cgccagcaca agaatcctca caataccgca gagtctagac tcgtggtgga cttctctcaa 8640
   ttttctaggg ggatctcccg tgtgtcttgg ccaaaattcg cagtccccaa cctccaatca 8700
   ctcaccaacc tcctgtcctc caatttgtcc tggttatcgc tggatgtgtc tgcggcgttt 8760
   tatcatattc ctcttcatcc tgctgctatg cctcatcttc ttattggttc ttctggatta 8820
   tcaaggtatg ttgcccgttt gtcctctaat tccaggatca acaacaacca gtacgggacc 8880
   atgcaaaacc tgcacgactc ctgctcaagg caactctatg tttccctcat gttgctgtac 8940
   aaaacctacg gatggaaatt gcacctgtat tcccatccca tcgtcctggg ctttcgcaaa 9000
   atacctatgg gagtgggcct cagtccgttt ctcttggctc agtttactag tgccatttgt 9060
   tcagtggttc gtagggcttt cccccactgt ttggctttca gctatatgga tgatgtggta 9120
   ttgggggcca agtctgtaca gcatcgtgag tccctttata ccgctgttac caattttctt 9180
   ttgtctctgg gtatacattt aataaggatc cggactagta actcgaggcc cctctccctc 9240
   ccccccccct aacgttactg gccgaagccg cttggaataa ggccggtgtg cgtttgtcta 9300
   tatgttattt tccaccatat tgccgtcttt tggcaatgtg agggcccgga aacctggccc 9360
   tgtcttcttg acgagcattc ctaggggtct ttcccctctc gccaaaggaa tgcaaggtct 9420
   gttgaatgtc gtgaaggaag cagttcctct ggaagcttct tgaagacaaa caacgtctgt 9480
   agcgaccctt tgcaggcagc ggaacccccc acctggcgac aggtgcctct gcggccaaaa 9540
   gccacgtgta taagatacac ctgcaaaggc ggcacaaccc cagtgccacg ttgtgagttg 9600
   gatagttgtg gaaagagtca aatggctctc ctcaagcgta ttcaacaagg ggctgaagga 9660
   tgcccagaag gtaccccatt gtatgggatc tgatctgggg cctcggtaca catgctttac 9720
   atgtgtttag tcgaggttaa aaaaacgtct aggccccccg aaccacgggg acgtggtttt 9780
   cctttgaaaa acacgatgat aatatggcca caaccatggt gagcaagggc gaggagctgt      9840
   tcaccggggt ggtgcccatc ctggtcgagc tggacggcga cgtaaacggc cacaagttca      9900
   gcgtgtccgg cgagggcgag ggcgatgcca cctacggcaa gctgaccctg aagttcatct      9960
   gcaccaccgg caagctgccc gtgccctggc ccaccctcgt gaccaccctg acctacggcg     10020
   tgcagtgctt cagccgctac cccgaccaca tgaagcagca cgacttcttc aagtccgcca     10080
   tgcccgaagg ctacgtccag gagcgcacca tcttcttcaa ggacgacggc aactacaaga     10140
   cccgcgccga ggtgaagttc gagggcgaca ccctggtgaa ccgcatcgag ctgaagggca     10200
   tcgacttcaa ggaggacggc aacatcctgg ggcacaagct ggagtacaac tacaacagcc     10260
   acaacgtcta tatcatggcc gacaagcaga agaacggcat caaggtgaac ttcaagatcc     10320
   gccacaacat cgaggacggc agcgtgcagc tcgccgacca ctaccagcag aacaccccca     10380
   tcggcgacgg ccccgtgctg ctgcccgaca accactacct gagcacccag tccgccctga     10440
   gcaaagaccc caacgagaag cgcgatcaca tggtcctgct ggagttcgtg accgccgccg     10500
   ggatcactct cggcatggac gagctgtaca agtaagtcga gggaattcga gctcggtacc     10560
   tttaagacca atgacttaca aggcagctgt agatcttagc cactttttaa aagaaaaggg     10620
   gggactggaa gggctaattc actcccaacg aagacaagat ctttttgctt gtactgggtc     10680
   tctctggtta gaccagatct gagcctggga gctctctggc taactaggga acccactgct     10740
   taagcctcaa taaagcttgc cttgagtgct tcaagtagtg tgtgcccgtc tgttgtgtga     10800
   ctctggtaac tagagatccc tcagaccctt ttagtcagtg tggaaaatct ctaga          10855
<210> .16
   <211> 11368
   <212> DNA
   <213> Artificial Sequence
<220>
   <223> Lentivirus contenant des séquences dérivées de HCV et HBV
<400> 16
   ttaattccgt gtattctata gtgtcaccta aatcgtatgt gtatgataca taaggttatg        60
   tattaattgt agccgcgttc taacgacaat atgtacaagc ctaattgtgt agcatctggc       120
   ttactgaagc agaccctatc atctctctcg taaactgccg tcagagtcgg tttggttgga       180
   cgaaccttct gagtttctgg taacgccgtc ccgcacccgg aaatggtcag cgaaccaatc       240
   agcagggtca tcgctagcca gatcctctac gccggacgca tcgtggccgg catcaccggc       300
   gccacaggtg cggttgctgg cgcctatatc gccgacatca ccgatgggga agatcgggct       360
   cgccacttcg ggctcatgag cgcttgtttc ggcgtgggta tggtggcagg ccccgtggcc       420
   gggggactgt tgggcgccat ctccttgcat gcaccattcc ttgcggcggc ggtgctcaac 480
   ggcctcaacc tactactggg ctgcttccta atgcaggagt cgcataaggg agagcgtcga 540
   atggtgcact ctcagtacaa tctgctctga tgccgcatag ttaagccagc cccgacaccc 600
   gccaacaccc gctgacgcgc cctgacgggc ttgtctgctc ccggcatccg cttacagaca 660
   agctgtgacc gtctccggga gctgcatgtg tcagaggttt tcaccgtcat caccgaaacg 720
   cgcgagacga aagggcctcg tgatacgcct atttttatag gttaatgtca tgataataat 780
   ggtttcttag acgtcaggtg gcacttttcg gggaaatgtg cgcggaaccc ctatttgttt 840
   atttttctaa atacattcaa atatgtatcc gctcatgaga caataaccct gataaatgct 900
   tcaataatat tgaaaaagga agagtatgag tattcaacat ttccgtgtcg cccttattcc 960
   cttttttgcg gcattttgcc ttcctgtttt tgctcaccca gaaacgctgg tgaaagtaaa 1020
   agatgctgaa gatcagttgg gtgcacgagt gggttacatc gaactggatc tcaacagcgg 1080
   taagatcctt gagagttttc gccccgaaga acgttttcca atgatgagca cttttaaagt 1140
   tctgctatgt ggcgcggtat tatcccgtat tgacgccggg caagagcaac tcggtcgccg 1200
   catacactat tctcagaatg acttggttga gtactcacca gtcacagaaa agcatcttac 1260
   ggatggcatg acagtaagag aattatgcag tgctgccata accatgagtg ataacactgc 1320
   ggccaactta cttctgacaa cgatcggagg accgaaggag ctaaccgctt ttttgcacaa 1380
   catgggggat catgtaactc gccttgatcg ttgggaaccg gagctgaatg aagccatacc 1440
   aaacgacgag cgtgacacca cgatgcctgt agcaatggca acaacgttgc gcaaactatt 1500
   aactggcgaa ctacttactc tagcttcccg gcaacaatta atagactgga tggaggcgga 1560
   taaagttgca ggaccacttc tgcgctcggc ccttccggct ggctggttta ttgctgataa 1620
   atctggagcc ggtgagcgtg ggtctcgcgg tatcattgca gcactggggc cagatggtaa 1680
   gccctcccgt atcgtagtta tctacacgac ggggagtcag gcaactatgg atgaacgaaa 1740
   tagacagatc gctgagatag gtgcctcact gattaagcat tggtaactgt cagaccaagt 1800
   ttactcatat atactttaga ttgatttaaa acttcatttt taatttaaaa ggatctaggt 1860
   gaagatcctt tttgataatc tcatgaccaa aatcccttaa cgtgagtttt cgttccactg 1920
   agcgtcagac cccgtagaaa agatcaaagg atcttcttga gatccttttt ttctgcgcgt 1980
   aatctgctgc ttgcaaacaa aaaaaccacc gctaccagcg gtggtttgtt tgccggatca 2040
   agagctacca actctttttc cgaaggtaac tggcttcagc agagcgcaga taccaaatac 2100
   tgtccttcta gtgtagccgt agttaggcca ccacttcaag aactctgtag caccgcctac 2160
   atacctcgct ctgctaatcc tgttaccagt ggctgctgcc agtggcgata agtcgtgtct 2220
   taccgggttg gactcaagac gatagttacc ggataaggcg cagcggtcgg gctgaacggg 2280
   gggttcgtgc acacagccca gcttggagcg aacgacctac accgaactga gatacctaca 2340
   gcgtgagcat tgagaaagcg ccacgcttcc cgaagggaga aaggcggaca ggtatccggt 2400
   aagcggcagg gtcggaacag gagagcgcac gagggagctt ccagggggaa acgcctggta 2460
   tctttatagt cctgtcgggt ttcgccacct ctgacttgag cgtcgatttt tgtgatgctc 2520
   gtcagggggg cggagcctat ggaaaaacgc cagcaacgcg gcctttttac ggttcctggc 2580
   cttttgctgg ccttttgctc acatgttctt tcctgcgtta tcccctgatt ctgtggataa 2640
   ccgtattacc gcctttgagt gagctgatac cgctcgccgc agccgaacga ccgagcgcag 2700
   cgagtcagtg agcgaggaag cggaagagcg cccaatacgc aaaccgcctc tccccgcgcg 2760
   ttggccgatt cattaatgca gctgtggaat gtgtgtcagt tagggtgtgg aaagtcccca 2820
   ggctccccag caggcagaag tatgcaaagc atgcatctca attagtcagc aaccaggtgt 2880
   ggaaagtccc caggctcccc agcaggcaga agtatgcaaa gcatgcatct caattagtca 2940
   gcaaccatag tcccgcccct aactccgccc atcccgcccc taactccgcc cagttccgcc 3000
   cattctccgc cccatggctg actaattttt tttatttatg cagaggccga ggccgcctcg 3060
   gcctctgagc tattccagaa gtagtgagga ggcttttttg gaggcctagg cttttgcaaa 3120
   aagcttggac acaagacagg cttgcgagat atgtttgaga ataccacttt atcccgcgtc 3180
   agggagaggc agtgcgtaaa aagacgcgga ctcatgtgaa atactggttt ttagtgcgcc 3240
   agatctctat aatctcgcgc aacctatttt cccctcgaac actttttaag ccgtagataa 3300
   acaggctggg acacttcaca tgagcgaaaa atacatcgtc acctgggaca tgttgcagat 3360
   ccatgcacgt aaactcgcaa gccgactgat gccttctgaa caatggaaag gcattattgc 3420
   cgtaagccgt ggcggtctgt accgggtgcg ttactggcgc gtgaactggg tattcgtcat 3480
   gtcgataccg tttgtatttc cagctacgat cacgacaacc agcgcgagct taaagtgctg 3540
   aaacgcgcag aaggcgatgg cgaaggcttc atcgttattg atgacctggt ggataccggt 3600
   ggtactgcgg ttgcgattcg tgaaatgtat ccaaaagcgc actttgtcac catcttcgca 3660
   aaaccggctg gtcgtccgct ggttgatgac tatgttgttg atatcccgca agatacctgg 3720
   attgaacagc cgtgggatat gggcgtcgta ttcgtcccgc caatctccgg tcgctaatct 3780
   tttcaacgcc tggcactgcc gggcgttgtt ctttttaact tcaggcgggt tacaatagtt 3840
   tccagtaagt attctggagg ctgcatccat gacacaggca aacctgagcg aaaccctgtt 3900
   caaaccccgc tttaaacatc ctgaaacctc gacgctagtc cgccgcttta atcacggcgc 3960
   acaaccgcct gtgcagtcgg cccttgatgg taaaaccatc cctcactggt atcgcatgat 4020
   taaccgtctg atgtggatct ggcgcggcat tgacccacgc gaaatcctcg acgtccaggc 4080
   acgtattgtg atgagcgatg ccgaacgtac cgacgatgat ttatacgata cggtgattgg 4140
   ctaccgtggc ggcaactgga tttatgagtg ggccccggat ctttgtgaag gaaccttact 4200
   tctgtggtgt gacataattg gacaaactac ctacagagat ttaaagctct aaggtaaata 4260
   taaaattttt aagtgtataa tgtgttaaac tactgattct aattgtttgt gtattttaga 4320
   ttccaaccta tggaactgat gaatgggagc agtggtggaa tgcctttaat gaggaaaacc 4380
   tgttttgctc agaagaaatg ccatctagtg atgatgaggc tactgctgac tctcaacatt 4440
   ctactcctcc aaaaaagaag agaaaggtag aagaccccaa ggactttcct tcagaattgc 4500
   taagtttttt gagtcatgct gtgtttagta atagaactct tgcttgcttt gctatttaca 4560
   ccacaaagga aaaagctgca ctgctataca agaaaattat ggaaaaatat tctgtaacct 4620
   ttataagtag gcataacagt tataatcata acatactgtt ttttcttact ccacacaggc 4680
   atagagtgtc tgctattaat aactatgctc aaaaattgtg tacctttagc tttttaattt 4740
   gtaaaggggt taataaggaa tatttgatgt atagtgcctt gactagagat cataatcagc 4800
   cataccacat ttgtagaggt tttacttgct ttaaaaaacc tcccacacct ccccctgaac 4860
   ctgaaacata aaatgaatgc aattgttgtt gttaacttgt ttattgcagc ttataatggt 4920
   tacaaataaa gcaatagcat cacaaatttc acaaataaag catttttttc actgcattct 4980
   agttgtggtt tgtccaaact catcaatgta tcttatcatg tctggatcaa ctggataact 5040
   caagctaacc aaaatcatcc caaacttccc accccatacc ctattaccac tgccaattac 5100
   ctagtggttt catttactct aaacctgtga ttcctctgaa ttattttcat tttaaagaaa 5160
   ttgtatttgt taaatatgta ctacaaactt agtagttgga agggctaatt cactcccaaa 5220
   gaagacaaga tatccttgat ctgtggatct accacacaca aggctacttc cctgattagc 5280
   agaactacac accagggcca ggggtcagat atccactgac ctttggatgg tgctacaagc 5340
   tagtaccagt tgagccagat aaggtagaag aggccaataa aggagagaac accagcttgt 5400
   tacaccctgt gagcctgcat gggatggatg acccggagag agaagtgtta gagtggaggt 5460
   ttgacagccg cctagcattt catcacgtgg cccgagagct gcatccggag tacttcaaga 5520
   actgctgata tcgagcttgc tacaagggac tttccgctgg ggactttcca gggaggcgtg 5580
   gcctgggcgg gactggggag tggcgagccc tcagatcctg catataagca gctgcttttt 5640
   gcctgtactg ggtctctctg gttagaccag atctgagcct gggagctctc tggctaacta 5700
   gggaacccac tgcttaagcc tcaataaagc ttgccttgag tgcttcaagt agtgtgtgcc 5760
   cgtctgttgt gtgactctgg taactagaga tccctcagac ccttttagtc agtgtggaaa 5820
   atctctagca gtggcgcccg aacagggact tgaaagcgaa agggaaacca gaggagctct 5880
   ctcgacgcag gactcggctt gctgaagcgc gcacggcaag aggcgagggg cggcgactgg 5940
   tgagtacgcc aaaaattttg actagcggag gctagaagga gagagatggg tgcgagagcg 6000
   tcagtattaa gcgggggaga attagatcgc gatgggaaaa aattcggtta aggccagggg 6060
   gaaagaaaaa atataaatta aaacatatag tatgggcaag cagggagcta gaacgattcg 6120
   cagttaatcc tggcctgtta gaaacatcag aaggctgtag acaaatactg ggacagctac 6180
   aaccatccct tcagacagga tcagaagaac ttagatcatt atataataca gtagcaaccc 6240
   tctattgtgt gcatcaaagg atagagataa aagacaccaa ggaagcttta gacaagatag 6300
   aggaagagca aaacaaaagt aagaccaccg cacagcaagc ggccgctgat cttcagacct 6360
   ggaggaggag atatgaggga caattggaga agtgaattat ataaatataa agtagtaaaa 6420
   attgaaccat taggagtagc acccaccaag gcaaagagaa gagtggtgca gagagaaaaa 6480
   agagcagtgg gaataggagc tttgttcctt gggttcttgg gagcagcagg aagcactatg 6540
   ggcgcagcgt caatgacgct gacggtacag gccagacaat tattgtctgg tatagtgcag 6600
   cagcagaaca atttgctgag ggctattgag gcgcaacagc atctgttgca actcacagtc 6660
   tggggcatca agcagctcca ggcaagaatc ctggctgtgg aaagatacct aaaggatcaa 6720
   cagctcctgg ggatttgggg ttgctctgga aaactcattt gcaccactgc tgtgccttgg 6780
   aatgctagtt ggagtaataa atctctggaa cagatttgga atcacacgac ctggatggag 6840
   tgggacagag aaattaacaa ttacacaagc ttaatacact ccttaattga agaatcgcaa 6900
   aaccagcaag aaaagaatga acaagaatta ttggaattag ataaatgggc aagtttgtgg 6960
   aattggttta acataacaaa ttggctgtgg tatataaaat tattcataat gatagtagga 7020
   ggcttggtag gtttaagaat agtttttgct gtactttcta tagtgaatag agttaggcag 7080
   ggatattcac cattatcgtt tcagacccac ctcccaaccc cgaggggacc cgacaggccc 7140
   gaaggaatag aagaagaagg tggagagaga gacagagaca gatccattcg attagtgaac 7200
   ggatctcgac ggtatcgccg aattcacaaa tggcagtatt catccacaat tttaaaagaa 7260
   aaggggggat tggggggtac agtgcagggg aaagaatagt agacataata gcaacagaca 7320
   tacaaactaa agaattacaa aaacaaatta caaaaattca aaattttcgg gtttattaca 7380
   gggacagcag agatccactt tatcgataag cttgggagtt ccgcgttaca taacttacgg 7440
   taaatggccc gcctggctga ccgcccaacg acccccgccc attgacgtca ataatgacgt 7500
   atgttcccat agtaacgcca atagggactt tccattgacg tcaatgggtg gagtatttac 7560
   ggtaaactgc ccacttggca gtacatcaag tgtatcatat gccaagtacg ccccctattg 7620
   acgtcaatga cggtaaatgg cccgcctggc attatgccca gtacatgacc ttacgggact 7680
   ttcctacttg gcagtacatc tacgtattag tcatcgctat taccatggtg atgcggtttt 7740
   ggcagtacac caatgggcgt ggatagcggt ttgactcacg gggatttcca agtctccacc 7800
   ccattgacgt caatgggagt ttgttttggc accaaaatca acgggacttt ccaaaatgtc 7860
   gtaacaactc cgccccattg acgcaaatgg gcggtaggcg tgtacggtgg gaggtctata 7920
   taagcagagc tcgtttagtg aaccgtcaga tcgcctggag acgccatcca cgctgttttg 7980
   acctccatag aagacaccga ctctagctag aggatccatg gaaacctaca ccaccggggg 8040
   gagtattgcc aaaaccgtgc aaggattcac cagttttttt accccaggcg ccaagcagga 8100
   catccagctg atcaacacca acggcagttg gcacatcaat cgcacggcct tgaactgtaa 8160
   tgcgagcctc gaaaccggct ggatagcggg gctcttctac tacaacaaat tcaactcctc 8220
   aggctgcccc gagaggatgg ccagctgcaa accccttgcc tatttcgccc aaggctgggg 8280
   ccctatcagc catgtcaacg gaagcggccc cgaacagcgc ccctactgct ggcactacgc 8340
   cccaaggcct tgtggtatcg tgtcagcaca gacagtatgt ggcccagtgt attgtttcac 8400
   tcctagcccc gtggtggtgg ggacgaccga caagttgggc gcgcctacct acaactgggg 8460
   tgagaatgat acggacgtct tcgtcctcaa taacaccagg ccaccgttgg gcaattggtt 8520
   cggttgcacc tggatgaact catctggatt caccaaagtg tgcggagcgc ctccctgtgc 8580
   catcggagga gtgggcaata acaccttgcg ctgtcccact gactgtttcc gcaagcatcc 8640
   ggaagctaca tactctcgat gtggctccgg tccctggatc acgcccaggt gcctggtcga 8700
   ctatccttat aggctctggc attatccttg cactgtcaac tacaccctgt ttaaaatcag 8760
   gatgtacgtg ggaggggtcg agcacaggct acaagctgct tgcaactgga cgcggggcga 8820
   gcgttgtgat ctggacgaca gggacaggtc cgagctcagc ccgctgctgc tgtccaccac 8880
   gcagtggcag gtccttccgt gttctttcac gaccttgcca gccttgacca ccggcctcat 8940
   ccacctccat cagaacatcg tggacgtgca atatttgtac ggggtggggt caagcattgt 9000
   gtcctgggcc atcaagtggg agtacgtcat tctcttgttt ctcctgcttg cagacgcgcg 9060
   catctgctcc tgcttgtgga tgatgctact catatcccaa acaagaatcc tcacaatacc 9120
   gcagagtcta gactcgtggt ggacttctct caattttcta gggggatctc ccgtgtgtct 9180
   tggccaaaat tcgcagtccc caacctccaa tcactcacca acctcctgtc ctccaatttg 9240
   tcctggttat cgctggatgt gtctgcggcg ttttatcata ttcctcttca tcctgctgct 9300
   atgcctcatc ttcttattgg ttcttctgga ttatcaaggt atgttgcccg tttgtcctct 9360
   aattccagga tcaacaacaa ccagtacggg accatgcaaa acctgcacga ctcctgctca 9420
   aggcaactct atgtttccct catgttgctg tacaaaacct acggatggaa attgcacctg 9480
   tattcccatc ccatcgtcct gggctttcgc aaaataccta tgggagtggg cctcagtccg 9540
   tttctcttgg ctcagtttac tagtgccatt tgttcagtgg ttcgtagggc tttcccccac 9600
   tgtttggctt tcagctatat ggatgatgtg gtattggggg ccaagtctgt acagcatcgt      9660
   gagtcccttt ataccgctgt taccaatttt cttttgtctc tgggtataca tttaataagg      9720
   atccggacta gtaactcgag gcccctctcc ctcccccccc cctaacgtta ctggccgaag      9780
   ccgcttggaa taaggccggt gtgcgtttgt ctatatgtta ttttccacca tattgccgtc      9840
   ttttggcaat gtgagggccc ggaaacctgg ccctgtcttc ttgacgagca ttcctagggg      9900
   tctttcccct ctcgccaaag gaatgcaagg tctgttgaat gtcgtgaagg aagcagttcc      9960
   tctggaagct tcttgaagac aaacaacgtc tgtagcgacc ctttgcaggc agcggaaccc     10020
   cccacctggc gacaggtgcc tctgcggcca aaagccacgt gtataagata cacctgcaaa     10080
   ggcggcacaa ccccagtgcc acgttgtgag ttggatagtt gtggaaagag tcaaatggct     10140
   ctcctcaagc gtattcaaca aggggctgaa ggatgcccag aaggtacccc attgtatggg     10200
   atctgatctg gggcctcggt acacatgctt tacatgtgtt tagtcgaggt taaaaaaacg     10260
   tctaggcccc ccgaaccacg gggacgtggt tttcctttga aaaacacgat gataatatgg     10320
   ccacaaccat ggtgagcaag ggcgaggagc tgttcaccgg ggtggtgccc atcctggtcg     10380
   agctggacgg cgacgtaaac ggccacaagt tcagcgtgtc cggcgagggc gagggcgatg     10440
   ccacctacgg caagctgacc ctgaagttca tctgcaccac cggcaagctg cccgtgccct     10500
   ggcccaccct cgtgaccacc ctgacctacg gcgtgcagtg cttcagccgc taccccgacc     10560
   acatgaagca gcacgacttc ttcaagtccg ccatgcccga aggctacgtc caggagcgca     10620
   ccatcttctt caaggacgac ggcaactaca agacccgcgc cgaggtgaag ttcgagggcg     10680
   acaccctggt gaaccgcatc gagctgaagg gcatcgactt caaggaggac ggcaacatcc     10740
   tggggcacaa gctggagtac aactacaaca gccacaacgt ctatatcatg gccgacaagc     10800
   agaagaacgg catcaaggtg aacttcaaga tccgccacaa catcgaggac ggcagcgtgc     10860
   agctcgccga ccactaccag cagaacaccc ccatcggcga cggccccgtg ctgctgcccg     10920
   acaaccacta cctgagcacc cagtccgccc tgagcaaaga ccccaacgag aagcgcgatc     10980
   acatggtcct gctggagttc gtgaccgccg ccgggatcac tctcggcatg gacgagctgt     11040
   acaagtaagt cgagggaatt cgagctcggt acctttaaga ccaatgactt acaaggcagc     11100
   tgtagatctt agccactttt taaaagaaaa ggggggactg gaagggctaa ttcactccca     11160
   acgaagacaa gatctttttg cttgtactgg gtctctctgg ttagaccaga tctgagcctg     11220
   ggagctctct ggctaactag ggaacccact gcttaagcct caataaagct tgccttgagt     11280
   gcttcaagta gtgtgtgccc gtctgttgtg tgactctggt aactagagat ccctcagacc     11340
   cttttagtca gtgtggaaaa tctctaga                                        11368